(19) Europäisches Patentamt
European Patent Office
Office européen des brevets

(11) EP 2 011 388 A1

(12) EUROPEAN PATENT APPLICATION

(43) Date of publication:
07.01.2009 Bulletin 2009/02

(51) Int Cl.:
*A01H 5/02* (2006.01)

(21) Application number: 08075608.3

(22) Date of filing: 07.07.2008

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(30) Priority: 06.07.2007 US 948380 P

(71) Applicant: Terra Nigra B.V.
1433 AP Kudelstaart (NL)

(72) Inventors:
• Van Os, Diana Petronella Maria
3602 AT Maarssen (NL)
• Stravers, Lambertus Johannes Maria
1433 NG Kudelstaart (NL)

(74) Representative: Dohmen, Johannes Maria Gerardus et al
Algemeen Octrooi- en Merkenbureau
P.O. Box 645
5600 AP Eindhoven (NL)

(54) **Gerbera with leafy flower stem trait and in bud shipping trait**

(57) New, distinct and stable *Gerbera L.* cultivars with foliage on the flower stem are disclosed. The new leafy flower stem *Gerbera L. cultivars* may have the ability to be packaged and transported during the in bud stage of development. The new *Gerbera L.* cultivars produce one or more flower stems with at least 5 or more full or partial leaves per flower stem. The leaves produced on a flower stem of a *Gerbera L.* cultivar of the present invention range in size from small to extra large and range in measurement from a) at least 40 mm or more in length, and b) at least 4 mm or more in width. Methods for the breeding of the leafy flower stem trait into diverse *Gerbera L.* genetic backgrounds, as well as, methods for increasing the degree of foliage per flower stem or plant are disclosed. *Gerbera L.* cultivars of the present invention are referred herein as the *Gerbera* brand GERFO-LIA™.

FIG. 2A

**Description**

CROSS REFERENCE TO RELATED PATENT APPLICATIONS

This application claims priority from United States Provisional Application 60/948,380 filed July 6, 2007, incorporated herein by reference in its entirety.

BACKGROUND OF THE INVENTION

1. Field of the Invention

The present invention relates to new, distinct and stable cultivars of *Gerbera L.* plants that produce one or more flower stems with at least 5 or more full or partial leaves per flower stem. A *Gerbera L.* cultivar of the present invention can produce one or more flower stems with at least 5, to about 30, or more than 30 full or partial leaves per flower stem. A *Gerbera L.* cultivar of the instant invention can produce at least 5, 6, 8, 10, 12, 14, 16, 18, 20, 22, 24, 26, 28, 30, any integer between 5 and 30, or more than 30 full or partial leaves per flower stem. The leaves produced on a flower stem of a *Gerbera L.* cultivar of the present invention range in size from small to extra-large. The leaves produced on a flower stem of a *Gerbera L.* cultivar of the present invention can range in size from a) at least 40 mm or more in length, and b) at least 4 mm or more in width. The leaves produced on a flower stem of a *Gerbera L.* cultivar of the present invention can range in size from a) at least 40 mm, to about 200 mm, or more than 200 mm in length, and b) at least 4 mm, to about 60 mm, or more than 60 mm in width. The leaves produced by a flower stem of a *Gerbera L.* cultivar of the present invention can range in size from a) at least 40, 50, 60, 70, 80, 90, 100, 110, 120, 130, 140, 150, 160, 170, 180, 190, 200, any integer between 40 and 200, or more than 200 mm in length, and b) at least 4, 6, 8, 10, 12, 14, 16, 18, 20, 22, 24, 26, 28, 30, 32, 34, 36, 38, 40, 42, 44, 46, 48, 50, 52, 54, 56, 58, 60, any integer between 4 and 60, or more than 60 mm in width. In addition, the leaves produced on a flower stem of a *Gerbera L.* cultivar of the instant invention are distributed either equally along the flower stem or along the top half of the flower stem. Further, *Gerbera L.* cultivars of the present invention, exhibiting at least 5 or more full or partial leaves per flower stem, may have the ability to be packaged and transported during the in bud stage of development.

The present invention relates to the new leafy flower stem trait which has been successfully introgressed from a *Gerbera L.* cultivar having the leafy flower stem trait in its genetic background into non-leafy *Gerbera L.* plants that contain less than 5 full or partial leaves per flower stem. The present invention further relates to methods for increasing the degree of the leafy flower stem trait per flower stem per plant. The leafy flower stem characteristic has also been combined with many desirable *Gerbera L.* traits, such as flowering type, flower color, flower size, flower shape, flower quality, ray floret and disc floret shape and size, flower stem length, flower stem quality, flower stem productivity and vase life. Accordingly, the leafy flower stem *Gerbera L.* plants of the present invention offer a highly desirable new phenotype for the *Gerbera L.* commercial market, which the typical *Gerbera L.* plants of the commercial market do not possess. *Gerbera L.* cultivars of the present invention, exhibiting the new leafy flower stem trait, are referred to as belonging to the *Gerbera L.* brand GERFOLIA™.

The present invention further relates to the in bud shipping trait which has been successfully introgressed from a *Gerbera L.* cultivar having the leafy flower stem trait in its genetic background into non-leafy *Gerbera L.* plants that contain less than 5 full or partial leaves per flower stem. *Gerbera L.* plants of the present invention, which exhibit the leafy flower stem trait, may also exhibit the in bud shipping trait, since expression of the new leafy flower stem trait confers expression of the in bud shipping trait. In addition, leafy flower stem *Gerbera L.* plants of the present invention, which may also exhibit the in bud shipping trait, maintain characteristics considered desirable in the *Gerbera* commercial, ornamental market, such as flowering type, flower color, flower size, flower shape, flower quality, ray floret and disc floret shape and size, flower stem length, flower stem quality, flower stem productivity and vase life. Accordingly, the leafy flower stem *Gerbera L.* plants of the present invention, which may express the in bud shipping trait, offer highly desirable phenotypes for the *Gerbera L.* commercial market, which the typical *Gerbera L.* plants of the commercial market do not possess.

Typical non-leafy *Gerbera L.* plants require open flower shipping, whereas the leafy flower stem trait of *Gerbera L.* plants of the present invention may have the ability to be packaged and shipped during the in bud shipping stage. As a result of GERFOLIA™ *Gerbera L.* plants of the present invention having the potential ability to be shipped during the in bud stage of development, flowers of the new GERFOLIA™ *Gerbera L.* plants that are shipped during the in bud stage are less prone to breakage and are generally associated with a longer shelf life when compared to typical non-leafy *Gerbera L.* plants which must be shipped during the open flower stage. In addition, shipment during the in bud shipping stage for GERFOLIA™ *Gerbera L.* plants of the present invention allows for commerical packaging and transport when the flowers of the *Gerbera L.* plants are more compact and uniform in size. Accordingly, GERFOLIA™ *Gerbera L.* plants of the present invention, which possess the ability to be shipped during the in bud stage of development, are more economical to grow and process, since i) the flower stems can be harvested earlier for packaging and transport, and ii) a larger number of leafy *Gerbera* L. flower stems can be packaged to fill a transport container in comparison to non-leafy *Gerbera L.* flower stems. In addition, damage during packaging and transport is reduced since the leaves on the flower stems of the GERFOLIA™ *Gerbera L.* of the present invention provide a form of natural protection over the flower stem. Lastly, GERFOLIA™ *Gerbera L.* of the present invention which have the ability to be shipped during the in bud stage of development, provide a new and unique *Gerbera L.* end product in the commerical market due to i) the leafy flower stem phenotype, and ii) the ability for the flowers on the flower stem to open completely in a vase after purchase by the consumer.

2. Background

The present invention relates generally to the field of ornamental *Gerbera* plants. The genus of *Gerbera* belongs to the *Asteraceae/Compositae* family. There are more than 40 different species of *Gerbera,* including *G. aberdarica, G. abyssinica, G. ambigua, G. anandria, G. aspleniifolia, G. aurantiaca, G. bojeri, G. bonatiana, G. bracteata, G. brevipes, G. burchellii, G. burmanni, G. candollei, G. cavaleriei, G. chilensis, G. cineraria, G. connata, G. conrathii, , G. cordata, G. coronopifolia, G. curvisquama, G. delavayi, G. discolor, G. diversifolia, G. elegans, G. elliptica, G. emirnensis, G. ferruginea, G. flava, G. galpinii, G. glandulosa, G. henryi, G. hieracioides, G. hirsuta, G. hypochaeridoides, G. integralis, G. integripetala, G. jamesonii, G. knorringiana, G. kokanica, G. kraussii, G. kunzeana, G. lacei, G. lagascae, G. lanuginosa, G. lasiopus, G. leandrii, G. leiocarpa, G. leucothrix, G. lijiangensis, G. lynchii, G. macrocephala, G. nepalensis, G. nervosa, G. nivea, G. parva, G. peregrina, G. perrieri, G. petasitifolia, G. piloselloides, G. plantaginea, G. plicata, G. podophylla, G. pterodonta, G. pulvinata, G. pumila, G. randii, G. raphanifolia, G. ruficoma, G. saxatilis, G. semifloscularis, G. serotina, G. speciosa, G. tanantii, G. tomentosa, G. tuberosa, G. uncinata, G. viridifolia, G. welwitschii, G. wrightii,* and which species are distributed from Africa into Madagascar into tropical Asia and into South America.

*Gerbera L.* is an important crop in ornamental horticulture worldwide. Through hybridization, *Gerbera* species are phenotypically diverse and offer a wide range of flower colors including whites, yellows, oranges, pinks, reds, purples and bi-colors. Since *Gerbera L.* flowers have a long vase life, *Gerbera L.* flowers are widely used in the commercial horticultural industry. A *Gerbera L.* cultivar can be grown as a cut flower, as well as, a potting plant.

The current invention is mainly suitable for cut flower stem production, though some cultivars can also be used as potting plants. The present invention allows the *Gerbera* market to expand as a completely new type of *Gerbera L.* flower stem is produced. The breeding of *Gerbera* cultivars, exhibiting the leafy flower stem trait, which produce one or more flower stems with at least 5, to about 30, or more than 30 full or partial leaves per flower stem, offers a unique, new *Gerbera* flower stem form that can be combined with different *Gerbera* flowering-type species, thereby expanding the range of phenotypic characteristics available in this popular horticultural plant.

Further, the breeding of *Gerbera L.* cultivars, exhibiting the leafy flower stem trait, which produce one or more flower stems with at least 5, to about 30, or more than 30 full or partial leaves per flower stem, offers an economic advantage in the commercial horticultural industry since the new type of leafy flower stem may have the ability to be shipped in the bud stage rather than in the open flower stage which is customary for *Gerbera* cultivars with non-leafy flower stems. First, the surface area of a bunch of GERFOLIA™ brand flowers which can be shipped in bud stage is less than the surface area of the same bunch count of flowers of any *Gerbera* cultivars which are shipped in open flower stage. Accordingly, standard commercial packages containing GERFOLIA™ brand flowers which are shipped in the bud stage have a larger packing density compared to standard commercial packages containing any *Gerbera* cultivars shipped in the open flower stage. Second, the leafy flower stem trait provides further economic advantage by i) reducing the overall production cost with an earlier cutting/harvest time, and ii) extending the allowable time for commercial transportation and distribution. The foliage on the leafy flower stem provide nutrients for subsequent growth (opening of flower and growth of leaves), and in turn, increases the holding quality of the flower stem. Thus, the *Gerbera* brand GERFOLIA™, which express the ability to be shipped in the bud stage, have the potential to have a significant advantage in the commercial market, since commercial distribution may be extended from a local or regional market to an international market.

SUMMARY OF THE INVENTION

An object of the present invention is to provide *Gerbera L.* plants which produce one or more flower stems with at least 5 or more full or partial leaves per flower stem.

Another object of the present invention is to provide *Gerbera L.* plants wherein substantially all the flower stems produced by said plant contain foliage with at least 5 or more full or partial leaves per flower stem.

Another object of the present invention is to provide *Gerbera L.* plants wherein the flower stems produced by said plant contain foliage with at least 5 or more full or partial leaves per flower stem, wherein the leaves of the flower stems are (i) small to extra large in size, ranging from (a) at least 40 mm or more in length, and (b) at least 4 mm or more in width, and (ii) distributed either equally along the flower stem or along the top half of the flower stem.

Another object of the present invention is to provide *Gerbera L.* plants which produce one or more flower stems with at least 5, to about 30, or more than 30 full or partial leaves per flower stem.

Another object of the present invention is to provide *Gerbera L.* plants wherein substantially all the flower stems produced by said plant contain foliage with at least 5, to about 30, or more than 30 full or partial leaves per flower stem.

Another object of the present invention is to provide *Gerbera L.* plants wherein the flower stems produced by said plant contain foliage with at least at least 5, to about 30, or more than 30 full or partial leaves per flower stem, wherein the leaves of the flower stems are (i) small to extra large in size, ranging from (a) at least 40 mm, to about 200 mm, or more than 200 mm in length, and (b) at least 4 mm, to about 60 mm, or more than 60 mm in width, and (ii) distributed either equally along the flower stem or along the top half of the flower stem.

Another object of the present invention is to provide *Gerbera L.* plants which produce one or more flower stems with at least 5, 6, 8, 10, 12, 14, 16, 18, 20, 22, 24, 26, 28, 30, any integer between 5 and 30, or more than 30 full or partial leaves per flower stem.

Another object of the present invention is to provide *Gerbera L.* plants wherein substantially all the flower stems produced by said plant contain foliage with at least 5, 6, 8, 10, 12, 14, 16, 18, 20, 22, 24, 26, 28, 30, any integer between 5 and 30, or more than 30 full or partial leaves per flower stem.

Another object of the present invention is to provide *Gerbera L.* plants wherein the flower stems produced by said plant contain foliage with at least 5, 8, 10, 12, 14, 16, 18, 20, 22, 24, 26, 28, 30, any integer between 5 and 30, or more than 30 full or partial leaves per flower stem, wherein the leaves of the flower stem are (i) small to extra large in size, ranging from (a) at least 40, 50, 60, 70, 80, 90, 100, 110, 120, 130, 140, 150, 160, 170, 180, 190, 200, any integer between 40 and 200, or more than 200 mm in length, and (b) at least 4, 6, 8, 10, 12, 14, 16, 18, 20, 22, 24, 26, 28, 30, 32, 34, 36, 38, 40, 42, 44, 46, 48, 50, 52, 54, 56, 58, 60, any integer between 4 and 60, or more than 60 mm in width, and (ii) distributed either equally along the flower stem or along the top half of the flower stem.

Another object of the present invention is to provide leafy flower stem *Gerbera L.* plants, wherein said plant is selected from the group consisting of *G. aberdarica, G. abyssinica, G. ambigua, G. anandria, G. aspleniifolia, G. aurantiaca, G. bojeri, G. bonatiana, G. bracteata, G. brevipes, G. burchellii, G. burmanni, G. candollei, G. cavaleriei, G. chilensis, G. cineraria, G. connata, G. conrathii, , G. cordata, G. coronopifolia, G. curvisquama, G. delavayi, G. discolor, G. diversifolia, G. elegans, G. elliptica, G. emirnensis, G. ferruginea, G. flava, G. galpinii, G. glandulosa, G. henryi, G. hieracioides, G. hirsuta, G. hypochaeridoides, G. integralis, G. integripetala, G. jamesonii, G. knorringiana, G. kokanica, G. kraussii, G. kunzeana, G. lacei, G. lagascae, G. lanuginosa, G. lasiopus, G. leandrii, G. leiocarpa, G. leucothrix, G. lijiangensis, G. lynchii, G. macrocephala, G. nepalensis, G. nervosa, G. nivea, G. parva, G. peregrina, G. perrieri, G. petasitifolia, G. piloselloides, G. plantaginea, G. plicata, G. podophylla, G. pterodonta, G. pulvinata, G. pumila, G. randii, G. raphanifolia, G. ruficoma, G. saxatilis, G. semifloscularis, G. serotina, G. speciosa, G. tanantii, G. tomentosa, G. tuberosa, G. uncinata, G. viridifolia, G. welwitschii and G. wrightii.*

Another object of the present invention is to provide methods for breeding of the leafy flower stem trait from a *Gerbera L.* plant possessing the trait into diverse *Gerbera L.* genetic backgrounds that do not possess the leafy flower stem trait.

Another object of the present invention is to provide methods for breeding of the leafy flower stem trait from a *Gerbera L.* plant possessing the traits into diverse Normal-type and Mini*Gerbera*-type *Gerbera L.* genetic backgrounds that do not possess the leafy flower stem trait.

Another object of the present invention is to provide methods for breeding of the leafy flower stem trait from a *Gerbera L.* plant possessing the traits into diverse single-type, semi-double-type, double-type, double-multi-type and multi-petalled-type *Gerbera L.* genetic backgrounds that do not possess the leafy flower stem trait.

Another object of the invention is to provide a method of breeding leafy flower stem *Gerbera L.* plants that produce one or more flower stems, including observing, measuring and comparing said flower stem having a degree of foliage per flower stem defined as a measure of at least 5 or more full or partial leaves per flower stem, comprising the steps of (a) crossing a first single-type *Gerbera L.* plant having the leafy flower stem trait in its genetic background, either as the male or female parent to: (i) a *Gerbera L.* plant without the leafy flower stem trait in its genetic background; or (ii) a second *Gerbera L.* plant having the leafy flower stem trait in its genetic background; (b) screening F1 progeny; (c) selecting F1 progeny having the leafy flower stem trait in its genetic background; (d) crossing said F1 progeny to: (i) a second *Gerbera L.* plant without the leafy flower stem trait in its genetic background; or (ii) a third *Gerbera L.* plant having the leafy flower stem trait in its genetic background; (e) screening F2 progeny; (f)selecting F2 progeny having the leafy flower stem trait in its genetic background; (g) crossing said F2 progeny to: (i) a third *Gerbera L.* plant without the leafy flower stem trait in its genetic background; or (ii) a fourth *Gerbera L.* plant having the leafy flower stem trait in its genetic background; (h) screening F3 progeny; (i) selecting F3 progeny having the leafy flower stem trait in its genetic background; (j) crossing said F3 progeny to: (i) a fourth *Gerbera L.* plant without the leafy flower stem trait in its genetic background; or (ii) a fifth *Gerbera L.* plant having the leafy flower stem trait in its genetic background; (k) screening F4 progeny; (l) selecting F4 progeny having the leafy flower stem trait in its genetic background; (m) crossing said F4 progeny to: (i) a fifth *Gerbera L.* plant without the leafy flower stem trait in its genetic background; or (ii) a sixth *Gerbera L.* plant having the leafy flower stem trait in its genetic background; (n) screening F5 progeny; (o) selecting F5 progeny having the leafy flower stem trait in its genetic background; (p) crossing said F5 progeny to: (i) a sixth *Gerbera L.* plant without the leafy flower stem trait in its genetic background; or (ii) a seventh *Gerbera L.* plant having the leafy flower stem trait in its genetic background; (q) screening F6 progeny; (r) selecting F6 progeny having the leafy flower stem trait in its genetic background; (s) crossing said F6 progeny to: (i) a seventh *Gerbera L.* plant without the leafy flower stem trait in its genetic background; or (ii) an eighth *Gerbera L.* plant having the leafy flower stem trait in its genetic background; (t) screening F7 progeny and (n) selecting leafy flower stem progeny. The first, second, third, fourth, fifth, sixth, seventh or eighth *Gerbera L.* plant having the leafy flower stem trait in its genetic background are the same or different cultivars.

Another object of the present invention is to provide a *Gerbera L.* plant, with one or more flower stems with at least 5 or more full or partial leaves per flower stem, which are a) small to extra large in size (ranging from at least 40 mm or more, to about 200 mm or more in length, and at least 4 mm or more, to about 60 mm or more in width), and b) which are distributed either equally along the flower stem or along the top half of the flower stem, wherein said *Gerbera L.* plant is produced by the methods disclosed.

Another object of the present invention is to provide a *Gerbera L.* plant, with one or more flower stems with at least 5, to about 30, or more than 30 full or partial leaves per flower stem, which are a) small to extra large in size (ranging from at least 40 mm, to about 200 mm, or more than 200 mm in length, and at least 4 mm, to about 60 mm or more than 60 mm in width), and b) which are distributed either equally along the flower stem or along the top half of the flower stem, wherein said *Gerbera L.* plant is produced by one of the disclosed methods.

Another object of the present invention is to provide a *Gerbera L.* plant, with one or more flower stems with at least 5, 6, 8, 10, 12, 14, 16, 18, 20, 22, 24, 26, 28, 30, any integer between 5 and 30, or more than 30 full or partial leaves per flower stem, which are a) small to extra large in size (ranging from at least 40, 50, 60, 70, 80, 90, 100, 110, 120, 130, 140, 150, 160, 170, 180, 190, 200, any integer between 40 and 200, or more than 200 mm in length, and b) at least 4, 6, 8, 10, 12, 14, 16, 18, 20, 22, 24, 26, 28, 30, 32, 34, 36, 38, 40, 42, 44, 46, 48, 50, 52, 54, 56, 58, 60, any integer between 4 and 60, or more than 60 mm in width), and b) which are distributed either equally along the flower stem or along the top half of the flower stem, wherein said *Gerbera L.* plant is produced by one of the disclosed methods.

BRIEF DESCRIPTION OF THE DRAWINGS

The patent or application file contains at least one drawing executed in color. Copies of this patent or patent application publication with color drawings will be provided by the Office upon request and payment of the necessary fees.

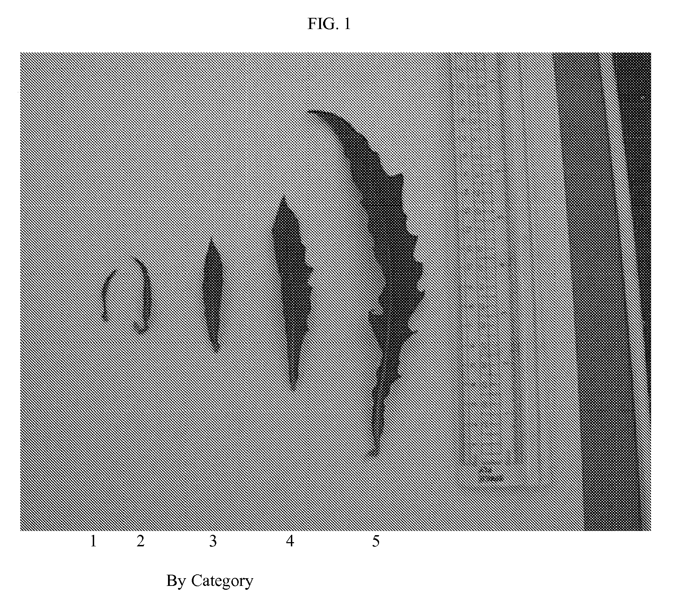

FIG. 1 shows a top view perspective of different leaf shapes and sizes of *Gerbera L.* as described by Categories 1-5 of Table 2.

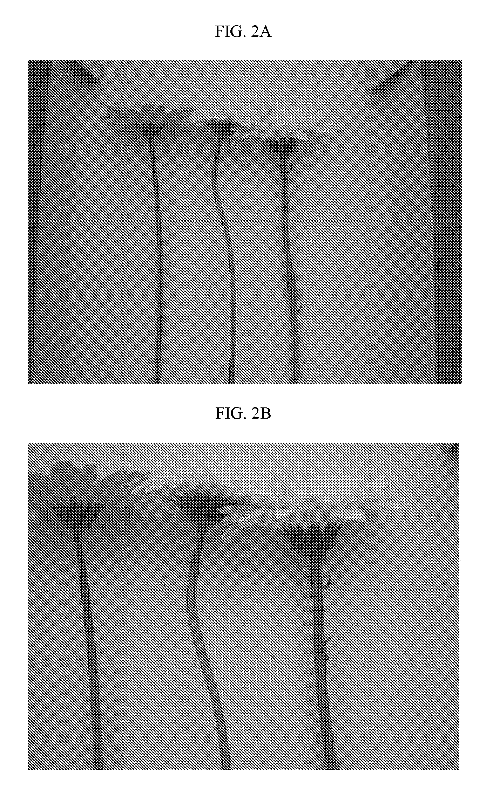

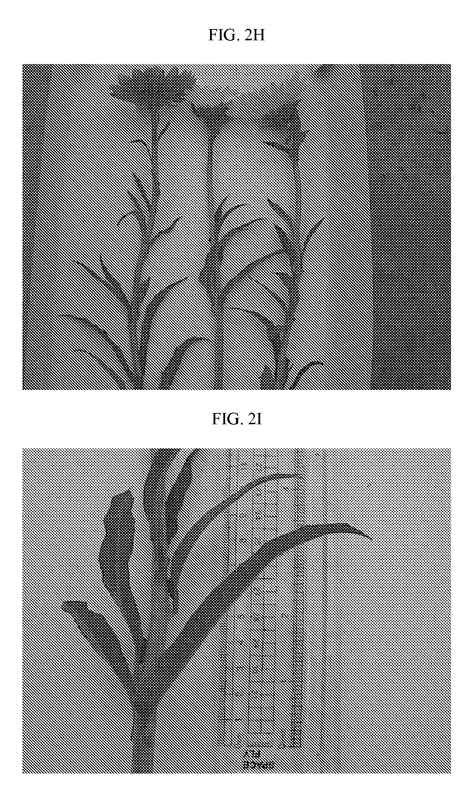

FIG. 2A shows a side view perspective and FIG. 2B shows a close-up side view perspective of typical leaves on the flower stem of *Gerbera L.* as described by Category 0 of Table 3.

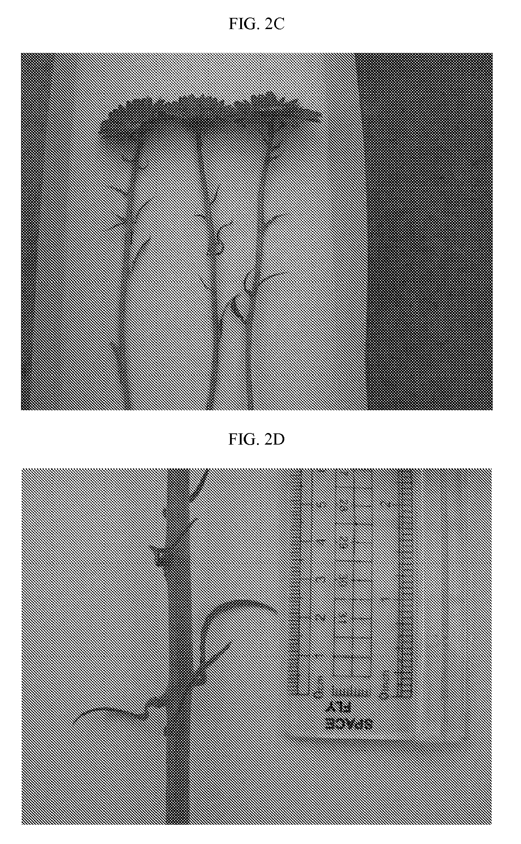

FIG. 2C shows a side view perspective and FIG. 2D shows a close-up side view perspective of typical leaves on the flower stem of *Gerbera L.* as described by Category 1 of Table 3.

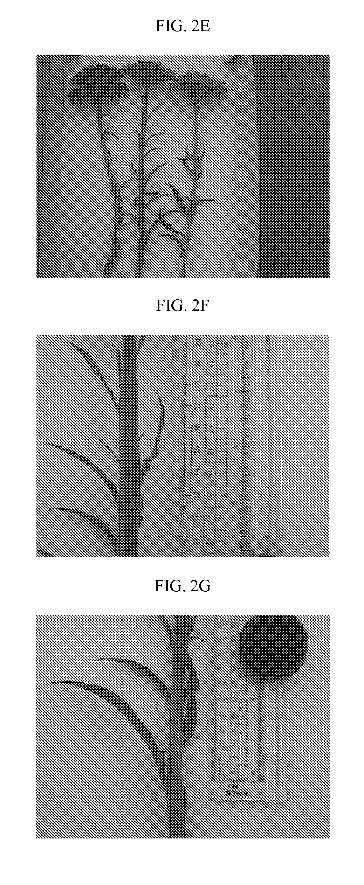

FIG. 2E shows a side view perspective of typical leaves on the flower stem of GERFOLIA™ *Gerbera,* FIG. 2F shows a close-up side view perspective of typical straight leaves on the flower stem of GERFOLIA™ *Gerbera L.,* and FIG. 2G shows a close-up side view perspective of curved, wrapped and twisted leaves on the flower stem of GERFOLIA™ *Gerbera L.,* as described by Category 2 of Table 3.

FIG. 2H shows a side view perspective and FIG. 21 shows a close-up side view perspective of typical leaves on the flower stem of GERFOLIA™ *Gerbera L.* as described by Category 3 of Table 3.

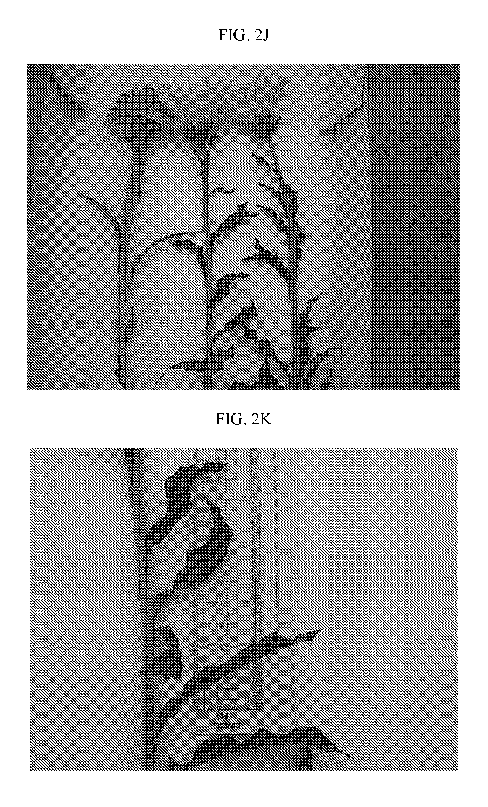

FIG. 2J shows a side view perspective and FIG. 2K shows a close-up side view perspective of typical leaves on the flower stem of GERFOLIA™ *Gerbera L.* as described by Category 4 of Table 3.

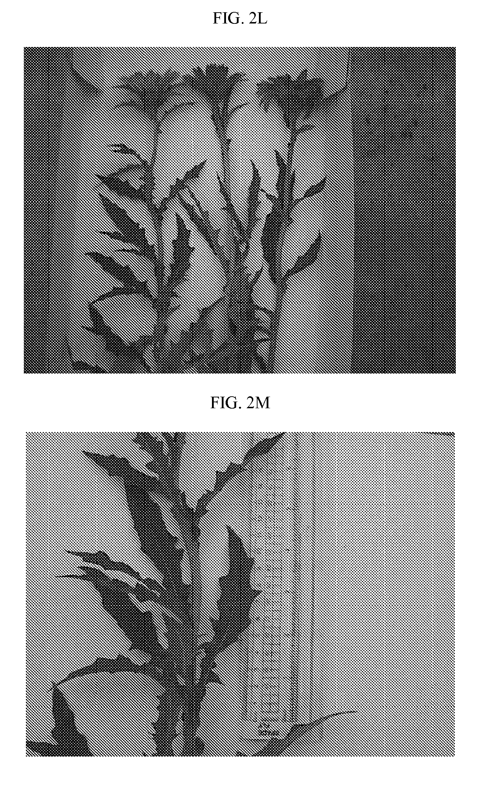

FIG. 2L shows a side view perspective and FIG. 2M shows a close-up side view perspective of typical leaves on the flower stem of GERFOLIA™ *Gerbera L.* as described by Category 5 of Table 3.

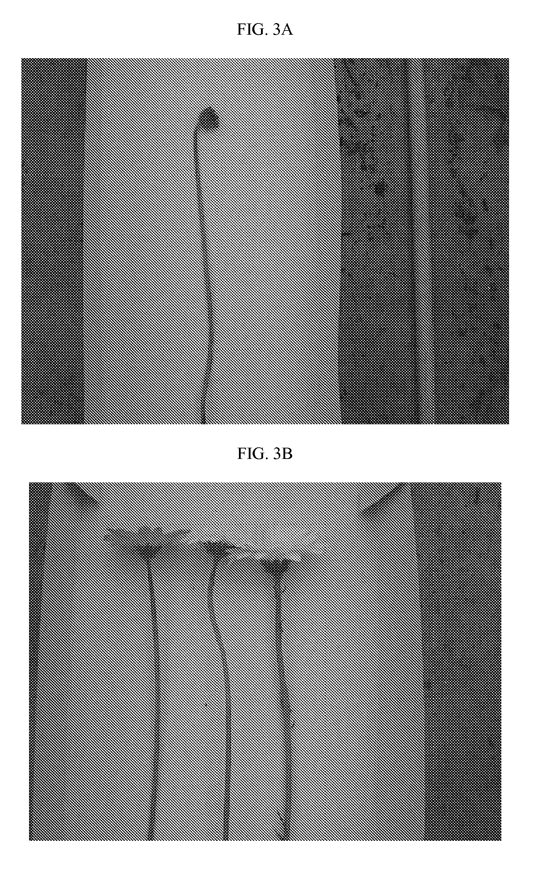

FIG. 3A shows a side view perspective of the distribution of typical leaves in the Bud Stage and FIG. 3B shows a side view perspective of the distribution of typical leaves in the Open Flower Stage of *Gerbera L.* as described by Category 0 of Table 4.

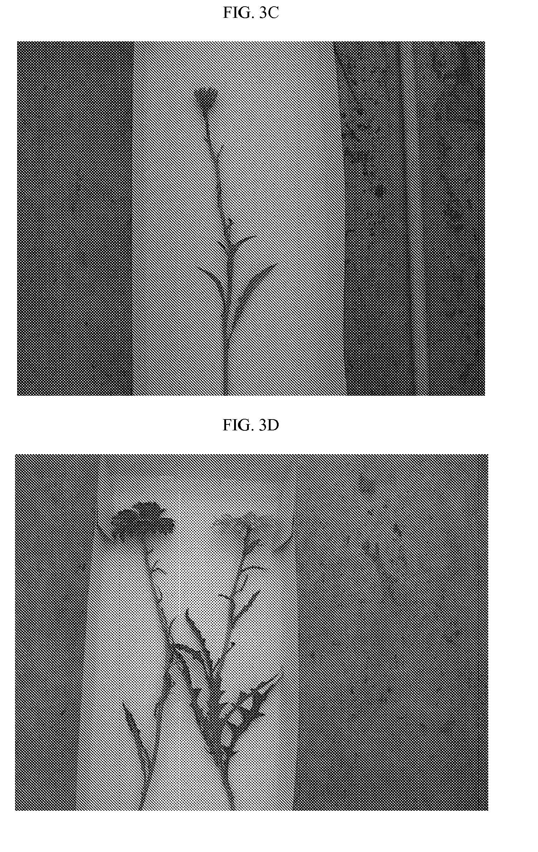

FIG. 3C shows a side view perspective of the distribution of typical leaves in the Bud Stage and FIG. 3D shows a side view perspective of the distribution of typical leaves in the Open Flower Stage of GERFOLIA™ *Gerbera L.* as described by Category1 of Table 4.

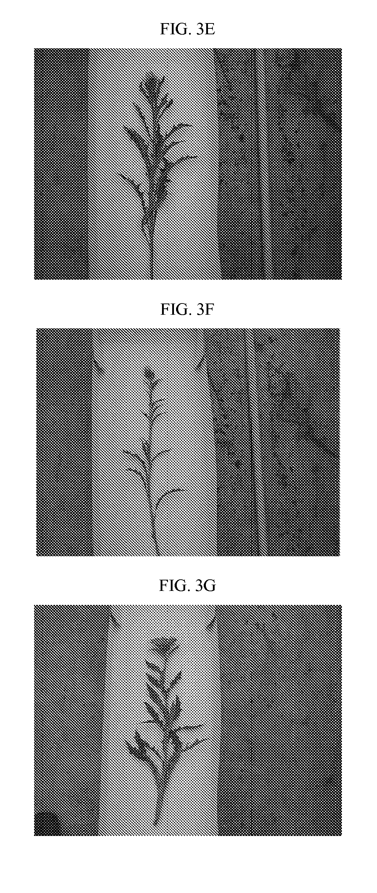

FIG. 3E shows a first example side view perspective of the distribution of typical leaves in the Bud Stage, FIG. 3F shows a second example side view perspective of the distribution of typical leaves in the Bud Stage, and FIG. 3G shows a side view perspective of the distribution of typical leaves in the Open Flower Stage of GERFOLIA™ *Gerbera* as described by Category 2 of Table 4.

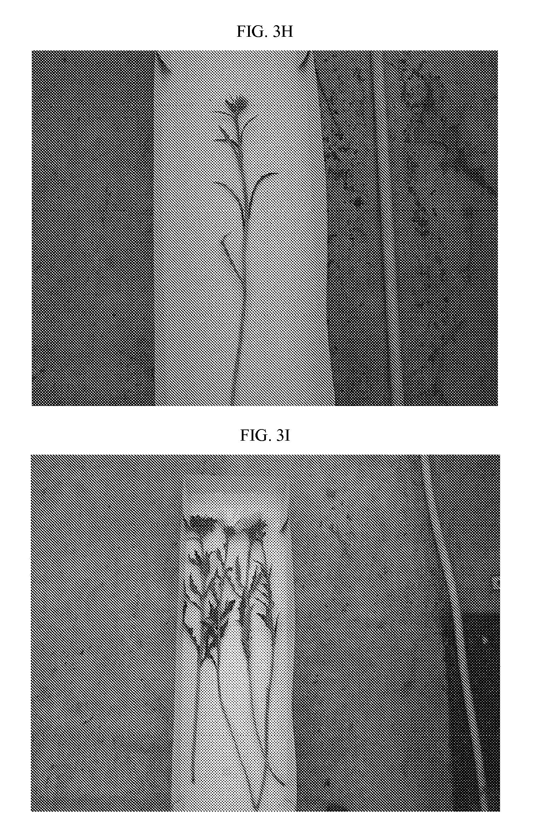

FIG. 3H shows a side view perspective of the distribution of typical leaves in the Bud Stage and FIG. 3I shows a side view perspective of the distribution of typical leaves in the Open Flower Stage of GERFOLIA™ *Gerbera L.* as described by Category 3 of Table 4.

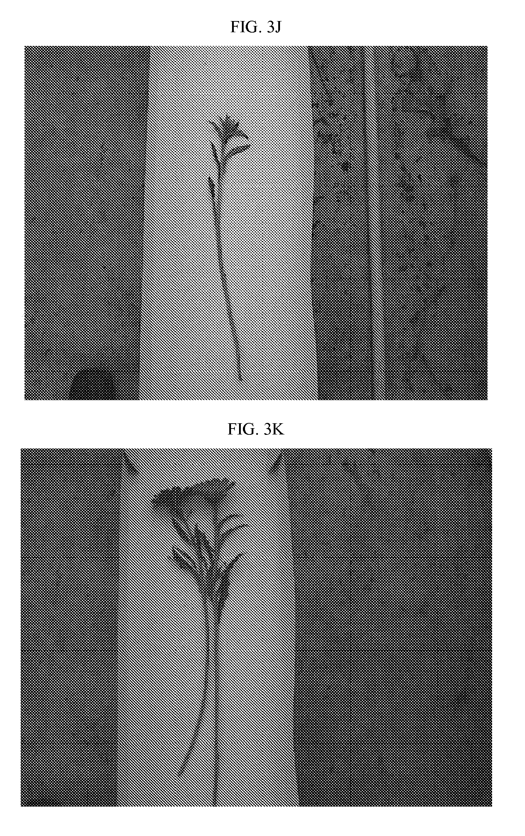

FIG. 3J shows a side view perspective of the distribution of typical leaves in the Bud Stage and FIG. 3K shows a side view perspective of the distribution of typical leaves in the Open Flower Stage of GERFOLIA™ *Gerbera L.* as described by Category 4 of Table 4.

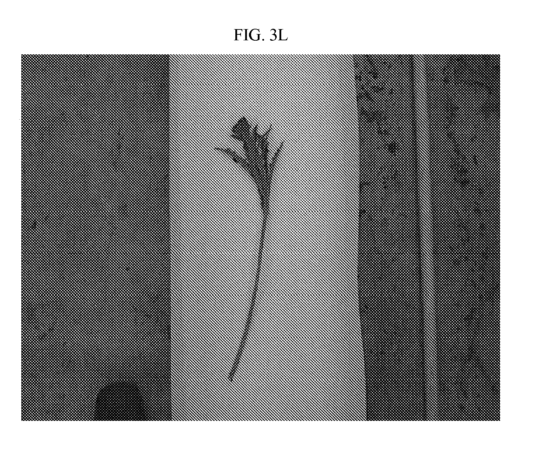

FIG. 3L shows a side view perspective of the distribution of typical leaves in the Bud Stage of GERFOLIA™ *Gerbera L.* as described by Category 5 of Table 4.

FIGS. 4A-4G show a close-up view perspective of the development stages of the leafy stem trait of GERFOLIA™ *Gerbera L..*

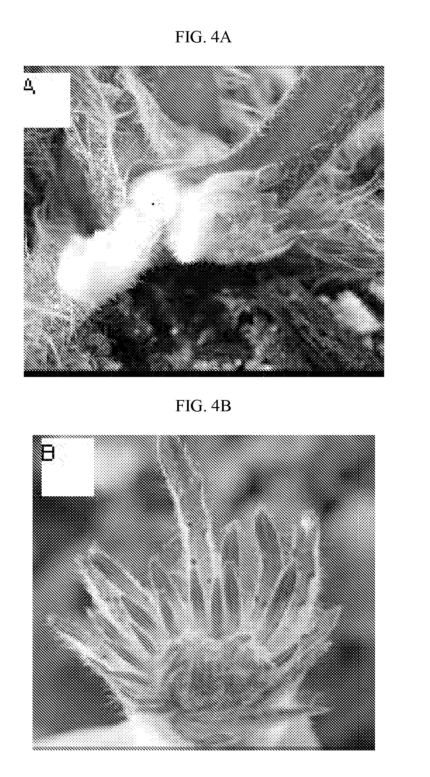

FIG. 4A and FIG. 4B show a close-up view of the small leaves, tightly surrounding the developing capitulum, which is covered with protective bracts during the early development of the scape.

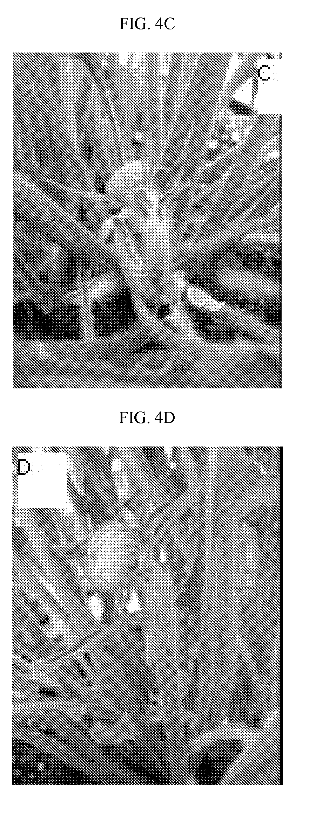

FIG. 4C shows a close-up view of the basal (lower) part of the scape which extends (quickly) and the leaves start to separate from each other, and FIG. 4D shows a close-up view of the upper part of the scape which then extends (slowly).

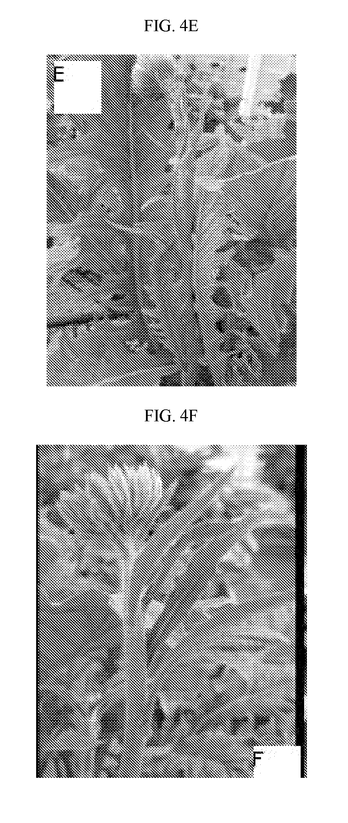

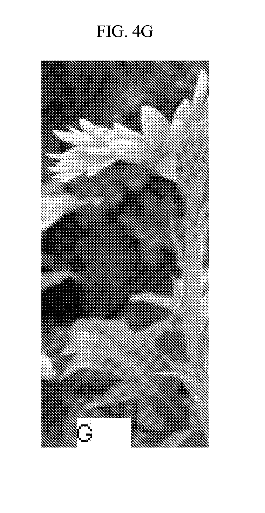

FIG. 4E, 4F and 4G show a close-up view of the leaves once the scape has extended to its final height.

FIG. 4E and 4F show a close up view of the lower and older leaves on the scape which grow to resemble rosette leaves, including a depiction of the mid veins as shown in FIG. 4F.

FIG. 4G shows a close-up view of the upper and younger leaves on the scape which are smaller and may seem to resemble bracts.

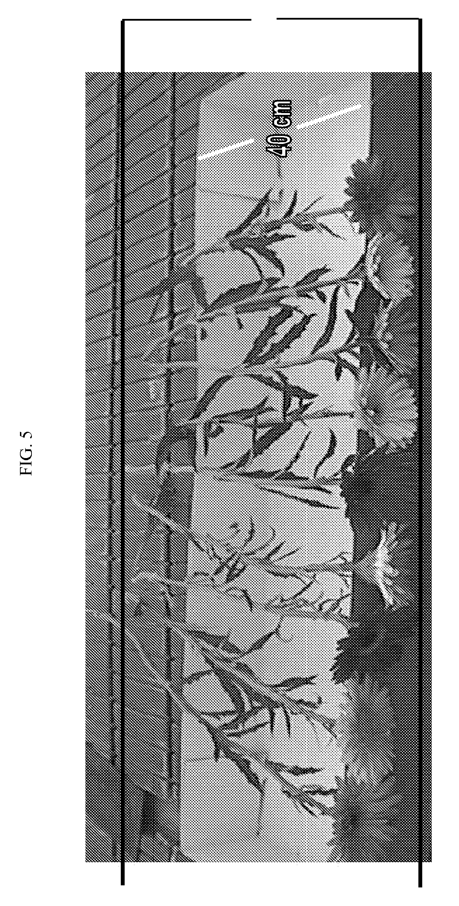

FIG. 5 shows a side view perspective of the leafy flower stem trait introgressed into the *Gerbera L.* typical flower stem length variation range of 60 cm to 70 cm.

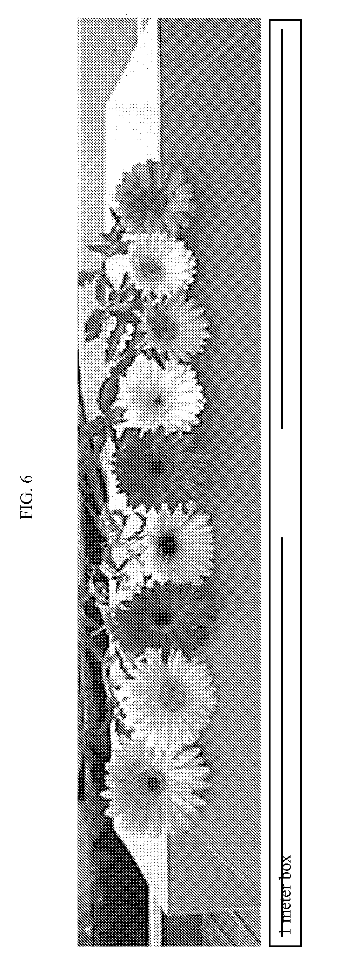

FIG. 6 shows a side view perspective of the leafy flower stem trait introgressed into the *Gerbera L.* typical flower head sizes and flower head colors.

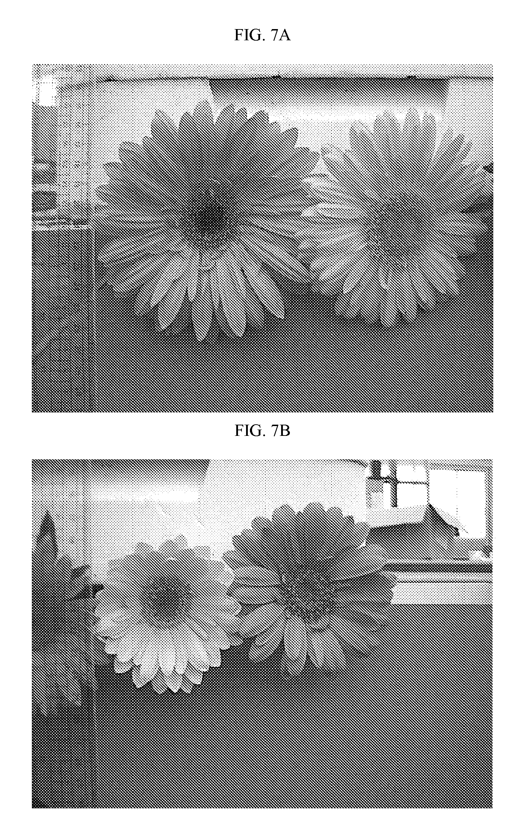

FIG. 7A shows a close-up side view perspective of the leafy flower stem trait introgressed into Normal *Gerbera* with typical flower head sizes ranging from 10 cm to 12 cm in diameter, and FIG. 7B shows a close-up side view perspective of the leafy flower stem trait introgressed into *MiniGerbera* with typical flower head sizes ranging from 7 cm to 9 cm in diameter.

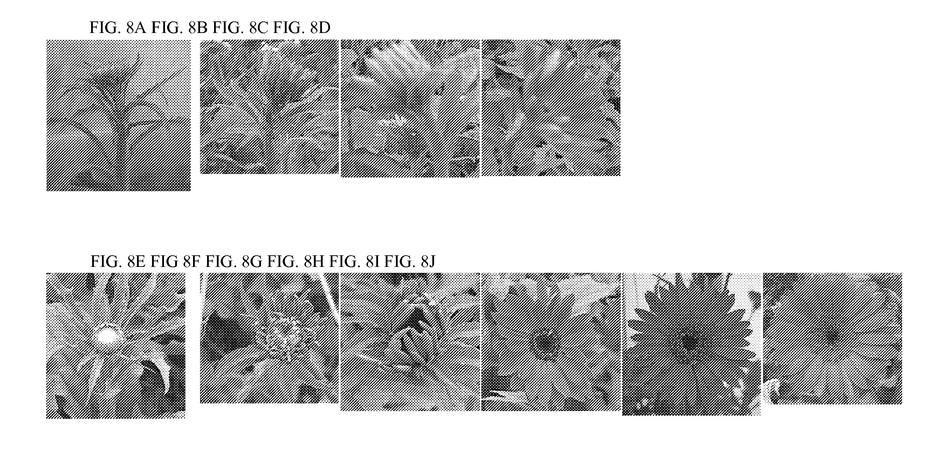

FIGS. 8A-8J show a close-up view perspective of the development stages of a bud of a GERFOLIA™ *Gerbera L..*

A close-up side view perspective of bud development of a GERFOLIA™ *Gerbera L.* during Stage 1 is shown in FIG. 8A, during Stage 2 is shown in FIG. 8B, during Stage 3 is shown in FIG. 8C, and during Stage 3.5 is shown in FIG. 8D.

A close-up top view perspective of bud development of a GERFOLIA™ Gerbera L. during Stage 1 is shown in FIG. 8E, during Stage 2 is shown in FIG. 8F, during Stage 3 is shown in FIG. 8G, and during Stage 3.5 is shown in FIG. 8H, during Stage 4 is shown in FIG. 8I and during Stage 5 is shown in FIG. 8J.

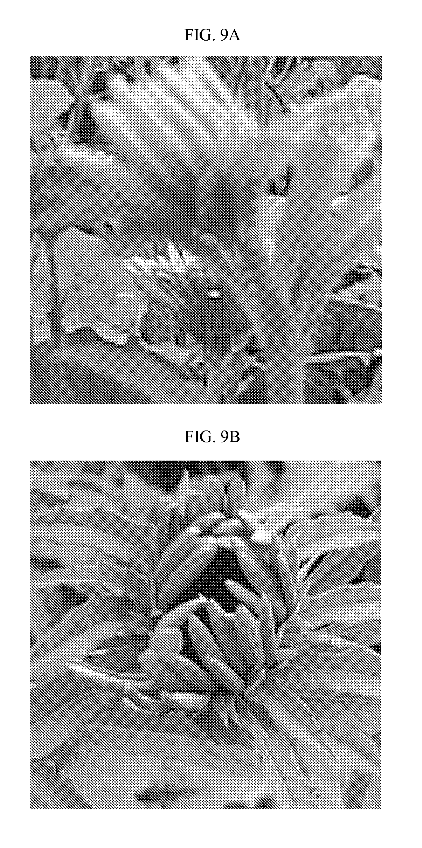

FIG. 9A shows a close-up side view perspective and FIG. 9B shows a close-up top view of a Stage 3 bud of a GERFOLIA™ *Gerbera L.* for shipping and transport.

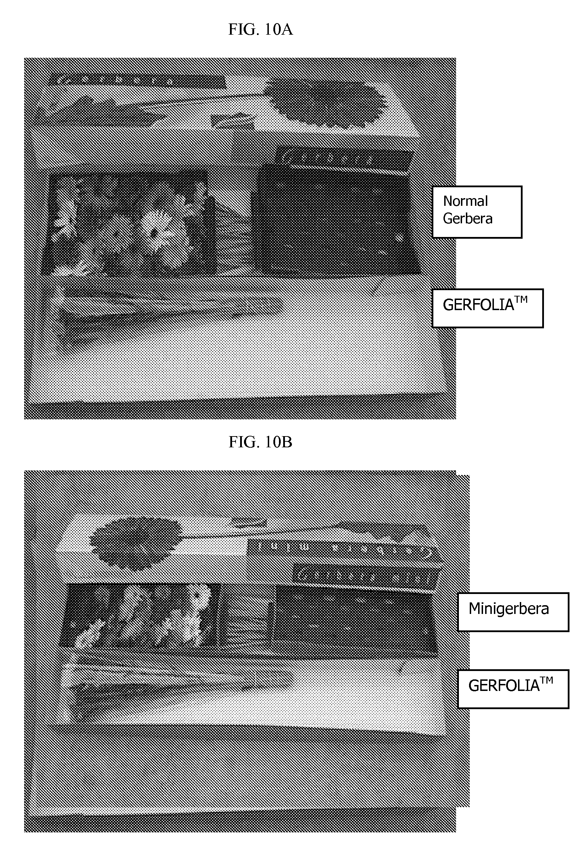

FIG. 10A shows a top view comparison of the packing density between Normal *Gerbera* and GERFOLIA™ *Gerbera* in a Dry Transport Box (10x30x12 cm) and

FIG. 10B shows a top view comparison of the packing density between *MiniGerbera* and GERFOLIA™ *Gerbera* in a Dry Transport Box (100x20x10 cm).

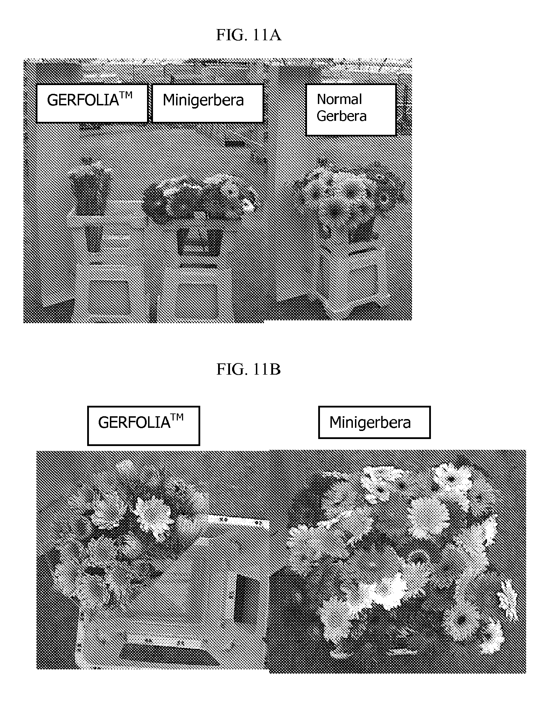

FIG. 11A shows a side view comparison of the packing density among GERFOLIA™ *Gerbera, MiniGerbera,* and Normal *Gerbera* in a Water Transport Container and FIG. 11B shows a top view comparison of the packing density between GERFOLIA™ *Gerbera* and *MiniGerbera* in a Water Transport Container.

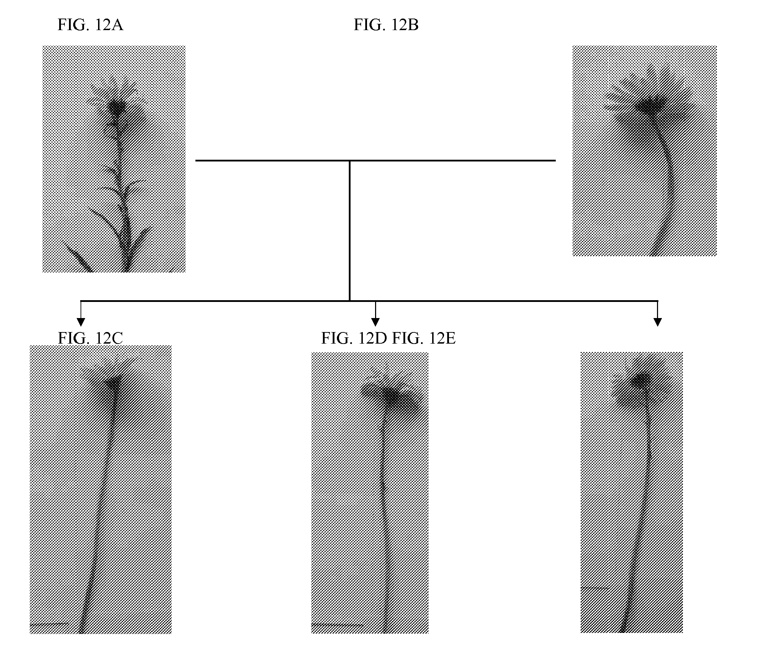

FIGS. 12A-12E show a close-up side view perspective of the 2 *Gerbera L.* parental seedlings and 3 selected *Gerbera L.* seedlings as provided in Crossing 1 of Table 19 showing successful introgression of the leafy stem trait into diverse *Gerbera L.* genetic backgrounds. FIG. 12A shows the mother *Gerbera L.* seedling '07.328', FIG. 12B shows the father *Gerbera L.* seedling '06.132', FIG. 12C shows Offspring 1-1, FIG. 12D shows Offspring 1-2, and FIG. 12E shows Offspring 1-3.

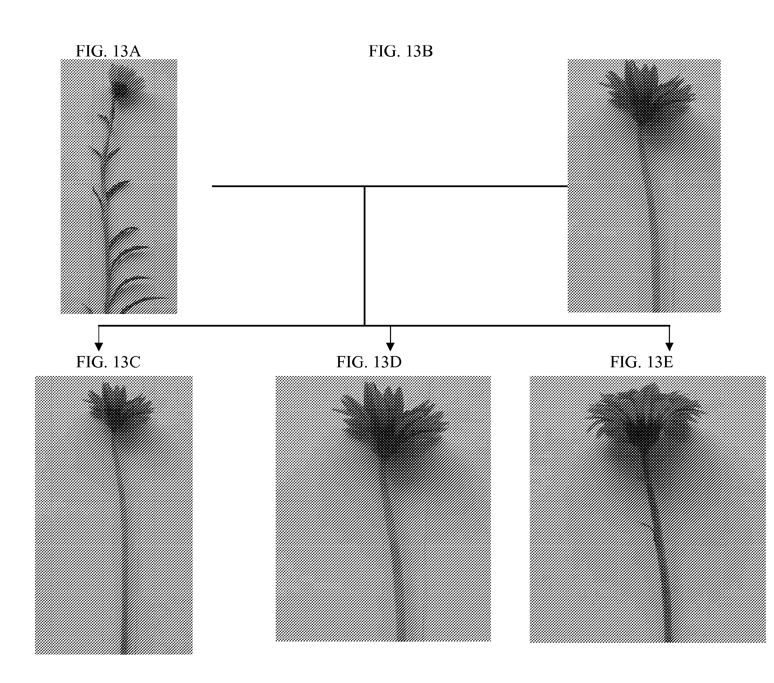

FIGS. 13A-13E show a close-up side view perspective of the 2 *Gerbera L.* parental seedlings and 3 selected *Gerbera L.* seedlings as provided in Crossing 2 of Table 19 showing successful introgression of the leafy stem trait into diverse *Gerbera L.* genetic backgrounds. FIG. 13A shows the mother *Gerbera L.* seedling 'BL06.702' (commercial name TERCARE), FIG. 13B shows the father *Gerbera L.* seedling 'M06.5 87', FIG. 13C shows Offspring 2-1, FIG. 13D shows Offspring 2-2, and FIG. 13E shows Offspring 2-3.

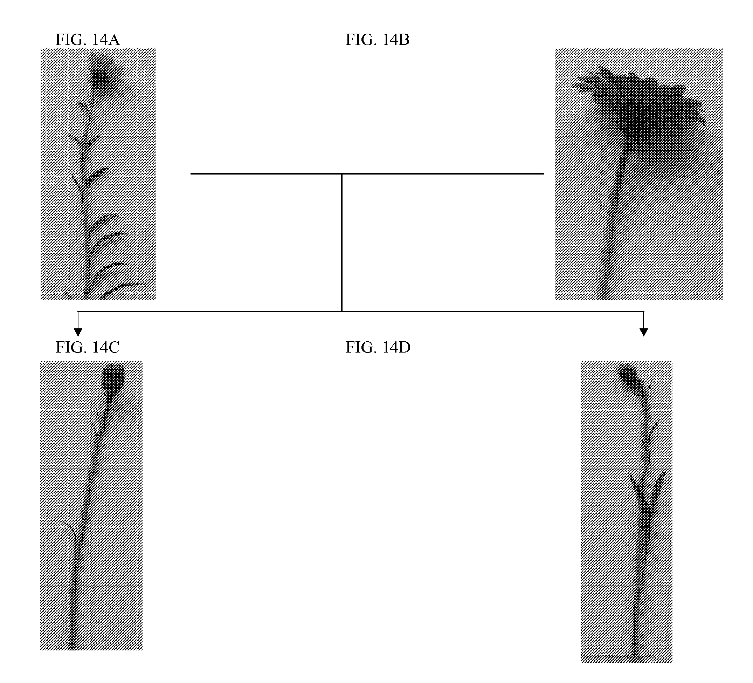

FIGS. 14A-14D show a close-up side view perspective of the 2 *Gerbera L.* parental seedlings and 2 selected *Gerbera L.* seedlings as provided in Crossing 3 of Table 19 showing successful introgression of the leafy stem trait into diverse *Gerbera L.* genetic backgrounds. FIG. 14A shows the mother *Gerbera L.* seedling 'BL06.702' (commercial name TERCARE), FIG. 14B shows the father *Gerbera L.* seedling 'M05.516', FIG. 14C shows Offspring 3-1, FIG. 14D shows Offspring 3-2.

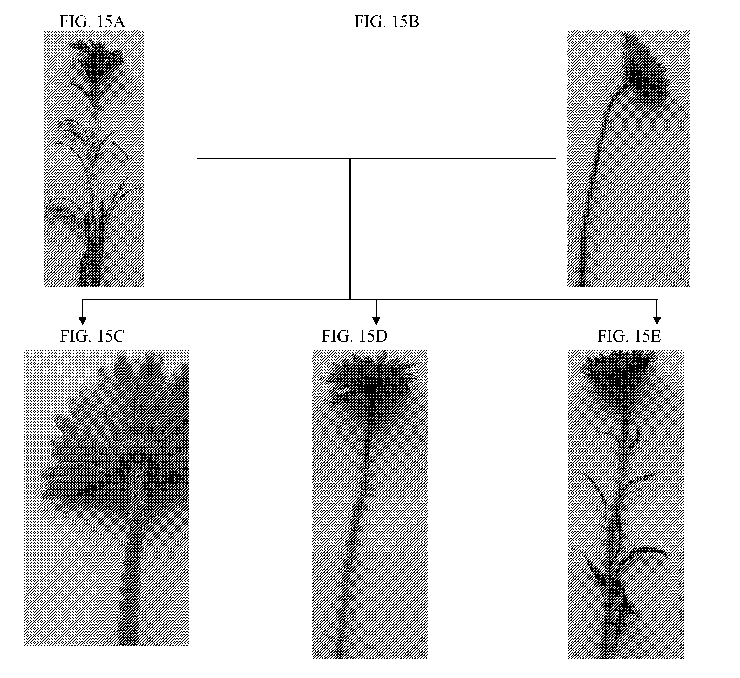

FIGS. 15A-15E show a close-up side view perspective of the 2 *Gerbera L.* parental seedlings and 3 selected *Gerbera L.* seedlings as provided in Crossing 4 of Table 19 showing successful introgression of the leafy stem trait into diverse *Gerbera L.* genetic backgrounds. FIG. 15A shows the mother *Gerbera L.* seedling 'BL06.706' (commercial name TERTRUE), FIG. 15B shows the father *Gerbera L.* seedling '03.113', FIG. 15C shows Offspring 4-1, FIG. 15D shows Offspring 4-2, and FIG. 15E shows Offspring 4-3.

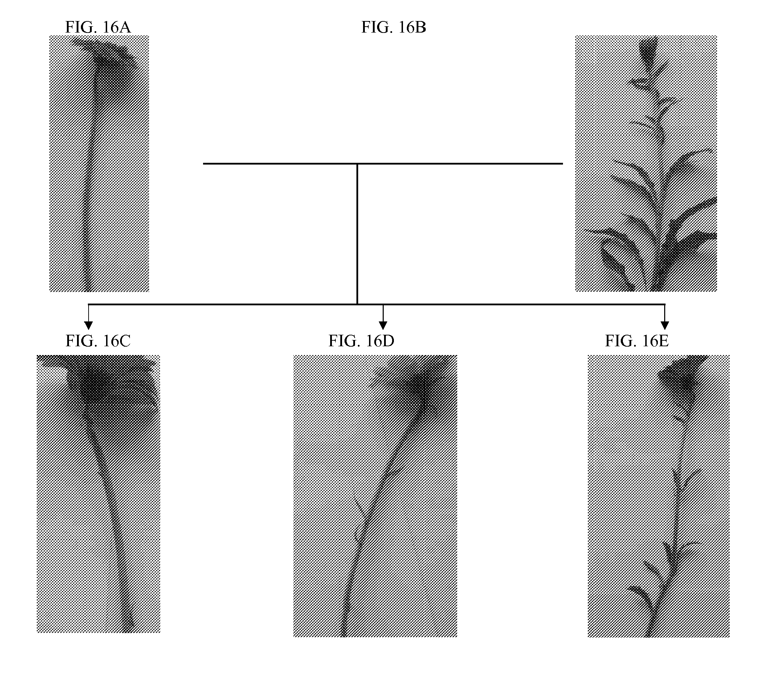

FIGS. 16A-16D show a close-up side view perspective of the 2 *Gerbera L.* parental seedlings and 3 selected *Gerbera L.* seedlings as provided in Crossing 5 of Table 19 showing successful introgression of the leafy stem trait into diverse *Gerbera L.* genetic backgrounds. FIG. 16A shows the mother *Gerbera L.* seedling 'M01.548' (commercial name TORPEDO), FIG. 16B shows the father *Gerbera L.* seedling 'BL07.774', FIG. 16C shows Offspring 5-1, FIG. 16D shows Offspring 5-2, and FIG. 16E shows Offspring 5-3.

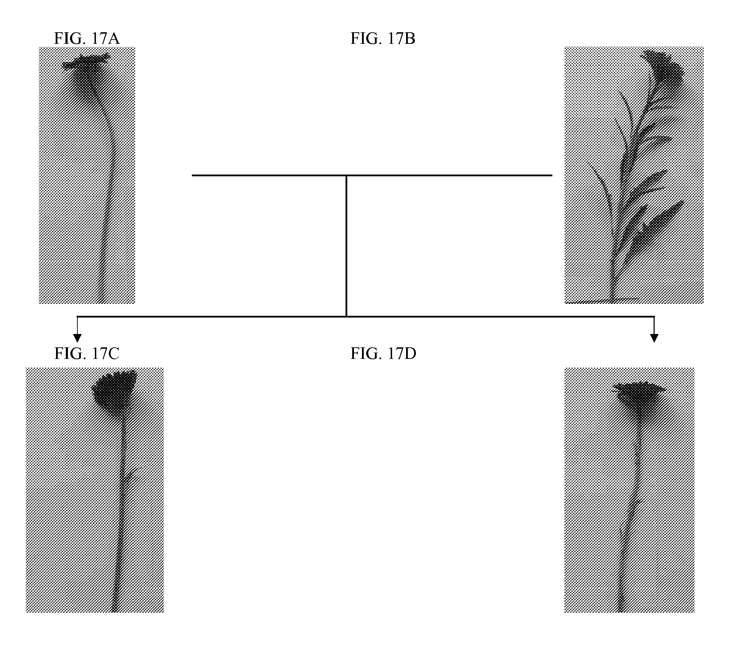

FIGS. 17A-17E show a close-up side view perspective of the 2 *Gerbera L.* parental seedlings and 2 selected *Gerbera L.* seedlings as provided in Crossing 6 of Table 19 showing successful introgression of the leafy stem trait into diverse *Gerbera L.* genetic backgrounds. FIG. 17A shows the mother *Gerbera L.* seedling 'M06.509', FIG. 17B shows the father *Gerbera L.* seedling 'BL07.790', FIG. 17C shows Offspring 6-1 and FIG. 17D shows Offspring 6-2.

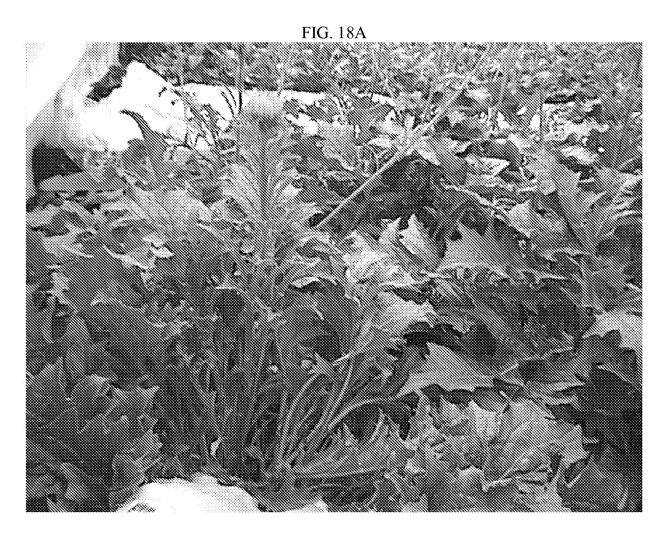

FIG. 18A shows a side view perspective, in the bud stage, of an individual GERFOLIA™ *Gerbera L.* plant within the BLK06-11 crossing as described in Table 21, as grown under greenhouse conditions.

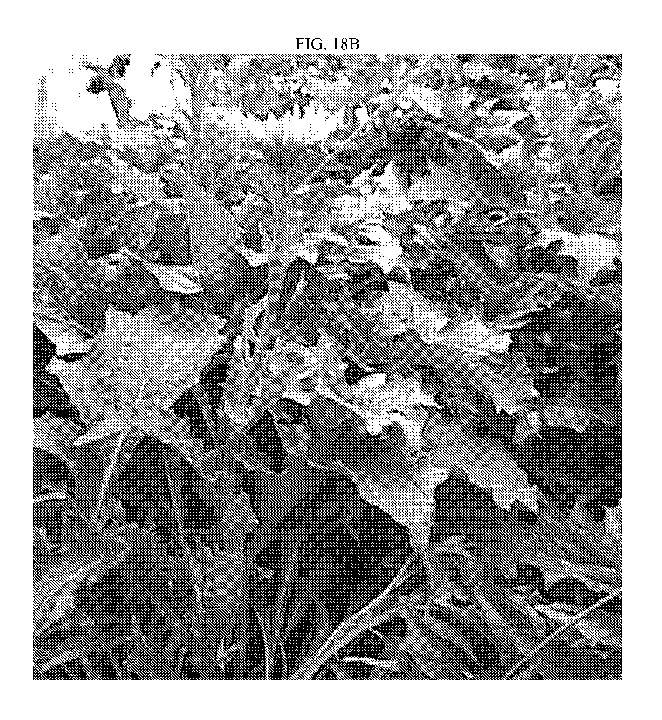

FIG. 18B shows a side view perspective, in the open flower stage, of an individual GERFOLIA™ *Gerbera L.* plant within the BLK06-11 crossing as described in Table 21, as grown under greenhouse conditions.

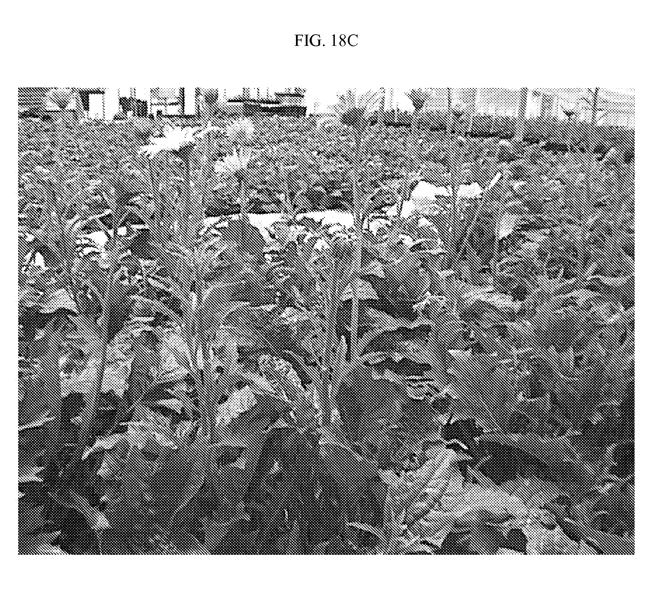

FIG. 18C shows a side view perspective of individual GERFOLIA™ *Gerbera L.* plants within the BLK06-11 crossing as described in Table 21, as grown under greenhouse conditions.

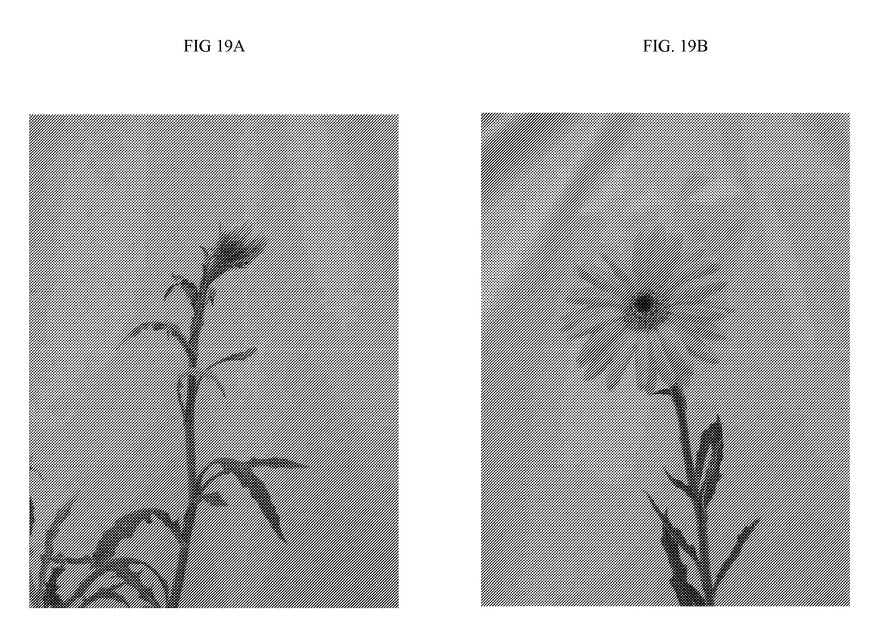

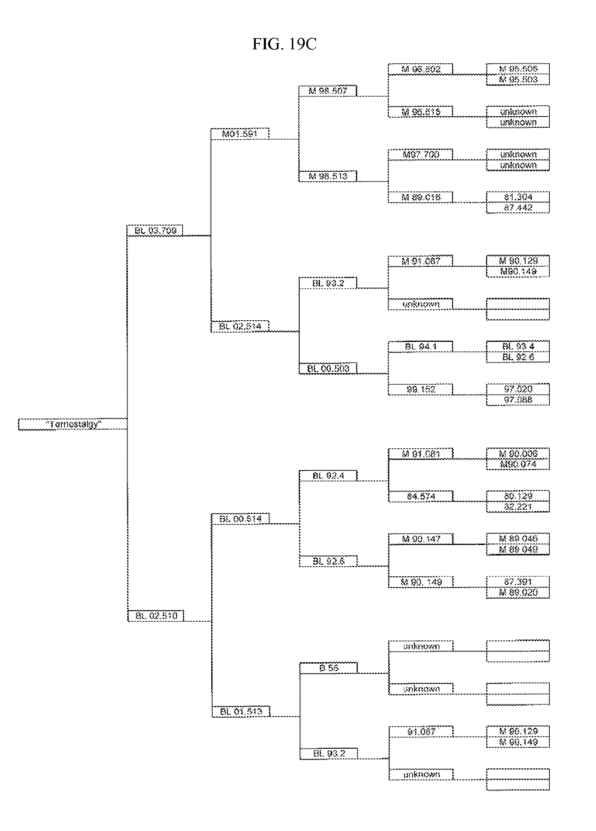

FIG. 19A shows a side view perspective, in the bud stage, of an individual inflorescence and flower stem of GERFOLIA™ *Gerbera jamesonii* designated 'TERNOSTALGY', at 5 weeks of age, as grown under greenhouse conditions, and FIG. 19B shows a side view perspective, in the open flower stage, of an individual inflorescence and flower stem of GERFOLIA™ *Gerbera jamesonii* designated 'TERNOSTALGY', at 6 weeks of age, as grown under greenhouse conditions. FIG. 19C provides genealogy of GERFOLIA™ *Gerbera jamesonii* designated 'TERNOSTALGY'.

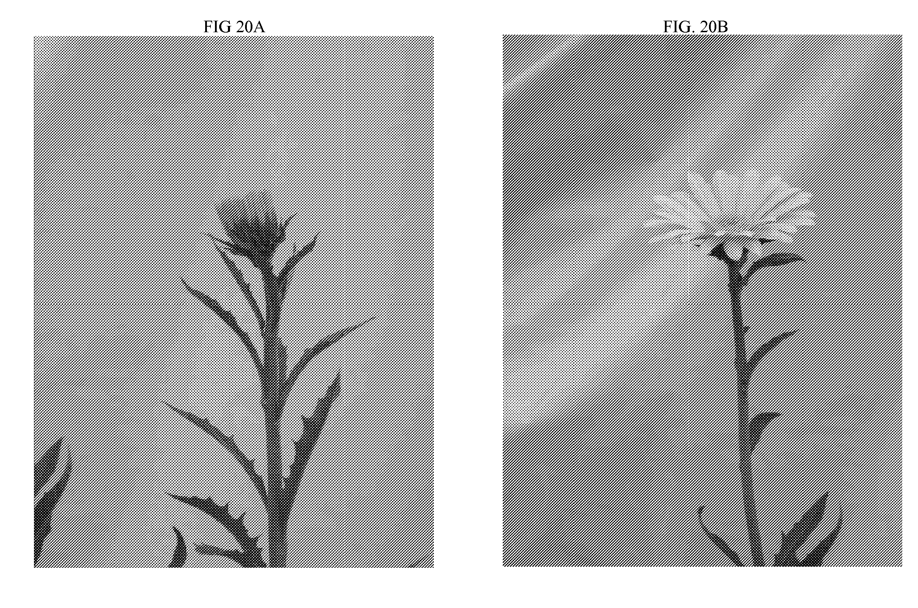

FIG. 20A shows a side view perspective, in the bud stage, of an individual inflorescence and flower stem of GERFOLIA™ *Gerbera jamesonii* designated 'TERZORG', at 5 weeks of age, as grown under greenhouse conditions, and FIG. 20B shows a side view perspective, in the open flower stage, of an individual inflorescence and flower stem of GERFOLIA™ *Gerbera jamesonii* designated 'TERZOG', at 6 weeks of age, as grown under greenhouse conditions.

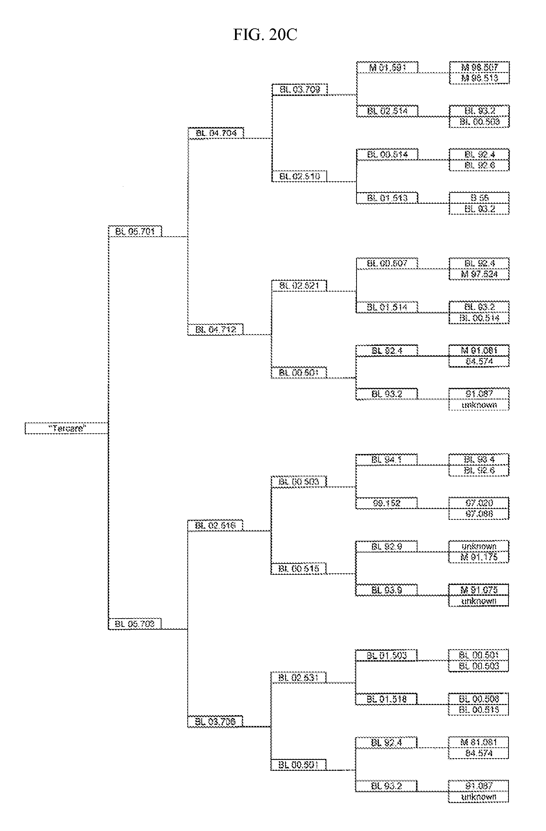

FIG. 20C provides genealogy of GERFOLIA™ *Gerbera jamesonii* designated 'TERZOG'.

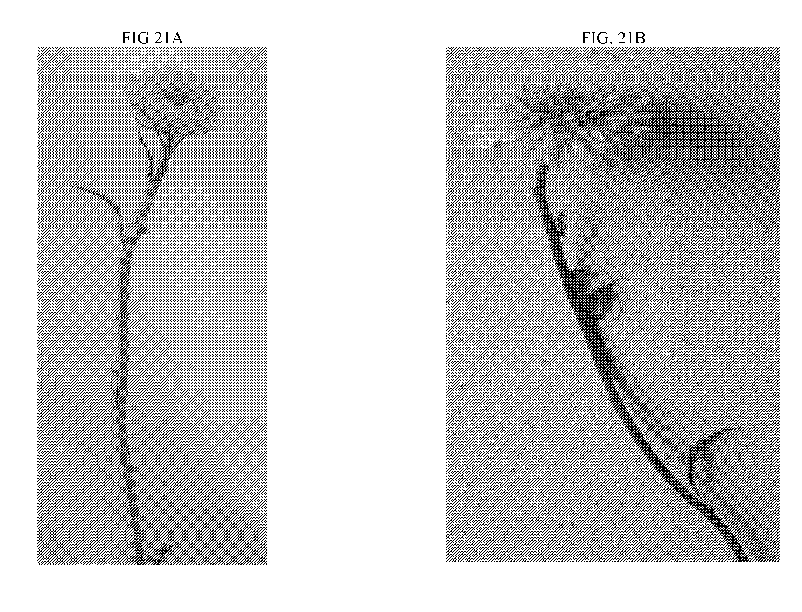

FIG. 21A shows a side view perspective, in the bud stage, of an individual inflorescence and flower stem of GERFOLIA™ *Gerbera jamesonii* designated 'BL02.528', at 5 weeks of age, as grown under greenhouse conditions, and FIG. 21B shows a side view perspective, in the open flower stage, of an individual inflorescence and flower stem of GERFOLIA™ *Gerbera jamesonii* designated 'BL02.528', at 6 weeks of age, as grown under greenhouse conditions.

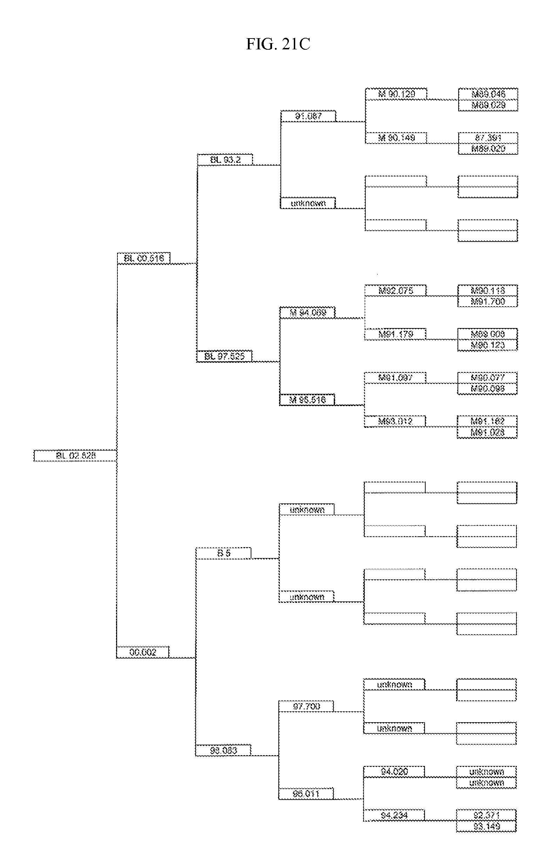

FIG. 21C provides genealogy of GERFOLIATM *Gerbera jamesonii* designated 'BL02.528'.

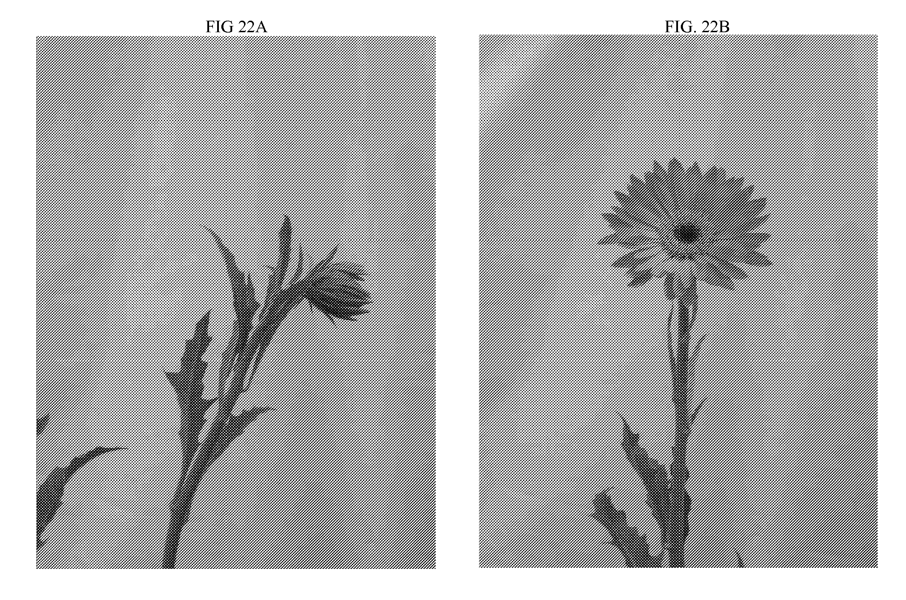

FIG. 22A shows a side view perspective, in the bud stage, of an individual inflorescence and flower stem of GERFOLIA™ *Gerbera jamesonii* designated 'TERTRUE', at 5 weeks of age, as grown under greenhouse conditions, and FIG. 22B shows a side view perspective, in the open flower stage, of an individual inflorescence and flower stem of GERFOLIA™ *Gerbera jamesonii* designated 'TERTRUE', at 6 weeks of age, as grown under greenhouse conditions.

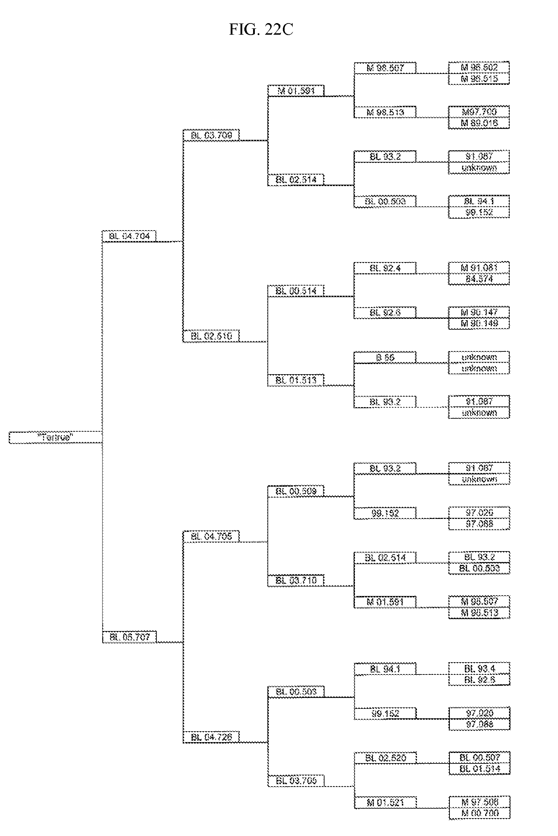

FIG. 22C provides genealogy of GERFOLIA™ *Gerbera jamesonii* designated 'TERTRUE'.

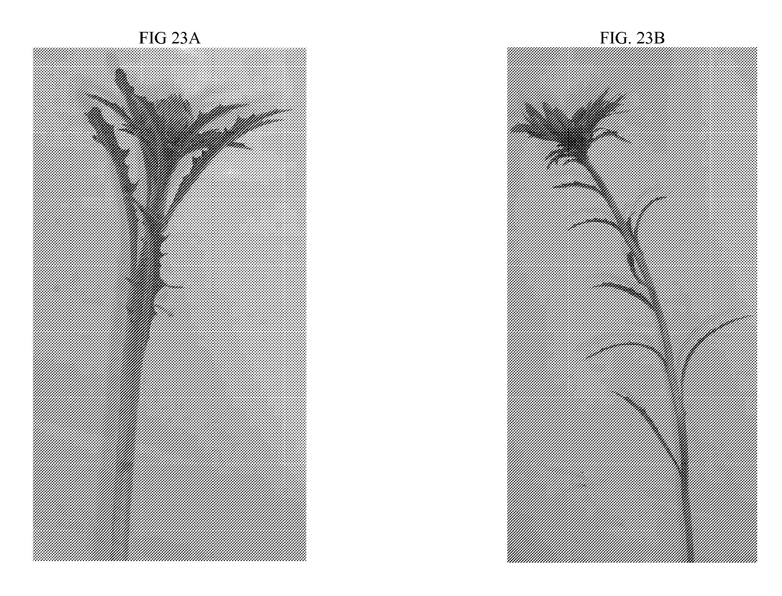

FIG. 23A shows a side view perspective, in the bud stage, of an individual inflorescence and flower stem of GERFOLIA™ *Gerbera jamesonii* designated 'BL07.777', at 5 weeks of age, as grown under greenhouse conditions, and FIG. 23B shows a side view perspective, in the open flower stage, of an individual inflorescence and flower stem of GERFOLIA™ *Gerbera jamesonii* designated 'BL07.777', at 6 weeks of age, as grown under greenhouse conditions.

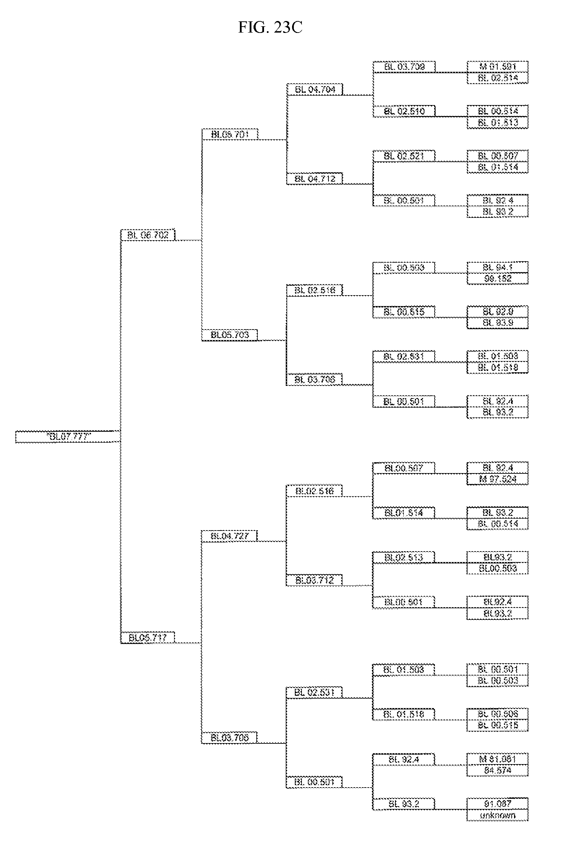

FIG. 23C provides genealogy of GERFOLIA™ *Gerbera jamesonii* designated 'BL07.777'.

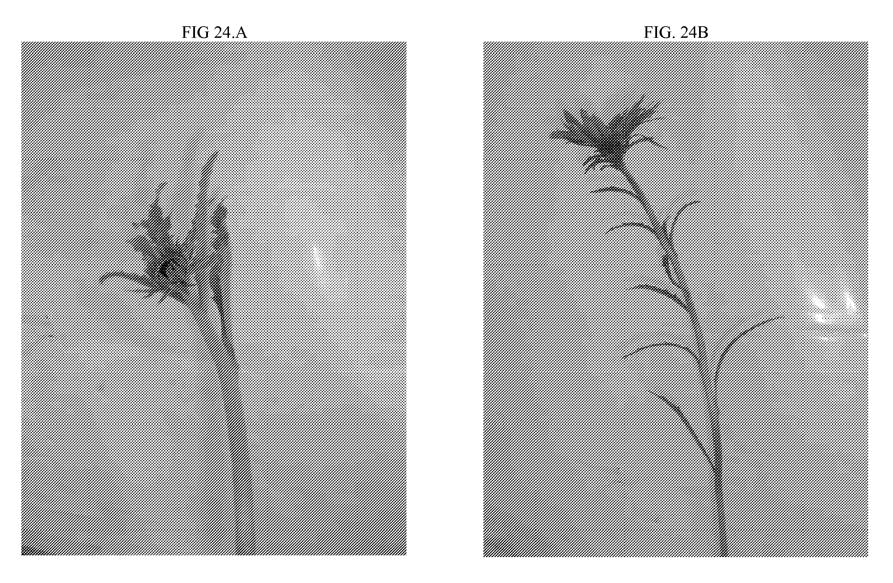

FIG. 24A shows a side view perspective, in the bud stage, of an individual inflorescence and flower stem of GERFOLIA™ *Gerbera jamesonii* designated 'BL07.790', at 5 weeks of age, as grown under greenhouse conditions, and FIG. 24B shows a side view perspective, in the open flower stage, of an individual inflorescence and flower stem of GERFOLIA™ *Gerbera jamesonii* designated 'BL07.790', at 6 weeks of age, as grown under greenhouse conditions.

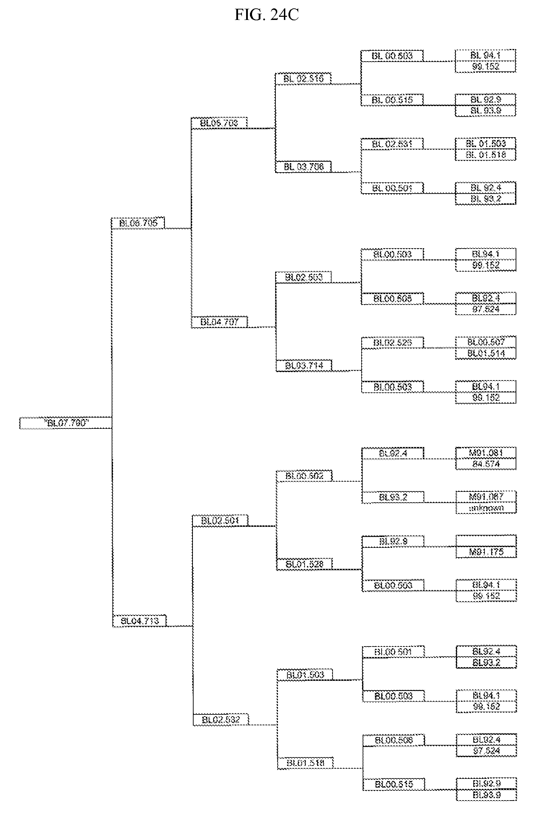

FIG. 24C provides genealogy of GERFOLIA™ *Gerbera jamesonii* designated 'BL07.790'.

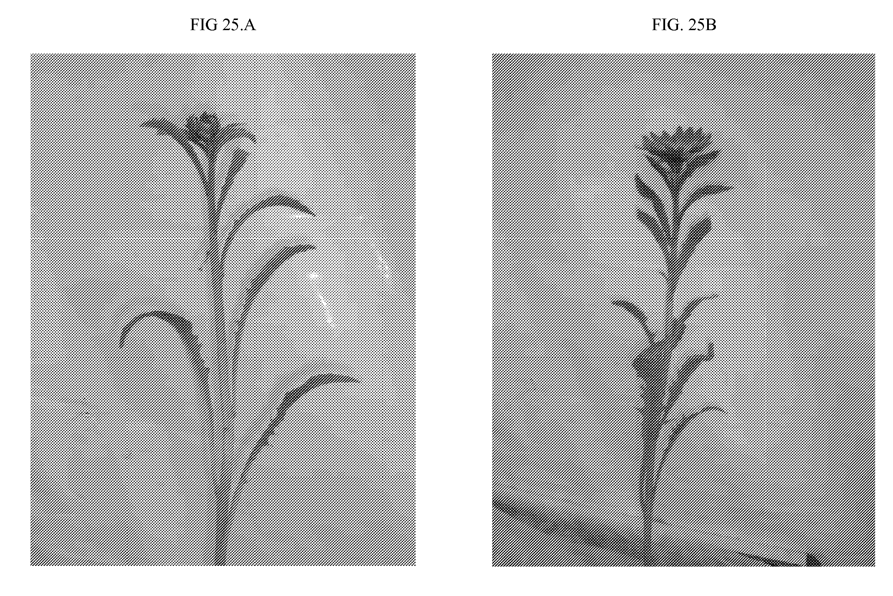

FIG. 25A shows a side view perspective, in the bud stage, of an individual inflorescence and flower stem of GERFOLIA™ *Gerbera jamesonii* designated 'BL07.774', at 5 weeks of age, as grown under greenhouse conditions, and FIG. 25B shows a side view perspective, in the open flower stage, of an individual inflorescence and flower stem of GERFOLIA™ *Gerbera jamesonii* designated 'BL07.774', at 6 weeks of age, as grown under greenhouse conditions.

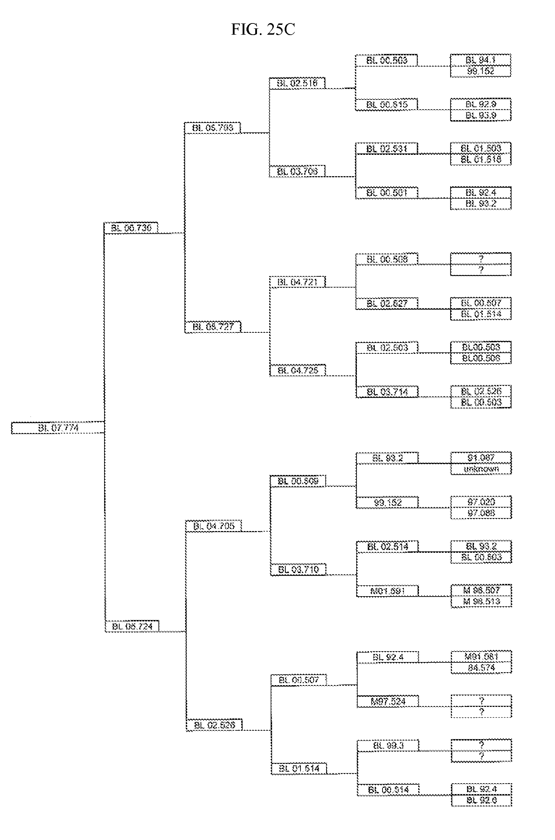

FIG. 25C provides genealogy of GERFOLIA™ *Gerbera jamesonii* designated 'BL07.790'.

EP 2 011 388 A1

DETAILED DESCRIPTION OF THE INVENTION

1. Definitions

In the description and tables which follow, a number of terms are used. In order to provide a clear and consistent understanding of the present invention, the following definitions are provided:

*Inflorescence* is defined as a type of flower in which there is more than one flower in a single structure.

*Flower Head* is defined as a composite inflorescence type which contains a dense, indeterminate mass of small, individual flowers (often called florets) that together appear to form a single flower. The compact cluster of florets are sessile or subsessile and are crowded on a compound receptacle, sometimes called a capitulum. The capitulum of an *Asteraceae/Compositae* can be composed of hundreds of individual flowers even though the flower head may appear to be only a single flower.

*Perianth* is defined as the part of the flower which surrounds the reproductive organs, generally the calyx (containing the sepals) and the corolla (containing the petals).

*Calyx* is defined as the outermost part of the perianth of a flower. The calyx is usually small and green but sometimes can be showy and brightly colored. The calyx of a flower is formed from the sepals (small leaves located directly under the flower) and which encloses the petals in a bud.

*Corolla* is defined as the inner part of the perianth of a flower.. If the units of the corolla are separate, they are called petals, and the corolla is said to be polypetalous.

*Pappus* is defined as the bristles, teeth or scales which surround the perianth of a composite flower.

*Petal* is defined as a unit of the corolla, which are usually colored or more or less showing. When the flower is open, the petals are located between the sepals and the flower's reproductive organs. In a flower head, the petals of a corolla arise from the same point and form a circle or circles which can be referred to as a whorl or whorls.

*Floret* is defined as a very small, individual flower in a flower head, especially when part of a dense inflorescence, such as *Composite.* Plants in the *Asteraceae/Compositae* family typically include one or both types of florets: ray florets and disc florets. Ray florets and disc florets of *Asteraceae/Compositae* plants are actinomorphic.

*Ray Floret* is defined as the "petal-like" part of a flower head which is characterized as long and strap-like (also referred to as a ligule). In a flower head of *Asteraceae/Compositae* plants, the outermost whorl or whorls of "petal-like" ligules are ray florets. In this invention, ray florets are sometimes referred to as "outer petals".

*Disc Floret* is defined as a small "petal-like" flower with a tubular corolla which is located in the inner whorl of a flower head of *Asteraceae/Compositae* plants. In this invention, disc florets are sometimes referred to as "inner petals". The color of the center of a *Gerbera L.* flower is determined by the combination of the disc florets (not specifically the color of the disc florets but rather the height of the disc florets in a particular stage of development) and the color of the pappus. The intensity of the color of the center of a *Gerbera L.* flower is determined by the position of the pappus in relation to the disc florets. The color of the center of a *Gerbera L.* flower may be black or green/yellow.

*A cultivar* or *variety* is a group of similar plants which belong to the same species and which by structural features and performance may be distinguished from other varieties within the same species. Two essential characteristics of a variety are identity and reproducibility. Identity is necessary so that the variety may be recognized and distinguished from other varieties within the crop species. The distinguishing features may be morphological characteristics, color markings, physiological functions, disease reaction, or performance. Most agricultural varieties are pure for those characteristics which identify the variety. Reproducibility is needed so that the characteristics by which the variety is identified will be reproduced in the progeny. A variety is derived from a strain; populations which are increased from a single genotype or a mixture of genotypes are referred to as strains, experimental strains, or lines. Once a strain is identified as superior, it may be named, increased, and made available commercially as a "cultivated variety" or "cultivar." The words "variety" and "cultivar" are used interchangeably, although cultivar is commonly used in scientific literature while variety is the term used by U.S. farmers and the seed trade.

A *consumer* refers to any entity, whether individual or corporate, which purchases *Gerbera L.* flowers in the commercial market. A consumer can refer to a distributor or middleman of the *Gerbera L.* commercial market. Consumers can purchase *Gerbera L.* flowers in a local or international market. Consumers can also purchase *Gerbera L.* flower wherever they may be displayed, i.e. in the home, in a store, in a hotel, in a business, in a museum, or in a nursery.

*A Gerbera L. cultivar* refers to any plant of the *Gerbera* genus, and includes more than 40 different species, including *G. aberdarica, G. abyssinica, G. ambigua, G. anandria, G. aspleniifolia, G. aurantiaca, G. bojeri, G. bonatiana, G. bracteata, G. brevipes, G. burchellii, G. burmanni, G. candollei, G. cavaleriei, G. chilensis, G. cineraria, G. connata, G. conrathii, , G. cordata, G. coronopifolia, G. curvisquama, G. delavayi, G. discolor, G. diversifolia, G. elegans, G. elliptica, G. emirnensis, G. ferruginea, G. flava, G. galpinii, G. glandulosa, G. henryi, G. hieracioides, G. hirsuta, G. hypochaeridoides, G. integralis, G. integripetala, G. jamesonii, G. knorringiana, G. kokanica, G. kraussii, G. kunzeana, G. lacei, G. lagascae, G. lanuginosa, G. lasiopus, G. leandrii, G. leiocarpa, G. leucothrix, G. lijiangensis, G. lynchii, G. macrocephala, G. nepalensis, G. nervosa, G. nivea, G. parva, G. peregrina, G. perrieri, G. petasitifolia, G. piloselloides,*

*G. plantaginea, G. plicata, G. podophylla, G. pterodonta, G. pulvinata, G. pumila, G. randii, G. raphanifolia, G. ruficoma, G. saxatilis, G. semifloscularis, G. serotina, G. speciosa, G. tanantii, G. tomentosa, G. tuberosa, G. uncinata, G. viridifolia, G. welwitschii, G. wrightii.* Further, in the present invention, a *Gerbera L. cultivar* expresses the new leafy flower stem trait, in combination with any other trait(s) within the phenotypic range of characteristics available in the *Gerbera* commercial market, including but not limited to, any desirable trait such as flowering type, flower shape, flower color, flower size, flower quality, ray floret and disc floret shape and size, flower stem length, flower stem quality, flower stem productivity, and vase life.

*Flowering type* is defined for a *Gerbera L.* plant by the number of full or partial outer petals (ray florets) produced by one or more flowers of a *Gerbera L.* plant. The different flowering types of *Gerbera L.* plants include: single, semi-double, double, double-multi and multi-petalled as defined below.

*Single (s)* or *single-flowering* or *single-type* are each defined as a *Gerbera L.* plant which produces one or more flowers having at least 50, to about 75, full or partial outer petals (ray florets) per flower or a *Gerbera L.* flower which has at least 50, to about 75, full or partial outer petals (ray florets).

*Semi-double (sd)* or *semi-double-flowering* or *semi-double-type* are each defined as a *Gerbera L.* plant which produces one or more flowers having at least 130, to about 170, full or partial outer petals (ray florets) per flower or a *Gerbera L.* flower which has at least 130, to about 170, full or partial outer petals (ray florets).

*Double (d)* or *double-flower* or *double-type* are each defined as a *Gerbera L.* plant which produces one or more flowers having at least 250, to about 270, full or partial outer petals (ray florets) per flower, or a *Gerbera L.* flower which has at least 250, to about 270, full or partial outer petals (ray florets).

*Double-multi (dm)* or *double-multi-flowering* or *double-multi-type* are each defined as a *Gerbera L.* plant which produces one or more flowers having at least 270, to about 350, full or partial outer petals (ray florets) per flower stem, or a *Gerbera L.* flower which has at least 270, to about 350, full or partial outer petals (ray florets). All double-multi-types are half products.

*Multi-petalled (mp)* or *multi-petalled-flowering* or *multi-petalled-type* are each defined as a *Gerbera L.* plant which produces one or more flower having at least 350, to about 550, full or partial outer petals (ray florets) per flower stem or a *Gerbera L.* flower stem which has at least 350, to about 550, full or partial outer petals (ray florets). The multi-petalling trait does not express itself to the fullest extent until after two generations.

*Flower shape* is defined for a *Gerbera L.* plant as the overall shape of the inflorescence of a *Gerbera L.* plant. The flower shape of a *Gerbera L.* plant may differ based on the flowering type, as well as, the outer petals (ray florets) shape and size. The shape of outer petals (ray florets) of *Gerbera L.* plants may be referred to, but is not limited to, the following descriptive terms: flat, slightly reflexed, frilly (looking ragged or shredded), curled inwards, fully incurving or twisted. The flower shape of *Gerbera L.* plants may be referred to, but is not limited to the following terms: flat, daisy-shaped, double-flower-shaped, globular-shaped, spider-shaped, or mono-bouquet.

*Inflorescence type* is defined for a *Gerbera L.* plant by the diameter size of the inflorescence of a *Gerbera L.* plant. The different inflorescence types of *Gerbera L.* plants include: pico (6 cm to 7 cm), mini (7 cm to 8 cm), midi (8 cm to 10 cm), big (10 cm to 12 cm), and giant (greater than 12 cm). Further, normal-type *Gerbera* are *Gerbera L.* cultivars with an inflorescence diameter measuring between about 9 cm to 12 cm, and Mini*Gerbera* are *Gerbera L.* cultivars with an inflorescence diameter measuring between about 7 cm and 9 cm.

The *flower quality and stem quality* for *Gerbera L.* plant is defined as a measure of 5 ratings: very good, good, average, poor, and very poor, as described in Table 1.

## Table 1

| | very good |
| | good |
| | average |
| | poor |
| | very poor |

**FLOWER QUALITY**
defined by the following characteristics

    **A**   Texture of petals, divided into 5 categories

    **B**   Amount of layers of outer petals, amount of layers mentioned

    **C**   Composition of the flower, open-compact

    **D**   Hardiness after dehydration, divided into 5 categories

    **E**   Hardiness for damage, divided into 5 categories

| A | B | C | D | E |
|---|---|---|---|---|
| 5 (very strong) | | | 5 (very strong) | 5 (very strong) |
| 4 | | compact | 4 | 4 |
| 3 | 2 | | 3 | 3 |
| 2 | | open | 2 | 2 |
| 1 (very soft) | 1 | | 1 (very soft) | 1 (very soft) |

**STEM QUALITY**
defined by the following characteristics

    **A**   Texture of the flower stem, hollow or concrete

    **B**   Thickness, divided into 4 categories

    **C**   Susceptibility to bending after dehydration during 48 h, divided into 5 categories

    **D**   Length of the flower stem, divided into 6 categories

| A | B | C | D |
|---|---|---|---|
| | >8 mm | 0 - 10° | 60 - 65 cm |
| concrete | 7 - 8 mm | 10 - 45° | 55 - 60 or 65 - 70 cm |
| | 5 - 7 mm | 45 - 90° | 45 - 55 cm |
| hollow | <5 mm | >90° bending | <45 cm or >70 cm |
| | | >90° bending-crack | |

*Vase life* and *productivity per plant* are defined as:

| | VASELIFE | PRODUCTION |
|---|---|---|
| | | for flower size 7-8 cm |
| | (days) | (flowers/plant/year) |
| very good | >16 | >79 |
| good | 12-16 | 71-79 |
| average | 8-12 | 58-70 |
| poor | <8 | <58 |

The *different foliage types* of *Gerbera L.* plants are classified according to the 6 categories (0, 1, 2, 3, 4 and 5) described in the following Tables 2 through 5. Tables 2-5 describe the quantity, size, shape and distribution of foliage per flower stem of *Gerbera L.* plants.

TABLE 2 : Overview Description of Leaves Per Flower Stem of *Gerbera L.* by Categories 0-5

| Category | Quantity of Leaves | Description of Size | General Shape of Leaf | Length of blade (mm) | Width of blade (mm) | Angle of apex | Shape of apex | Depth of incisions | Margin of lobes | Extensions of margins |
|---|---|---|---|---|---|---|---|---|---|---|
| 0 | 0 | no leaves or extremely small | | | | | | | | |
| 1 | 1-4 | small | thin, often moon shaped | 24-36 | 2-4 | very acute to acute | Pointed | absent | entire or almost enitre | n.a. or small (3 per side) |
| 2 | 5-8 | small to medium | broader, longer, more straight | 42-64 | 4-8 | very acute | Pointed | absent to shallow | "entire" to sinuate to serrate | small to medium |
| 3 | 9-12 | medium | in general straight | 82-115 | 10-17 | very acute | Pointed | absent or very shallow | sinuate to serrate | very small to small |
| 4 | 13-16 | large | long, sinuate margins | 112-160 | 22-23 | very acute | Pointed | shallow to medium | sinuate to serrate | medium |
| 5 | >16 | very large | long, broad leaves with typical *Gerbera* leaf shape | 155-193 | 28-53 | very acute to acute | Pointed | shallow to medium | serrate to sinuate | medium to large |

TABLE 3 : Description of Leaves Per Flower Stem by Ranges within Categories 0-5

| Category | Range | Quantity of Leaves | General Shape of Leaf | Length of blade (mm) | Width of blade (mm) | Angle of apex | Shape of apex | Depth of incisions | Margin of lobes | Extensions of margins |
|---|---|---|---|---|---|---|---|---|---|---|
| **0** (As shown in FIGS. 2A and 2B) | (i) | 0 | No leaves at all; some rudiments below flower head | 2 | 1 | | | | | |
| | (ii) | 0 | Some rudiments over entire flower stem | 4 | 1 | | | | | |
| | | | | | | | | | | |
| **1** (As shown in FIGS. 2C and 2D) | (i) | 1-4 | Turn at base, at point of attachment | 25 | 3 | Very acute | Pointed | Absent | Entire or almost entire | N/A |
| | (ii) | 1-4 | Moon shape | 30 | 2 | Very acute | Pointed | Absent | Entire or almost entire | N/A |
| | (iii) | 1-4 | Moon shape | 24 | 3 | Very acute | Pointed | Absent | Entire or almost entire | N/A |
| | (iv) | 1-4 | Moon shape | 36 | 2 | Very acute | Pointed | Absent | Entire or almost entire | N/A |
| | (v) | 1-4 | Moon shape | 31 | 4 | Acute | Pointed | Absent | Entire or almost entire | Small (3 per side) |

(continued)

| Category | Range | Quantity of Leaves | General Shape of Leaf | Length of blade (mm) | Width of blade (mm) | Angle of apex | Shape of apex | Depth of incisions | Margin of lobes | Extensions of margins |
|---|---|---|---|---|---|---|---|---|---|---|
| **2** (As shown in FIGS. 2E, 2F and 2G) | (i) | 5-8 | Small moon curve | 43 | 4 | Very acute | Pointed | Shallow | Serrate (rev) | Medium |
| | (ii) | 5-8 | Straight | 42 | 6 | Very acute | Pointed | Very shallow | Sinuate | Very small |
| | (iii) | 5-8 | Straight, swing at point of attachment | 43 | 7 | Very acute | Pointed | Absent | "Entire" | Small (1 per side) |
| | (iv) | 5-8 | Straight | 45 | 8 | Very acute | Pointed | Very shallow | "Entire" | Medium (3 per side) |
| | (v) | 5-8 | Straight | 54 | 8 | Very acute | Pointed | Very shallow | "Entire" | Medium (3 per side) |
| | (vi) | 5-8 | Thin, curved, wrapped and twisted | 64 | 6 | Very acute | Pointed | Absent | Entire | Very small (1 per side) |

EP 2 011 388 A1

| Category | Range | Quantity of Leaves | General Shape of Leaf | Length of blade (mm) | Width of blade (mm) | Angle of apex | Shape of apex | Depth of incisions | Margin of lobes | Extensions of margins |
|---|---|---|---|---|---|---|---|---|---|---|
| **3** (As shown in FIGS. 2H and 2I) | (i) | 9-12 | Straight | 82 | 15 | Very acute | Pointed | Very shallow | Sinuate | Small (4 per side) |
| | (ii) | 9-12 | Straight | 88 | 13 | Very acute | Pointed | Very shallow | Sinuate | Small (4 per side) |
| | (iii) | 9-12 | Somewhat curved downwards | 89 | 13 | Very acute | Pointed | Very shallow | Sinuate | Very small (3 per side) |
| | (iv) | 9-12 | Tip curved sidewards | 91 | 14 | Very acute | Pointed | Absent | Sinuate | Very small (3 per side) |
| | (v) | 9-12 | Straight | 100 | 14 | Very acute | Pointed | Absent | Sinuate | Very small to absent |
| | (vi) | 9-12 | Tip curved sidewards | 112 | 17 | Very acute | Pointed | Absent | Sinuate | Very small (2 per side) |
| | (vii) | 9-12 | Straight | 115 | 10 | Very acute | Pointed | Very shallow | Serrate | Small (3 per side) |
| | | | | | | | | | | |
| | | | | | | | | | | |
| **4** (As shown in FIGS. 2J and 2K) | (i) | 13-16 | Somewhat curved sidewards | 112 | 23 | Very acute | Pointed | Medium | Serrate | Medium |
| | (ii) | 13-16 | Somewhat curved sidewards | 140 | 23 | Very acute | Pointed | Shallow | Sinuate | Medium |
| | (iii) | 13-16 | Somewhat curved sidewards | 160 | 22 | Very acute | Pointed | Shallow | Sinuate | Medium |
| | | | | | | | | | | |

(continued)

| Category | Range | Quantity of Leaves | General Shape of Leaf | Length of blade (mm) | Width of blade (mm) | Angle of apex | Shape of apex | Depth of incisions | Margin of lobes | Extensions of margins |
|---|---|---|---|---|---|---|---|---|---|---|
| **5** (As shown in FIGS. 2L and 2M) | (i) | >16 | Miniature *Gerbera* leaf | 155 | 45 | Very acute | Pointed | Medium | Serrate | Large |
| | (ii) | >16 | Very strong (straight with stiff texture) | 173 | 28 | Very acute | Pointed | Shallow | Sinuate | Small to medium |
| | (iii) | >16 | Miniature *Gerbera* leaf | 178 | 53 | Very acute | Pointed | Medium | Serrate | Large |
| | (iv) | >16 | Very strong (straight with stiff texture) | 193 | 38 | Acute | Pointed | Shallow | Serrate | Large |

TABLE 4: Distribution of Leaves Per Flower Stem by Categories 0-5

| Category | Drawing of Distribution | Description of Distribution in Bud Stage | Example of Distribution in Bud Stage | Description of Distribution in Open Flower Stage | Example of Distribution in Open Flower Stage |
|---|---|---|---|---|---|
| 0 | | No leaves or hardly any | Shown in FIG 3A | Same as Bud Stage | Shown in FIG 3B |
| 1 | | Category 4 or 5 at Base of Flower Stem Category 1 or 2 at Top of Flower Stem | Shown in FIG 3C | Same as Bud Stage | Shown in FIG 3D |
| 2 | | Leaves (Equally Divided) Over Flower Stem | Example 1: Categories 4 & 5 Shown in FIG 3E | Same as Bud Stage | Shown in FIG 3G |
| | | | Maximum of 1 Category Difference in Leaf Size Between Top and Bottom of Flower Stem | Example 2: Categories 2 & 3 Shown in FIG 3F | | |

(continued)

| Category | Drawing of Distribution | Description of Distribution in Bud Stage | Example of Distribution in Bud Stage | Description of Distribution in Open Flower Stage | Example of Distribution in Open Flower Stage |
|---|---|---|---|---|---|
| 3 | | Leaves only at Upper Half of Flower Stem Typically only 1 Category of Leaves (Maximum 2) | Shown in FIG 3H | Same as Bud Stage | Shown in FIG 3I |
| 4 | | Leaves Only at Upper 1/3 to 1/4 of Flower Stem Only One Category Size of Leaves Per Flower Stem | Shown in FIG 3J | Same as Bud Stage | Shown in FIG 3K |
| 5 | | Leaves Only at Upper 1/5 of Flower Stem Only One Category Size of Leaves Per Flower Stem | Shown in FIG 3L | Same as Bud Stage | Under Development |

TABLE 5: Distribution of Different Leaf Sizes (accordingly to Categories 1-5) Along Flower Stem At Open Flower Stage

| Category | Drawing Description Of Leaf Distribution | Possible combinations in leaf sizes at different positions | | | | | | | | | | | | |
|---|---|---|---|---|---|---|---|---|---|---|---|---|---|---|
| 1 | | Top | 1 | 1 | 2 | 1 | 1 | 2 | | | | | | |
| | | Middle | 1 | 2 | 2 | 1 | 2 | 2 | | | | | | |
| | | Base | 4 | 4 | 4 | 5 | 5 | 5 | | | | | | |

(continued)

| Category | Drawing Description Of Leaf Distribution | Possible combinations in leaf sizes at different positions | | | | | | | | | | | | |
|---|---|---|---|---|---|---|---|---|---|---|---|---|---|---|
| 2 | | Top | 1 | 1 | 1 | 2 | 2 | 2 | 3 | 3 | 3 | 4 | 4 | 4 | 5 |
| | | Middle | 1 | 1 | 2 | 2 | 2 | 3 | 3 | 3 | 4 | 4 | 4 | 5 | 5 |
| | | Base | 1 | 2 | 2 | 2 | 3 | 3 | 3 | 4 | 4 | 4 | 5 | 5 | 5 |
| 3 | | Top | 1 | 1 | 2 | 2 | 3 | 3 | 4 | 4 | 5 | | | | |
| | | Middle | 1 | 2 | 2 | 3 | 3 | 4 | 4 | 5 | 5 | | | | |
| | | Base | 0 | 0 | 0 | 0 | 0 | 0 | 0 | 0 | 0 | | | | |
| 4 | | Top | 1 | 2 | 3 | 4 | 5 | | | | | | | | |
| | | Middle | 0 | 0 | 0 | 0 | 0 | | | | | | | | |
| | | Base | 0 | 0 | 0 | 0 | 0 | | | | | | | | |
| 5 | | Top | 1 | 2 | 3 | 4 | 5 | | | | | | | | |
| | | Middle | 0 | 0 | 0 | 0 | 0 | | | | | | | | |
| | | Base | 0 | 0 | 0 | 0 | 0 | | | | | | | | |

In the present application, *foliage type* is defined for a *Gerbera L.* plant by the i) quantity, ii) size, and iii) distribution of the full or partial leaves produced by one or more flower stems of a *Gerbera L.* plant, according to the 6 categories (0, 1, 2, 3, 4 and 5) described in Tables 2 through 5. Typical, non-leafy *Gerbera L.* plants produce flower stems with either a) no foliage, classified as category 0, or b) less than five (5) extremely small to small-sized full or partial leaves per flower stem, classified as either category 0 or 1, and which may be distributed at the base or top of the flower stem. Typical, leafy flower stem *Gerbera L.* plants produce flower stems with at least five or more full or partial leaves per flower stem, classified by categories 2-5, which are small to extra-large in size, classified by categories 2-5, and which may be distributed either equally along the flower stem or along the top half of the flower stem.

*Leaves* are defined as a tissue expansion growing along the side of the branches or from the flower stem or rootstock of a plant, and are one of the parts of the plant which constitutes the plant's foliage. Leaves vary according to their attachment (sessile or petiolated), type (simple or compound), arrangement (alternate, opposite, whorled or rosulate/ rosette), as well as, blade shape, size, margin, texture, venation pattern and color. Most leaves consist of a blade that is supported on a petiole which continues through the blade as the midrib and then off-shoots into ribs and veins that support the cellular texture. Most leaves are green and contain chlorophyll, and are the part of the plant that performs photosynthesis, the process that converts sunlight and carbon dioxide into energy for the plant.

In the present invention, the leaves on the flower stem of GERFOLIA™ *Gerbera L.* plants are defined as 'real' leaves, *rosette leaves* (defined as a cluster of leaves arranged in a circular disc, sometimes at the base of plant) and not *bracts* (defined as a modified leaf at the base of a flower or flower cluster, which are often smaller than regular leaves and modified (scaly, brown or different shape), based on the rosette leaf morphology of the mature leaves on the flower stem of GERFOLIA™ *Gerbera L.* plants.

During the breeding program of the present invention, the development of the leaves on the flower stem of GERFOLLA™ *Gerbera L.* plants was closely observed in order to determine whether the full or partial leaves on the flower stem of leafy *Gerbera L.* plants may be referred to more properly as full or partial bracts or leaves on the flower stem of leafy *Gerbera L.* plants.

A close-up view of the development stages of the leaves on the flower stem of GERFOLIA™ *Gerbera L.* plants of the present invention is shown in FIGS. 4A-4G. FIG. 4A and FIG. 4B show a close-up view of the small leaves, tightly surrounding the developing capitulum, which is covered with protective bracts during the early development of the scape. FIG. 4C shows a close-up view of the basal (lower) part of the scape which extends (quickly) and the leaves start to separate from each other, and FIG. 4D shows a close-up view of the upper part of the scape which then extends (slowly). FIG. 4E, 4F and 4G show a close-up view of the leaves once the scape has extended to its final height. FIG. 4E and 4F show a close up view of the lower and older leaves on the scape which grow to maturity to resemble rosette leaves, including a depiction of the mid veins as shown in FIG. 4F. FIG. 4G shows a close-up view of the upper and younger (immature) leaves on the scape which are smaller and may seem to resemble bracts.

During the 2006 - 2008 breeding program years, the inventors continued to observe that the leaves on the flower stems of selected GERFOLIA™ *Gerbera L.* seedlings showed an increasing morphological resemblance to rosette leaves. FIGS. 19B, 20B, 21B, 22B, 23B, 24B and 25B show a close-up view of the morphological resemblance of the mature leaves of GERFOLIA™ *Gerbera L.* seedlings of the present invention to rosette leaves. Further, FIG. 24B shows a close-up view of the mature leaves, close to the involucre, of the GERFOLIA™ *Gerbera jamesonii* 'BL07.790' that resemble rosette leaves and not bracts. In addition, Examples 4-10 provide a botanical description of GERFOLIA™ *Gerbera L.* seedlings of the present invention, with a description of the leaves on the flower stems, including a description of the presence of extensions of the margins of the leaves

The *degree of foliage per flower stem* is defined as a measure of the number of extra full or partial leaves per flower stem produced beyond the four (4) full or partial leaves typically produced per flower stem on non-leafy *Gerbera L.* plants. The greater the degree of foliage per flower stem, the greater the number of full or partial leaves produced per flower stem.

The *degree of foliage per plant* is defined as a measure of the number of flower stems per *Gerbera L.* plant which produce beyond the four (4) full or partial leaves typically produced per flower stem on non-leafy *Gerbera L.* plants. The greater the degree of foliage per *Gerbera L.* plant, the higher the percentage of flower stems per *Gerbera L.* plant which produce beyond the four (4) full or partial leaves produced per flower stem.

Preferably, the new *Gerbera L.* plant of the instant invention has substantially all leafy flower stems, and each flower stem has at least 5, to about 30, or more than 30 full or partial leaves per flower stem. *Gerbera L.* plants of the instant invention produce flower stems with a relatively uniform number of leaves per flower stem and this characteristic is stable through asexual reproduction. Alternatively, *Gerbera L.* plants of the instant invention may produce flower stems with a wide range in the number of leaves per flower stem and this characteristic is also stable through asexual reproduction.

A *trademark,* otherwise known as a brand, may be a word, name, symbol, device, design or phrase adopted and used to identify its goods and services and to distinguish them from the goods and services of others.

GERFOLIA™ *brand plants* are *Gerbera L.* cultivars of the present invention that exhibit the leafy flower stem trait. A GERFOLIA™ brand *Gerbera L.* cultivar of the present invention may exhibit both the leafy flower stem trait and in bud shipping trait. GERFOLIA™ brand plants of the present invention produce flower stems with at least 5 or more full or partial leaves per flower stem. A GERFOLIA™ brand *Gerbera L.* cultivar of the present invention can produce one or more flower stems with at least 5, to about 30, or more than 30 full or partial leaves per flower stem. A GERFOLIA™ brand *Gerbera L.* cultivar of the instant invention can produce at least 5, 6, 8, 10, 12, 14, 16, 18, 20, 22, 24, 26, 28, 30, any integer between 5 and 30, or more than 30 full or partial leaves per flower stem. The leaves produced on a flower stem of a GERFOLIA™ brand *Gerbera L.* cultivar of the present invention range in size from small to extra-large. The leaves produced by a GERFOLIA™ *brand* flower stem of a *Gerbera L.* cultivar of the present invention can range in size from a) at least 40 mm or more in length, and b) at least 4 mm or more in width. The leaves produced by a GERFOLIA™ *brand* flower stem of a *Gerbera L.* cultivar of the present invention can range in size from a) at least 40 mm, to about 200 mm, or more than 200 mm in length, and b) at least 4 mm, to about 60 mm, or more than 60 mm in width. The leaves produced by a flowers stem of a *Gerbera L.* cultivar of the present invention can range in size from a) at least 40, 50, 60, 70, 80, 90, 100, 110, 120, 130, 140, 150, 160, 170, 180, 190, 200, any integer between 40 and 200, or more than 200 mm in length, and b) at least 4, 6, 8, 10, 12, 14, 16, 18, 20, 22, 24, 26, 28, 30, 32, 34, 36, 38, 40, 42, 44, 46, 48, 50, 52, 54, 56, 58, 60, any integer between 4 and 60, or more than 60 mm in width. In addition, the leaves produced on a flower stem of a *Gerbera L.* cultivar of the instant invention are distributed either equally along the flower stem or along the top half of the flower stem. Further, *Gerbera L.* cultivars of the present invention, exhibiting at least 5 or more full or partial leaves per flower stem, may have the ability to be packaged and transported during the in bud stage of development.

2. Plant Selection and Breeding

General breeding methods of *Gerbera* cultivars is described in Barigozzi, C. and L. Quagliotti, 1978, "Current Research on Breeding of Gerbera", Proceedings of the Eucarpia Meeting on Carnation and Gerbera, Allasio, pp. 57-68, and Van Os, D.P.M., 1995, "Stageverslag Gerbera Veredeling, Practical Period Agriculture", University Wageningen, Dept. of Plant Breeding.

The development of *Gerbera L.* cultivars wherein substantially all the flower stems produced by said plant contain foliage, with at least 5, to about 30, or more than 30 full or partial leaves per flower stem, has been a gradual process involving many selections over the past 17 years. Breeding and selection for the leafy flower stem trait began in 1991, and the inventors' aim was to develop new *Gerbera L.* cultivars producing substantial foliage on flower stem.

The first crossings in the breeding program were made in an effort to develop a novel and unique type of *Gerbera L.* plant which was specifically designed to suit the needs and specifications of the *Gerbera* ornamental market by offering a new and distinct leafy flower stem trait that the present *Gerbera L.* plants do not exhibit. The breeding program successfully produced a new type of *Gerbera L.* plant exhibiting a new and distinct leafy flower stem trait, that typical *Gerbera L.* cultivars lack. In addition, the breeding program successfully produced some leafy flower stem *Gerbera L.* cultivars which have the ability to be shipped during the in bud stage of development. The new leafy flower stem type of *Gerbera L.* plants, which may have the ability to be shipped during the in bud stage of development, express a desirable new phenotype while also maintaining characteristics considered desirable in the *Gerbera* commercial ornamental market.

<u>1999</u>

On very limited scale, from 1991 through 1999, the inventors began a breeding program in Kudelstaart, The Netherlands, for a new type of *Gerbera L.* cultivar with leafy flower stems. The first selection of *Gerbera L.* seedlings exhibiting the leafy flower stem trait, even though small, was in 1999. At that time, the inventors decided that the leafy flower stem trait had commercial potential and the breeding program was expanded.

Table 6 shows the genetic basis for the 14 most important parents (all having been generated either directly or with one more generation in between from Normal-type *Gerbera* or Mini*Gerbera)*, which were used and selected based on their specific characteristics to obtain the first *Gerbera L.* plants exhibiting the leafy flower stem trait.

Table 6: List of parents forming the basis of the newly introduced leafy flower stem trait

| Selected Seedling Code | Mother | | Father | Size* | Color | Type** | Centre | Leafiness*** | Remarks |
|---|---|---|---|---|---|---|---|---|---|
| | | | | | | | | (base-middle-top) | |
| BL92.002 | M91.081 | x | 84.574 | Midi | Red | s | green | 0-2-1 | Cup flower |
| | mini | | Big | | | | | | |
| BL92.004 | M91.081 | x | 84.574 | Mini | Pink | s | black | 0-1-1 | Cup flower |
| | mini | | Big | | | | | | |
| BL92.006 | M90.147 Merenque | x | M90.149 | Mini | soft yellow | sd | black | 0-1-1 | Short stem |
| | mini | | Mini | | | | | | |
| BL92.009 | unknown | x | M91.175 | Mini | Pink | sd | green | 0-3-1 | Big leaves |
| | ? | | Mini | | | | | | |
| BL93.002 | 91.087 | x | unknown | Midi | Orange | sd | green | 0-2-1 | Strong stem |
| | standard | | ? | | | | | | |
| BL93.004 | unknown | x | unknown | Midi | soft orange | d | green | 0-0-1 | Very double |
| BL93.006 | 91.087 | x | BL92.001 | Mini | soft pink | d | green | 0-1-1 | Very double |

(continued)

| Selected Seedling Code | Mother | | Father | Size* | Color | Type** | Centre | Leafiness*** | Remarks |
|---|---|---|---|---|---|---|---|---|---|
| | big | | Leafy mini | | | | | | |
| BL93.009 | M91.075 | x | unknown | Mini | Yellow | sd | green | 0-2-1 | Strong stem |
| | mini | | ? | | | | | | |
| BL93.010 | unknown | x | unknown | Mini | Pink | sd | green | 0-1-1 | |
| BL94.001 | BL93.004 | x | BL92.006 | Pico | soft orange | sd | green | 0-2-1 | |
| | leafy midi | | leafy mini | | | | | | |
| BL97.524 | M95.561 Cabaret | x | M94.014 Giga | Mini | White | sd | green | 0-2-1 | |
| | mini | | mini | | | | | | |
| BL97.525 | M94.069 Eurostar | x | M95.516 Jazz | Mini | Orange | sd | green | 0-2-1 | |
| | mini | | midi | | | | | | |
| BL98.001 | 95.109 | x | 95.121 | Big | Pink | sd | black | 0-1-1 | Very weak plant |
| | big | | big | | | | | | |
| BL99.142 | 95.145 Evergreen | x | 97.016 | Big | soft pink | s | black | 2-2-1 | |
| | giant | | big | | | | | | |
| BL99.152 | 97.020 Red Dino | x | 97.088 Totem | Big | Red | s | black | 0-0-2 | Only leaves below flower |
| | big | | big | | | | | | |
| * Size: pico = 6-7 cm, mini = 7-8 cm, midi = 8-10 cm, big = 10-12 cm, giant = >12 cm. ** Type (flowering type): s = single, sd = semi-double, d = double ** In 1999, the early selection was not described by the categories and positions as is currently done since the leafy stem trait was not as developed. The description given in Table 6 is rewritten into the currently used categories for size and position of the leaves. | | | | | | | | | |

Table 7 shows the most important seedlings, resulting from the crossings of Table 6, which were selected for additional crossings. To avoid inbreeding, the inventors conducted 89 crossings, of which 18 crossings produced promising *Gerbera L.* seedlings exhibiting the new leafy flower stem trait. Another six crossings were added by introduction of a new entry from a mixed seedling population, hereinafter designated by seedling code 3135, and which is chararacterized as a Mini*Gerbera,* orange-red inflorescence color with a black center, semi-double flowering type, foliage distribution of 0-0-0, and which produced very small loose bracts below the flower head.

The seeds of the selected *Gerbera L.* seedlings were harvested and sown. After the selection procedure during 1999, 16 seedlings were selected and coded for further introgression of the leafy stem trait. From the 22 crossings conducted, 10 rendered acceptable results (45.5%).

Table 7: 1999-2000 Breeding Program - Promising seedlings selected from parental crosses of Table 6 and Cross Information

| Mother | Father | Q/seeds (when known) | Q/ sel | Selected seedlings (leaf size : b-m-t) | Description (size-color-type-centre) |
|---|---|---|---|---|---|
| BL92.002 | BL97.524 | | 4 | BL00.505 (0-2-1) BL00.506 (0-2-1) BL00.507(0-2-1) BL00.511 (2-2-2) | Mini-cream-sd-gc Mini-soft pink-s-gc Mini-cream-s-gc Mini-soft pink-sd-gc |
| BL92.004 | BL92.006 | 73 | 1 | BL00.514 (0-2-1) | Mini-soft orange-sd-gc |
| | BL93.002 | | 3 | BL00.501 (0-3.5-1) BL00.502 (0-2-1) BL00.504 (0-2-1) | Mini-soft yellow-s-gc Mini- ochre -s-bc Mini-red-s-bc |
| | BL99.152 | | 0 | | |
| BL92.006 | BL97.524 | 8 | 0 | | |
| BL92.009 | BL93.002 | 68 | 0 | | |
| | BL93.009 | 8 | 1 | BL00.515 (0-2-1) | Mini-soft pink-s-gc |
| BL93.002 | BL97.525 | 34 | 1 | BL00.516 (0-2-2) | Mini-mix pink-sd-gc |
| | BL99.152 | | 1 | BL00.509 (0-0-2) | Big-red-s-bc |
| BL93.004 | BL97.525 | | 0 | | |
| BL93.009 | BL97.525 | | 1 | BL00.510 (0-2-1) | Mini-pink-sd-gc |
| | BL99.142 | | 0 | | |
| | BL99.152 | | 0 | | |
| BL93.010 | BL94.525 | 34 | 0 | | |
| | BL99.142 | | 0 | | |
| BL94.001 | BL99.152 | | 2 | BL00.503 (0-2-2) BL00.508 (0-2-2) | Mini-red-sd-bc Midi-red-s-gc |
| 3135 | BL93.002 | | 1 | BL00.512 (0-1-1) | Pico-orange-sd-bc |
| | BL93.004 | | 0 | | |
| | BL93.009 | | 1 | BL00.513 (0-1-1) | Pico-orange-sd-bc |
| | BL93.010 | | 0 | | |
| | BL94.001 | | 0 | | |
| | BL95.524 | | 0 | | |

The breeding results from the first generation of crossings can be summarized as follows. When crossing with *Gerbera L.* plants exhibiting a flower stem leaf distribution of 0-0-1 or less, the results are poor for selecting seedling material, but it is possible to select a small percentage of *Gerbera L.* plants exhibiting a flower stem leaf distribution of 0-0-1 for future breeding.

In addition to screening by presence of the leafy flower stem trait, criteria for selection also considered the number and size of the foliage on the flower stem, as well as, the presence of other desirable commercial traits that apply for *Gerbera L.* breeding, such as, flower stem length, flower quality, vase life, and productivity. Accordingly, no selected seedlings from a cross does not indicate that there was no transfer of the leafy flower stem genetic information into the progeny, rather the commercial success of the characteristics of a resulting seedling as a whole was determined not favourable by the inventors. Further, the inventors discovered that when crossing seedlings with more developed leafy flower stems, the resulting seedlings exhibit at least the same or an increased leaf size. The selected seedling BL00.509 revealed that the distribution of the leaves on the flower stem could be stabilized. Lastly, all selected seedlings of the 1999 breeding program revealed to the inventors that flower size and flower color (ray floret and disc floret color) transfer as usual and that variation is maintained in the population.

<u>2000</u>

The inventors goal for the 2000 breeding program was to increase both the leaf size and number of leaves on the flower stem of new *Gerbera L.* seedlings exhibiting the leafy flower stem trait. The breeding program was further expanded to consider the possibility of modifying the distribution of the leaves on the flower stem.

Table 8: 2000-2001 Breeding Program - Promising seedlings from crosses of Table 7 and Cross Information

| Mother | Father | Q/seeds (when known) | Q/ sel | Selected seedlings (leaf size : b-m-t) | Description (size-color-type-centre) |
|---|---|---|---|---|---|
| BL92.004 | BL92.009 | | 1 | BL01.508 | Mini-yellow-s-bc |
| | BL00.508 | 141 | 0 | | |
| | BL00.509 | 5 | 0 | | |
| | BL00.513 | 25 | 1 | BL01.501 | Midi-red-s-bc |
| BL92.009 | BL94.001 | 35 | 0 | | |
| | BL99.152 | | 3 | BL01.502 BL01.505 BL01.509 | Mini-pink-sd-gc Mini-cream-s-gc Mini-pink-sd-gc |
| | BL00.503 | 19 | 1 | BL01.528 | Mini-orange-sd-gc |
| | BL00.507 | | 3 | BL01.517 BL01.531 BL01.532 | Mini-yellow-sd-gc Mini-pink-sd-gc Mini-cream-s-gc |
| | BL00.513 | 12 | 0 | | |
| | BL00.516 | 36 | 2 | BL01.515 BL01.520 | Mini-pink-sd-gc Mini-yellow-s-gc |
| BL93.002 | BL94.001 | 48 | 0 | | |
| | BL99.152 | | 6 | BL01.516 BL01.523 BL01.524 BL01.525 BL01.526 BL01.527 | Mini-cream-s-gc Mini-pink-sd-gc Mini-red-sd-gc Mini-orange-sd-gc Mini-pink-sd-gc Mini-pink-s-gc |
| | MIR55 | | 1 | BL01.513 | Big-yellow-s-gc |
| | BL00.505 | 1 | 0 | | |
| | BL00.508 | 22 | 0 | | |
| | BL00.514 | 18 | 2 | BL01.514 BL01.519 | Mini-yellow-s-gc Mini-orange-sd-gc |

(continued)

| Mother | Father | Q/seeds (when known) | Q/ sel | Selected seedlings (leaf size : b-m-t) | Description (size-color-type-centre) |
|---|---|---|---|---|---|
| BL94.001 | BL00.501 | 3 | 0 | | |
| | BL00.504 | 2 | 0 | | |
| | BL00.506 | 29 | 0 | | |
| BL00.501 | BL00.503 | 6 | 1 | BL01.503 | Midi-red-s-bc |
| | BL00.511 | 7 | 0 | | |
| | BL00.516 | 10 | 0 | | |
| BL00.504 | BL99.152 | | 1 | BL01.506 | Mini-yellow-sd-gc |
| BL00.506 | BL99.152 | | 4 | BL01.504 BL01.507 BL01.510 BL01.512 | Mini-yellow-sd-gc Mini-pink-sd-gc Mini-pink-sd-gc Mini-pink-s-gc |
| | BL00.515 | 15 | 2 | BL01.518 BL01.522 | Mini-yellow-s-gc Mini-yellow-sd-gc |
| BL00.510 | BL00.511 | 5 | 0 | | |
| BL00.511 | BL00.508 | 7 | 0 | | |
| | BL00.514 | 7 | 1 | BL01.521 | Mini-cream-sd-gc |
| BL00.513 | BL00.509 | | 2 | BL01.529 BL01.530 | Mini-red-sd-gc Mini-yellow-sd-bc |

From the crosses of the 2000-2001 breeding program, the inventors selected 31 seedlings, all of which exhibited leafy flower stems with an increased number of leaves which were larger in size. All crossings in the 2000-2001 breeding program, except one were between BL-codes, and from the resulting, selected seedlings, 14 out 27 crossings yielded good results (51.9%). The increased success in the BLxBL crossings (from 45.5% in 1999 to 51.9% in 2000) revealed that the leafy flower stem trait was successfully being introduced into the *Gerbera L.* population.

2001

Until 2001, about 90% of the selected seedlings possessed flowers in soft colors, such as creams, soft pinks, soft yellows and soft-oranges. In 2001, the inventors added the following two new goals to the breeding and selection of new *Gerbera L.* seedlings exhibiting the leafy flower stem trait: 1) introgressing the leafy flower stem trait to flowers in bright colors, such as oranges, reds and purples, and 2) increasing leaf size and number of leaves per flower stem.
For the first goal, the inventors performed crosses combining *Gerbera L.* seedlings exhibiting both the leafy flower stem trait and mini and mirage flower head types in order to increase the expression of the leafy flower stem trait with bright flower colors. In a total of 11 crossings made, 222 seedlings were obtained and observed. Two promising seedlings, exhibiting bright flower color and the leafy flower stem trait were selected and are described below.

**M97.508 (Violetta) x BL00.504**

    5 seedlings
    1 selected (**BL02.518**) (0-1-1)

**BL00.516 x MIR00.002**

6 seedlings
1 selected (**BL02.528**) (0-3.5-1)

The success of the crossings in this category is 18%. The two parents are further removed in the leafy flower stem trait, so it will take (an)other crossing(s) to achieve the intended result. However, the most important discovery of this breeding year is that the crosses and the resulting seedlings revealed that is possible to introduce the leafy flower stem trait into bright color genotypes of *Gerbera L.* plants.

For the second goal, the inventors discovered that it was necessary to make crossings within the leafy *Gerbera L.* types, taking care not to do too much inbreeding, in order to increase the leaf size and number of leaves per flower stem. In a total of 23 crossings made between BL-codes, 1048 seedlings were obtained and observed. 31 seedlings were selected with the improved leafy flower stem trait (3%) coming from 13 crossings (56.5%).

In Table 9, the crossing schedule for 2001-2002 is depicted, including the resulting, selected seedlings. Of the resulting, selected seedlings, the size of the leaves increased from category 1 or 2 into 2, 3 and 3.5. The expression of the leafy flower stem trait was further improved in the resulting, seedling population, as a result of the 3$^{rd}$ or 4$^{th}$ generation crossing for the trait. In addition, the selected seedlings exhibit all flower colors and inflorescence sizes, as well as, single, semi-double and double flowering types.

Table 9: 2001-2002 Breeding Program - Promising seedlings from crosses of Table 8 and Cross Information

| Mother | Father | Q/seeds (when known) | Q/ sel | Selected seedlings (leaf size : b-m-t) | Description (size-color-type-centre) |
|---|---|---|---|---|---|
| M97.508 Violetta | BL00.504 | 5 | 1 | BL02.518 (0-1-1) | Mini-pink-sd-gc |
| M98.507 Terra Gina | BL00.501 | 26 | 0 | | |
| M98.574 Flirt | BL00.508 | 23 | 0 | | |
| M99.555 Maroesjka | BL01.507 | 55 | 0 | | |
| M99.607 | BL00.501 | 27 | 0 | | |
| | BL01.520 | 6 | 0 | | |
| M00.517 Tombola | BL00.501 | 15 | 0 | | |
| | BL01.503 | 44 | 0 | | |
| | BL01.529 | 7 | 0 | | |
| BL93.002 | BL00.503 | 100 | 4 | BL02.513<br>BL02.514<br>BL02.523<br>BL02.524 (0-3-1) | Mini-red-s-bc*<br>Midi-yellow-sd-bc<br>Mini-orange-sd-gc<br>Mini-red-sd-bc |
| BL00.502 | BL00.503 | 54 | 0 | | |
| | BL01.528 | 32 | 3 | BL02.501<br>BL02.515<br>BL02.525 | Mini-cream-s-gc<br>Mini-orange-sd-gc*<br>Mini-yellow-sd-bc |
| BL00.503 | BL00.506 | 36 | 2 | BL02.502<br>BL02.503 | *<br>Mini-purple-s-bc |
| | BL00.515 | 136 | 4 | BL02.504<br>BL02.505<br>BL02.516 (0-2-1)<br>BL02.517 | Mini-cream-sd-gc*<br>Mini-red-s-gc*<br>Mini-soft pink-s-gc<br>Mini-pink-sd-gc |
| | BL01.503 | 154 | 0 | | |
| BL00.507 | BL01.514 | 69 | 7 | BL02.506<br>BL02.519 | Mini-cream-s-gc*<br>Mini-purple-sd-gc* |

(continued)

| Mother | Father | Q/seeds (when known) | Q/ sel | Selected seedlings (leaf size : b-m-t) | Description (size-color-type-centre) |
|---|---|---|---|---|---|
| | | | | BL02.520<br>BL02.521 (0-2-1)<br>BL02.526<br>BL02.527<br>BL02.529 | Mini-purple-sd-gc<br>Mini-white-s-gc<br>Mini-cream-sd-gc<br>Mini-yellow-s-gc<br>Mini-pink-sd-gc |
| BL00.508 | BL00.511 | 10 | 1 | BL02.507 (0-2.5-1) | Midi/big-yellow-d-bc |
| BL00.511 | BL00.516 | 11 | 0 | | |
| | BL01.529 | 21 | 1 | BL02.508 | Mini-cream-sd-gc* |
| BL00.514 | BL01.513 | 64 | 2 | BL02.509<br>BL02.510 (!) | Mini-yellow-s-gc*<br>Midi-yellow/orange-s-gc |
| | BL01.518 | 6 | 0 | | |
| | BL01.530 | 24 | 0 | | |
| BL00.516 | MIR00.002 | 6 | 1 | BL02.528 (0-3.5-1) | Big purple mix -d-gc |
| BL01.503 | BL01.518 | 27 | 3 | BL02.530<br>BL02.531<br>BL02.532 (0-3.5-1) | Mini-yellow-s-bc*<br>Mini-red-sd-gc<br>Pico-orange/pink-sd-gc |
| BL01.505 | BL01.529 | 36 | 0 | | |
| BL01.513 | BL00.508 | 68 | 1 | BL02.511 | Mini-purple-s-gc |
| | BL00.514 | 30 | 0 | | |
| | BL01.530 | 8 | 0 | | |
| | MIR00.002 | 8 | 0 | | |
| BL01.515 | BL00.510 | 10 | 0 | | |
| BL01.519 | BL01.528 | 3 | 0 | | |
| BL01.522 | BL01.524 | 82 | 2 | BL02.522<br>BL02.533 | Mini-cream-s-gc<br>Mini-pink-s-gc |
| BL01.526 | BL01.503 | 30 | 0 | | |
| BL01.528 | BL01.512 | 7 | 1 | BL02.512 | Mini-red-sd-bc* |

The selected seedlings marked with an * unfortunately died after being selected, due to cultivation related problems in the greenhouse.

2002

During 2002-2003, the inventors focused on improvement of flower color intensity, flower quality and continued increase in leaf size and number of leaves per flower stem. Since the 2002-2003 breeding year is the 4th or 5th generation, it was possible to use some of the original parents from the 1999 breeding program to broaden the basis without suffering from inbreeding.

The selected *Gerbera L.* seedlings to date had fewer ray florets per flower head when compared to typical non-leafy *Gerbera L.* varieties. Accordingly, the inventors used more double-type flowering varieties in crosses to improve flower quality expression in the leafy flower stem type gene pool.

Table 10: 2002-2003 Breeding Program - Promising seedlings from crosses of Table 9 and Cross Information

| Mother | Father | Q/seeds (when known) | Q/ sel | Selected seedlings (leaf size : b-m-t) | Description (size-color-type-centre) |
|---|---|---|---|---|---|
| BL00.503 | BL02.518 | | 2 | BL03.707 BL03.716 | Midi-red/pink-s-gc Mini-pink-s-bc |
| | BL02.521 | | 2 | BL03.708 BL03.717 | Mini-pink-sd-gc Mini-purple-sd-gc |
| BL00.508 | BL02.515 | | 4 | BL03.700 (2-3-2) BL03.701 BL03.702 BL03.704 (!) BL03.718 | Mini-yellow-s-gc Mini-orange-s-gc Mini-bright orange-d-gc Mini-red-s-gc ? |
| | BL02.521 | | 2 | BL03.719 BL03.720 | Mini-yellow-sd-gc Mini-pink-sd-gc |
| | BL02.531 | | 1 | BL03.721 | Mini-red-sd-gc |
| BL00.509 | 95.145 Evergreen | 0 | 0 | | |
| BL01.504 | M01.592 | 0 | 0 | | |
| BL01.526 | M96.504 Icedance | | 1 | BL03.703 (0-1-1) | Mini-white-sd-gc |
| BL02.507 | M98.502 Bandola | 0 | 0 | | |
| BL02.511 | BL93.002 | | 2 | BL03.715 BL03.722 | Midi-red-s-gc Mini-purple-sd-gc |
| | M95.561 Cabaret | | 0 | | |
| | BL00.503 | | 0 | | |
| BL02.514 | M01.591 | | 3 | BL03.709 BL03.710 BL03.711 | Mini-bright yellow-s-bc Mini-bright red-sd-bc Mini-red/pink-sd-bc |
| BL02.517 | BL00.501 | | 0 | | |
| BL02.520 | M01.521 | | 1 | BL03.705 | Mini-red-sd-gc |
| BL02.521 | M98.514 Homerun | 0 | 0 | | |
| BL02.526 | BL00.503 | | 1 | BL03.714 | Mini-pink-s-bc |
| BL02.531 | BL00.501 | | 2 | BL03.706 BL03.712 (2-3-2) | Mini-red-s-gc Mini-pink-s-gc |

Selected from the non-leafy *Gerbera L.* breeding program, a new entry was made with seedling (BL03.713) coming from the cross SP02.557 x M01.506. The leaves on the flower stem of BL03.713 were only very small, but the unique, pure white flower color of BL03.713 is a difficult flower color to introduce into the gene pool of the leafy *Gerbera L.* For that reason this seedling was added to the leafy *Gerbera L.* gene pool.

Seedling 95.145 (Evergreen) is one of the original ancestors that had been introduced successfully before in 1999. Re-entry of 95.145 (Evergreen) was made due to the desirable traits of flower size and flower quality of this seedling. Unfortunately no seeds were harvested.

In 2001, a cross had been made with a non-leafy Mini*Gerbera* seedling M98.507 (Terra Gina). No seedlings had been selected, as they did not fit the criteria for selection of *Gerbera L.* as a whole. The parent seedling M98.507 (Terra Gina) is interesting however for the leafy trait. For that reason, two offsprings (M01.591 and M01.592) with M98.507 (Terra Gina) as a common parent have been used in this schedule. Introduction of M01.591 has shown to be very successful as a cross, even though M01.592 has unfortunately not given any seeds.

For the improvement of the flower quality, the parents M96.504 (Icedance), M98.502 (Bandola), M95.561 (Cabaret) and M98.514 (Homerun) have been used. Of this list M95.561 (Cabaret) is also one of the early ancestors. For color improvement, the parents M98.502 (Bandola), M01.591 and M01.521 were of interest. One of the parents of M01.521 is M97.508 (Violetta) which had already been used as a successful parent in 2001.

Of the 8 total leafy x non-leafy *Gerbera L.* crossings, 4 crossings did not result in any seed. Of the remaining four crosses, three were successful. It became clear to the inventors that knowledge of the seedling crosses from the past breeding years and repeated introduction helps to more effectively introgress the leafy trait into non leafy type *Gerbera L.* plants.

In total for the BL x BL crosses, the success rate has increased to 80% and the leafy flower stem trait is more clearly present among resulting seedlings, and is expressed with other commercially desirable traits.

In 2002, the inventors managed to have a 75% success rate when crossing leafy x non leafy genotypes, and an 80% success rate when crossing leafy x leafy genotypes. The leafy flower stem trait is well present in the *Gerbera L.* genotype and the genetic basis is broad enough to aim higher in regards to general market demands. Therefore, the inventors' selection criteria for the 2003 breeding program places more emphasis on flower quality, productivity, stem length and quality.

2003

After 4 or 5 generations of breeding specifically for the leafy flower stem trait, in 2003, the aspect of all marketable *Gerbera* traits was of higher importance, as the inventors approach the marketing stage of the leafy flower stem product. Accordingly, an increase in the amount of crossings was made in order to produce a marketable product as soon as possible.

In a total of 64 crosses made, 62 resulted in promising seedlings. 28 crosses were leafy type x leafy type, of which only 2 did not result into seedlings. 36 crosses were leafy type x non-leafy type, of which all resulted into seedlings. All of the leafy x leafy crosses have yielded leafy types in the off spring, varying from 5.6% to 100% leafy type seedlings, with an average of 52%. The lowest percentage (5.6%) occurred twice, and in both cases, the crosses used the first generation seedling BL03.713 which originates from two non-leafy types.

11 crosses from the 36 leafy x non-leafy type crosses did not show any category 0-1 size leaves (30.6%). 7 crosses showed category 0-1 size leaves (19.4%), 50% showed good results with obvious leaves to a reasonable extent. Three out of 36 selected seedlings came from these crosses.

Table 11: 2003-2004 Breeding Program - Cross Information

| Cross* BLK | Mother | Father | Q/Seedlings | Q/ dead | Q/sel | % leafy | Remarks |
|---|---|---|---|---|---|---|---|
| 1 | BL00.508 | BL02.506 | 5 | 1 | 0 | 25 | |
| 2 | BL02.501 | BL02.532 | 18 | 3 | 3 | 66.7 | |
| 3 | BL02.503 | BL93.002 | 10 | 0 | 0 | 10 | |
| 4 | BL02.507 | BL02.526 | 8 | 1 | 3 | 42.8 | |
| 5 | BL02.518 | BL02.531 | 0 | | | | |
| 6 | BL02.521 | BL00.501 | 8 | 0 | 1 | 100 | |
| 7 | BL02.521 | BL02.504 | 8 | 0 | 1 | 87.5 | |
| 8 | BL02.521 | BL02.523 | 4 | 1 | 0 | 66.7 | |
| 9 | BL02.503 | M99.534 | 20 | 0 | 0 | 35 | |
| 10 | BL02.521 | SP01.578 | 18 | 0 | 0 | 5.6 | |
| 11 | BL03.713 | SP01.578 | 18 | 2 | 0 | 0 | |
| 12 | BL03.719 | 3856 | 4 | 0 | 0 | 0 | |

(continued)

| Cross* BLK | Mother | Father | Q/Seedlings | Q/ dead | Q/sel | % leafy | Remarks |
|---|---|---|---|---|---|---|---|
| 13 | BL03.720 | M99.534 | 18 | 0 | 0 | 11.1 | |
| 14 | BL00.503 | BL02.501 | 18 | 0 | 1 | 66.7 | |
| 15 | BL00.508 | BL02.517 | 16 | 2 | 2 | 42.8 | |
| 16 | BL00.503 | BL03.705 | 18 | 1 | 2 | 64.7 | = BLK 48 |
| 17 | BL00.503 | M03.501 | 18 | 0 | 0 | 5.5 | |
| 18 | BL02.527 | 3856 | 18 | 0 | 0 | 0 | Cat. 0-1 |
| 19 | BL00.508 | M03.523 | 18 | 0 | 0 | 0 | Cat. 0-1 |
| 20 | BL02.523 | M03.510 | 18 | 0 | 0 | 0 | Cat. 0-1 |
| 21 | BL00.504 | BL03.705 | 18 | 1 | 3 | 44.4 | |
| 22 | BL02.521 | BL03.713 | 18 | 0 | 0 | 5.6 | |
| 23 | BL00.501 | M01.592 | 18 | 0 | 0 | 0 | |
| 24 | BL00.501 | M03.501 | 18 | 0 | 0 | 0 | |
| 25 | BL01.507 | M00.539 | 18 | 0 | 0 | 5.6 | |
| 26 | BL02.520 | M02.510 | 10 | 0 | 0 | 20 | |
| 27 | BL01.526 | M02.515 | 18 | 0 | 0 | 5.6 | |
| 28 | BL03.709 | BL02.510 | 18 | 0 | 2 | 55.6 | |
| 29 | 3135 | BL03.705 | 16 | 1 | 0 | 0 | |
| 30 | BL00.508 | BL02.527 | 16 | 0 | 1 | 50 | |
| 31 | BL00.501 | BL02.503 | 7 | 0 | 0 | 14.3 | |
| 32 | BL02.520 | BL00.503 | 16 | 0 | 1 | 61.1 | |
| 33 | BL01.504 | M03.583 | 16 | 0 | 0 | 12.5 | |
| 34 | BL02.510 | BL94.001 | 18 | 2 | 2 | 50 | |
| 35 | BL03.711 | M03.556 | 18 | 0 | 0 | 33.3 | |
| 36 | BL03.716 | M99.534 | 18 | 0 | 0 | 33.3 | Cat. 1 |
| 37 | BL00.506 | BL03.713 | 18 | 0 | 0 | 5.6 | Cat. 1 |
| 38 | BL00.506 | M02.508 | 18 | 0 | 0 | 16.7 | |
| 39 | BL00.509 | BL03.710 | 18 | 0 | 2 | 50 | |
| 40 | BL00.509 | M03.588 | 20 | 0 | 0 | 10 | |
| 41 | BL00.502 | M02.548 | 18 | 0 | 0 | 5.6 | |
| 42 | BL02.516 | M03.526 | 18 | 0 | 0 | 0 | Cat. 0-1 |
| 43 | BL02.503 | BL03.714 | 18 | 1 | 4 | 88.2 | |
| 44 | BL02.524 | BL03.715 | 10 | 2 | 0 | 25 | Inbreeding |
| 45 | BL03.714 | M03.557 | 18 | 0 | 0 | 5.6 | |
| 46 | BL02.527 | M02.571 | 18 | 1 | 1 | 17.6 | |
| 47 | BL02.503 | M01.521 | 18 | 0 | 0 | 50 | |
| 48 | BL03.705 | BL00.503 | 18 | 1 | 1 | 76.5 | =BLK 16 |
| 49 | BL02.514 | M02.506 | 16 | 0 | 0 | 0 | |
| 50 | BL02.525 | 3135 | 10 | 0 | 0 | 60 | |

(continued)

| Cross* BLK | Mother | Father | Q/Seedlings | Q/ dead | Q/sel | % leafy | Remarks |
|---|---|---|---|---|---|---|---|
| 51 | BL02.527 | BL03.706 | 10 | 0 | 0 | 40 | |
| 52 | BL02.524 | BL03.705 | 2 | 1 | 1 | 100 | |
| 53 | BL00.508 | BL03.712 | 18 | 2 | 0 | 37.5 | |
| 54 | BL03.722 | M01.521 | 18 | 0 | 0 | 0 | |
| 55 | BL03.711 | M02.548 | 18 | 0 | 0 | 0 | |
| 56 | BL00.508 | M02.502 | 16 | 0 | 0 | 0 | |
| 57 | BL02.511 | M02.591 | 16 | 0 | 0 | 0 | |
| 58 | BL02.516 | BL03.712 | 16 | 0 | 4 | 68.8 | |
| 59 | BL02.516 | 3135 | 16 | 0 | 0 | 6.3 | |
| 60 | BL02.520 | M00.534 | 14 | 0 | 0 | 0 | Cat. 0-1 |
| 61 | BL02.523 | M98.507 | 16 | 0 | 0 | 0 | |
| 62 | BL03.716 | 3135 | 16 | 0 | 2 | 68.8 | |
| 63 | BL03.718 | BL03.712 | 0 | | | | |
| 64 | BL03.715 | M01.504 | 16 | 0 | 0 | 6.3 | |
| BLK code 'not shaded' = leafy x leafy cross (i.e., 1-8), and shaded gray = leafy x non-leafy cross (i.e., 9-13) | | | | | | | |

Seedling 3135 had been used in 1999 as well and resulted in two selected seedlings at that time. The crosses that have been performed in 2003 with seedling 3135, and further developed combining parents, showed an improved expression of the leafy flower stem trait. The resulting two seedlings were selected based on the improved criteria.

In the total set of crossings, a repeat had been carried out to independently check the accuracy and repeatability of results. Crossings BLK16 and BLK48 are from the same crossing. In both cases, a high percentage of inheritance of the leafy flower stem trait is observed (64.7% and 76.5% respectively). In both cases, the cross has resulted in selection of seedlings (2 and 1 respectively). These results demonstrated to the inventors that repeatability is favorable.

Cross BLK44 involves a small inbreeding coefficient. The parents of BL02.524 are BL93.002 and BL00.503. The parents of BL03.715 are BL93.002 and BL02.511. The inbreeding coefficient (F):

$$= \Sigma (1/2)^{(Nm+Nf+1)} * (1 + F_A)$$

$$= \Sigma (1/2)^{(2+2+1)} * (1 + 0)$$

$$= 3.1\%,$$

which is not significant to demonstrate to the inventors an inbreeding problem within *Gerbera.* The further effects of the inbreeding coefficient is not strong enough to make any further comments.

The selected seedlings have improved significantly in the expression of the leafy flower stem trait as evidenced in Table 12.

Table 12: 2003-2004 Breeding Program - Promising seedlings from crosses of Table 11

| Code | Cross BLK | Overall | Leaf size | | | Flower size | Color | Type | Centre |
|---|---|---|---|---|---|---|---|---|---|
| | | | at base | middle | at top | | | | |
| BL04.701 | 14 | 3 | 3 | 4 | 2.5 | mini | yellow | s | Gc |
| BL04.702 | 15 | 3 | 0 | 4 | 2.5 | mini | soft pink | sd | Gc |
| BL04.703 | 28 | 4 | 0 | 4 | 2.5 | mini | yellow-gold | s | Be |

(continued)

| Code | Cross BLK | Overall | Leaf size | | | Flower size | Color | Type | Centre |
|------|-----------|---------|---|---|---|-------------|-------|------|--------|
| BL04.704 | 28 | 4 | 0 | 4 | 2.5 | midi | yellow | s | Be |
| BL04.705 | 39 | 3 | 0 | 3.5 | 1 | big | red | s | Be |
| BL04.706 | 43 | | | | | | lilac | s | Be |
| BL04.707 | 43 | 2 | 0 | 4 | 1.5 | mini | pink | s | Bc |
| BL04.708 | 46 | 2 | 2 | 2 | 1 | mini | yellow | s | Gc |
| BL04.709 | 15 | 2 | 4 | 2 | 1 | mini | red | sd | Gc |
| BL04.710 | 58 | | | | | | pink | s | Gc |
| BL04.711 | 58 | | | | | | orange | s | Gc |
| BL04.712 | 6 | 3 | 4 | 3.5 | 2.5 | mini | soft yellow | s | Gc |
| BL04.713 | 2 | 3 | 5 | 3 | 1 | mini | orange | s | Bc |
| BL04.714 | 16 | | | | | | red | sd | Bc |
| BL04.715 | 2 | | | | | | peach | sd | Gc |
| BL04.716 | 4 | | | | | | purple | s | Bc |
| BL04.717 | 4 | | | | | | yellow | s | Gc |
| BL04.718 | 16 | | | | | | lilac | s | Gc |
| BL04.719 | 21 | 3 | 5 | 2 | | mini | orange | s | Bc |
| BL04.720 | 21 | | | | | | yellow | sd | gc |
| BL04.721 | 30 | 3 | 0 | 5 | 1 | mini | yellow | sd | gc |
| BL04.722 | 32 | | | | | | lilac | s | Bc |
| BL04.723 | 34 | | | | | | pink | sd | gc |
| BL04.724 | 43 | | | | | | lilac | s | gc |
| BL04.725 | 43 | 3 | 0 | 4 | | mini | pink | s | bc |
| BL04.726 | 48 | | | | | | red | s | gc |
| BL04.727 | 58 | 3 | 0 | 4 | 1 | mini | soft red | s | gc |
| BL04.728 | 58 | | | | | | soft pink | s | gc |
| BL04.729 | 2 | 4 | 0 | 4 | 1 | mini | peach | sd | gc |
| BL04.730 | 4 | | | | | | purple | sd | bc |
| BL04.731 | 34 | | | | | | mix | s | Gc |
| BL04.732 | 39 | 2 | 0 | 5 | 1 | mini | red/purple | s | Bc |
| BL04.733 | 52 | | | | | | red | sd | Bc |
| BL04.734 | 62 | 2 | 1 | 3.5 | 1 | mini | purple | d | Bc |
| BL04.735 | 62 | | | | | | red | s | Bc |
| BL04.736 | 7 | | | | | | yellow | Sd | Gc |
| BL04.737 | 21 | 3 | 0 | 5 | 1 | mini | red | Sd | Gc |

<u>2004</u>

Every year of the breeding program, the inventors focused on increasing both the quantity and size of the leaves on the flower stems of the new GERFOLIA™ *Gerbera L.* seedlings. A primary goal of the inventors for the 2004-2005 breeding program was to increase both the number and size of the leaves on the flower stems of new GERFOLIA™ *Gerbera L.* seedlings. From the 54 crosses of the 2004-2005 breeding program, the inventors selected 33 promising seedlings, all of which exhibited leafy flower stems with an increased number of leaves that were larger in size, as provided in Table 13. In addition, for the first time, the inventors observed crenated leaves, which resemble rosette leaves, on the leafy flower stems of the selected *Gerbera L.* seedlings.

Table 13: 2004-2005 Breeding Program- Parental Crosses & Promising Seedlings

| Cross BLK | Mother | Father | Q/Seedlings | Q/selected | Selected seedlings | % leafy of crossing |
|---|---|---|---|---|---|---|
| 1 | BL04.701 | BL04.712 | 10 | 0 | | 90 |
| 2 | BL04.701 | BL04.723 | 16 | 0 | | 87.5 |
| 3 | BL04.701 | BL02.527 | 18 | 1 | BL05.718 | 100 |
| 4 | BL04.701 | BL01.504 | 8 | 0 | | 25 |
| 5 | BL04.703 | BL03.702 | 18 | 1 | BL05.719 | 72.2 |
| 6 | BL04.703 | BL02.523 | 6 | 0 | | 50 |
| 7 | BL04.703 | BL04.732 | 16 | 0 | | 56.25 |
| 8 | BL04.704 | BL04.712 | 18 | 5 | BL05.701 | 160 |
| | | | | | BL05.706 | |
| | | | | | BL05.720 | |
| | | | | | BL05.721 | |
| | | | | | BL05.722 | |
| 9 | BL04.705 | BL04.726 | 18 | 3 | BL05.707 | 72.2 |
| | | | | | BL05.708 | |
| | | | | | BL05.723 | |
| 10 | BL04.705 | BL02.526 | 10 | 1 | BL05.724 | 70 |
| 11 | BL04.705 | BL94.001 | 16 | 1 | BL05.725 | 75 |
| 12 | BL04.706 | BL03.722 | 8 | 0 | | 75 |
| 13 | BL04.706 | BL04.730 | 18 | 0 | | 72.2 |
| 14 | BL04.708 | BL03.701 | 18 | 0 | | 50 |
| 15 | BL04.710 | BL04.716 | 18 | 1 | BL05.714 | 38.8 |
| 16 | BL04.712 | BL04.713 | 18 | 0 | | 66.7 |
| 17 | BL04.712 | BL04.723 | 18 | 1 | BL05.709 | 88.8 |
| 18 | BL04.712 | BL02.532 | 18 | 1 | BL05.704 | 100 |
| 19 | BL04.713 | BL04.719 | 3 | 0 | | 100 |
| 20 | BL04.713 | BL03.714 | 9 | 0 | | 66.7 |
| 21 | BL04.714 | BL02.526 | 16 | 0 | | 68.7 |
| 22 | BL0.715 | BL04.717 | 16 | 0 | | 100 |
| 23 | BL04.716 | M90.052 | 20 | 0 | | 50 |
| 24 | BL04.716 | BL03.722 | 8 | 0 | | 50 |
| 25 | BL04.717 | BL04.721 | 20 | 0 | | 75 |
| 26 | BL04.717 | BL04.727 | 18 | 1 | BL05.716 | 94.4 |
| 27 | BL04.717 | BL03.704 | 2 0 | | | 50 |
| 28 | BL04.718 | BL04.724 | 6 | 0 | | 66.7 |

(continued)

| Cross BLK | Mother | Father | Q/Seedlings | Q/selected | Selected seedlings | % leafy of crossing |
|---|---|---|---|---|---|---|
| 29 | BL04.720 | BL03.704 | 18 | 0 | | 55.5 |
| 30 | BL04.721 | BL03.701 | 18 | 1 | BL05.726 | 83.3 |
| 31 | BL04.721 | BL04.725 | 16 | 1 | BL05.727 | 100 |
| 32 | BL04.721 | BL03.709 | 5 | 0 | | 80 |
| 33 | BL04.722 | BL03.722 | 18 | 1 | BL05.710 | 66.7 |
| 34 | BL04.722 | BL04.723 | 20 | 2 | BL05.728 | 75 |
| | | | | | BL05.733 | |
| 35 | BL04.724 | BL02.526 | 16 | 0 | | 75 |
| 36 | BL04.724 | BL03.705 | 10 | 0 | | 70 |
| 37 | BL04.725 | BL04.727 | 18 | 1 | BL05.702 | 100 |
| 38 | BL04.725 | BL02.516 | 7 | 0 | | 71.4 |
| 39 | BL04.726 | BL02.527 | 4 | 1 | BL05.729 | 75 |
| 40 | BL04.726 | BL02.507 | 18 | 0 | | 88.8 |
| 41 | BL04.727 | BL03.706 | 18 | 1 | BL05.717 | 100 |
| 42 | BL04.728 | BL00.507 | 18 | 1 | BL05.730 | 66.7 |
| 43 | BL04.728 | BL02.521 | 2 | 1 | BL05.705 | 50 |
| 44 | BL04.728 | M90.052 | 20 | 1 | BL05.731 | 40 |
| 45 | BL02.516 | BL03.706 | 12 | 3 | BL05.703 | 83.3 |
| | | | | | BL05.711 | |
| | | | | | BL05.712 | |
| 46 | BL02.516 | BL02.525 | 18 | 2 | BL05.713 | 66.7 |
| | | | | 0 | BL05.732 | |
| 47 | BL02.516 | BL02.523 | 6 | 0 | | 33 |
| 48 | BL02.528 | BL03.704 | 9 | 1 | BL05.715 | 33 |
| 49 | BL04.734 | BL02.503 | 12 | | | 66.7 |
| 50 | BL04.734 | BL04.723 | 21 | | | 80.9 |
| 51 | M01.553 | BL02.503 | 5 | | | 60 |
| 52 | M01.553 | BL04.701 | 3 | | | 33 |
| 53 | M01.553 | BL03.701 | 1 | | | 0 |
| 54 | M01.553 | BL03.706 | 12 | | | 50 |

<u>2005</u>

A primary goal of the inventors for the 2005-2006 breeding program was to expand the range of *Gerbera L.* flower head colors, by successfully introgressing the leafy flower stem trait into *Gerbera L.* with bright, primary-colored flower heads.

Another goal of the inventors for the 2005-2006 breeding program was to produce more leaves which are larger in size and surround the flower head, since larger leaves around the flower head provides a better presentation of the *Gerbera* flower in a bunch. Two advantages of producing more leaves which are larger in size and surround the flower head are: (1) additional foliage in a bouquet is not required, thus, giving a cost price reduction to the floral trade, and (2) because only one cut flower species is in the bunch, it can be optimally treated to improve vase life.

From the 43 crosses conducted during the 2005-2006 breeding program, the inventors selected 40 promising *Gerbera L.* seedlings, all of which exhibited leafy flower stems with an increased number of leaves that were larger in size, as provided in Table 14. The inventors' success of introgressing the leafy flower stem trait into *Gerbera L.* plants the produce many, large-sized leaves per flower stem allowed for both (1) selection of better GERFOLIA™ *Gerbera L.* seedling types than was possible in previous growing years, and (2) replacement of *Gerbera L.* seedlings which only produce a few, very small or small-sized leaves per flower stem.

Table 14: 2005-2006 Breeding Program- Parental Cross & Promising Seedlings

| Cross BLK | Mother | Father | Q/Seedlings | Q/selected | Selected seedlings | % leafy of crossing |
|---|---|---|---|---|---|---|
| 1 | M01.533 | BL04.727 | 8 | 0 | | 25 |
| 2 | M01.533 | BL04.712 | 10 | 0 | | 60 |
| 3 | M01.533 | BL04.713 | 2 | 0 | | 50 |
| 4 | M01.533 | BL05.720 | 1 | 0 | | 0 |
| 5 | M02.589 | M94.071 | 16 | 0 | | 0 |
| 6 | BL03.702 | BL04.713 | 10 | 0 | | 80 |
| 7 | BL03.702 | BL04.719 | 12 | 1 | BL06.721 | 75 |
| 8 | BL03.703 | BL02.526 | 10 | 0 | | 60 |
| 9 | BL03.703 | M05.580 | 16 | 0 | | 0 |
| 10 | BL03.703 | M05.640 | 16 | 0 | | 31.25 |
| 11 | BL03.703 | BL05.710 | 2 | 0 | | 0 |
| 12 | BL03.703 | BL05.730 | 18 | 1 | BL06.722 | 83.3 |
| 13 | BL03.713 | M02.589 | 16 | 0 | | 12.5 |
| 14 | M05.584 | M02.589 | 16 | 0 | | 0 |
| 15 | M05.584 | BL03.713 | 12 | 0 | | 50 |
| 16 | BL04.704 | BL05.703 | 18 | 1 | BL06.739 | 83.3 |
| 17 | BL04.704 | BL04.707 | 15 | 2 | BL06.708 | 100 |
| | | | | | BL06.71 0 | |
| 18 | BL04.704 | BL04.713 | 7 | 3 | BL06.709 | 100 |
| | | | | | BL06.711 | |
| | | | | | BL06.737 | |
| 19 | BL04.704 | BL04.718 | 12 | 0 | | 75 |
| 20 | BL04.704 | BL04.719 | 20 | 0 | | 75 |
| 21 | BL04.704 | BL04.720 | 12 | 1 | BL06.712 | 100 |
| 22 | BL04.704 | BL04.730 | 8 | 1 | BL06.713 | 100 |
| 23 | BL04.704 | BL04.734 | 18 | 0 | | 50 |
| 24 | BL04.704 | BL05.707 | 20 | 5 | BL06.706 | 95 |
| | | | | | BL06.707 | |
| | | | | | BL06.714 | |
| | | | | | BL06.738 | |
| | | | | | BL06.740 | |
| 25 | BL04.704 | BL05.727 | 16 | 0 | | 100 |
| 26 | BL04.707 | BL04.730 | 8 | 0 | | 75 |

(continued)

| Cross BLK | Mother | Father | Q/Seedlings | Q/selected | Selected seedlings | % leafy of crossing |
|---|---|---|---|---|---|---|
| 27 | BL04.707 | BL05.704 | 13 | 0 | | 100 |
| 28 | BL04.707 | BL05.724 | 6 | 2 | BL06.723 | 100 |
| | | | | | BL06.724 | |
| 29 | BL04.712 | BL04.727 | 16 | 1 | BL06.701 | 100 |
| 30 | BL04.712 | BL05.718 | 18 | 1 | BL06.715 | 83.3 |
| 31 | BL04.713 | BL00.508 | 6 | 0 | | 50 |
| 32 | BL04.719 | BL00.508 | 8 | 0 | | 50 |
| 33 | BL04.729 | BL05.701 | 16 | 0 | | 75 |
| 34 | BL04.730 | M01.521 | 18 | 0 | | 50 |
| 35 | BL04.730 | M04.563 | 18 | 0 | | 16.6 |
| 36 | BL05.701 | BL05.703 | 18 | 6 | BL06.702 | 100 |
| | | | | | BL06.703 | |
| | | | | | BL06.704 | |
| | | | | | BL06.716 | |
| | | | | | BL06.717 | |
| | | | | | BL06.725 | |
| 37 | BL05.703 | BL04.707 | 12 | 1 | BL06.705 | 100 |
| 38 | BL05.703 | BL04.718 | 6 | 1 | BL06.726 | 83.3 |
| 39 | BL05.703 | BL05.724 | 18 | 6 | BL06.718 | 94.4 |
| | | | | | BL06.719 | |
| | | | | | BL06.727 | |
| | | | | | BL06.728 | |
| | | | | | BL06.733 | |
| | | | | | BL06.734 | |
| 40 | BL05.703 | BL05.727 | 16 | 4 | BL06.729 | 16 |
| | | | | | BL06.730 | |
| | | | | | BL06.732 | |
| | | | | | BL06.735 | |
| 41 | BL05.704 | BL04.727 | 12 | 1 | BL06.720 | 91.6 |
| 42 | BL05.707 | BL04.733 | 2 | | | 100 |
| 43 | BL05.716 | BL05.727 | 9 | 2 | BL06.731 | 77.7 |
| | | | | | BL06.736 | |

2006

For commercialization purposes, if the leafy flower stem *Gerbera L.* plants are to be sold in the form of a mono-bouquet, it is advantageous that both the flower head diameter not be small nor the leaves close to and surrounding the flower head, since more flower stems are required to fill a bouquet when the flower head and/or the leaves close to and surrounding the flower head are small. In addition, when the flower head diameter and/or leaves close to and surrounding the flower head are larger, the leafy flower stem *Gerbera L.* plants have a better market presentation. Accordingly, a primary goal of the inventors for the 2006-2007 breeding program was to expand the range of available *Gerbera L.* flower head sizes, by successfully introgressing the leafy flower stem trait into *Gerbera L.* with flower head diameters of over 12 cm., and a secondary goal was to increase the size of the leaves per flower stem, and in particular, the leaves close to and surrounding the *Gerbera L.* flower head.

No less than 109 crossings were made during the 2006-2007 breeding program. The inventors selected 170 *Gerbera L.* seedlings, all of which exhibited leafy flower stems with an increased number of leaves that were larger in size. The selected leafy flower stem *Gerbera L.* seedlings of this breeding program, will allow for future successful breeding of leafy flower stem *Gerbera L.* seedlings with many, very big leaves per flower stem, since crossing of a leafy flower stem *Gerbera L.* seedling resulting from the 2006-2007 breeding program to a non-leafy *Gerbera L.* seedling leads to a leafy flower stem *Gerbera L.* seedling in about 3 generations.

Table 15 shows the available genepool in the 2006-2007 breeding program that has proven to transfer the leafy flower stem trait successfully into diverse *Gerbera L.* genetic backgrounds.

Table 15

| Code | Category | Size of Leaves (when open) | | | | Distribution Over stem | | Color | Type | Centre | Size |
|---|---|---|---|---|---|---|---|---|---|---|---|
| | Number of Leaves | At base | Middle | At top | Overall | In Bud Stage | Open Flower Stage | | | | |
| BL93.002 | 3 | 0 | 2 | 1 | 2 | 3 | 3 | Orange - light | Semi-double | Green | |
| BL94.001 | 3 | 0 | 3.5 | 1 | 2 | 3 | 3 | Yellow-light | Semi-double | Green | |
| BL00.501 | 3 | 0 | 3.5 | 1 | 2 | 3 | 3 | Yellow | Single | Green | |
| BL01.513 | 2 | 0 | 2 | 1 | 2 | 4 | 3 | Yellow | Single | Green | Big |
| BL02.516 | 3 | 0 | 2 | 1 | 2 | 3 | 3 | Pink - soft | Single | Green | |
| BL02.521 | 3 | 0 | 2 | 1 | 2 | 3 | 3 | White | Single | Green | |
| BL02.524 | 3 | 0 | 3 | 1 | 2 | 3 | 3 | Red | Semi-double | Black | |
| BL02.507 | 2 | 0 | 2.5 | 1 | 2 | 4 | 3 | Yellow | Double | Black | Midi/big |
| BL02.528 | 3 | 0 | 3.5 | 1 | 3 | 4 | 3 | Mix | Double | Green | Big/round |
| BL02.532 | 3 | 0 | 3.5 | 1 | 2 | 3 | 3 | Orange/Pink | Semi-double | Green | Pico |
| BL03.703 | 3 | 0 | 1 | 1 | 1 | 3 | 3 | White | Semi-double | Green | |
| BL04.702 | 3 | 0 | 4 | 2.5 | 3 | 3 | 2 | Pink-soft | Semi-double | Green | |
| BL04.704 | 4 | 0 | 4 | 2.5 | 4 | 4 | 3 | Yellow-Gold | Single | Black | Midi |
| BL04.705 | 3 | 0 | 3.5 | 1 | 3 | 4 | 3 | Red | Single | Black | big |
| BL04.707 | 3 | 0 | 4 | 2.5 | 2 | 5 | 3 | Pink | Single | Black | |
| BL04.709 | 3 | 4 | 2 | 1 | 2 | 2 | 2 | Red | Semi-double | Green | |
| BL04.712 | 4 | 4 | 3.5 | 1 | 3 | 2 | 2 | Yellow | Single | Green | |
| BL04.713 | 4 | 5 | 3 | 1 | 3 | 2 | 2 | Orange | Single | Black | |
| BL04.719 | 3 | 5 | 2 | 1 | 3 | 2 | 2 | Orange | Semi-double | Black | |
| BL04.721 1 | 2 | 0 | 5 | 1 | 3 | 3 | 3 | Yellow | Single | Green | |
| BL04.725 | 2 | 0 | 4 | 1 | 3 | 3 | 3 | Pink | Single | Black | |
| BL04.727 | 3 | 0 | 4 | 1 | 3 | 3 | 3 | Soft Red | Semi-double | Green | |
| BL04.729 | 2 | 0 | 4 | 1 | 4 | 3 | 3 | Peach | Semi-double | Green | |

EP 2 011 388 A1

| Code | Category | Size of Leaves (when open) | | | | Distribution Over stem | | Color | Type | Centre | Size |
|------|----------|---------|--------|--------|---------|--------------|-------------------|-------|------|--------|------|
| | Number of Leaves | At base | Middle | At top | Overall | In Bud Stage | Open Flower Stage | | | | |
| BL04.732 | 3 | 0 | 5 | 1 | 3 | 3 | 3 | Red/Purple | Semi-double | Black | |
| BL04.733 | | | | | 3 | | | | | | |
| BL04.734 | 3 | 1 | 3.5 | 1 | 3 | 2 | 2 | Purple | Double | Black | |
| BL04.737 | 2 | 0 | 5 | 1 | 4 | 3 | 3 | Red | Semi-double | Green | |
| BL05.701 | 3 | 3.5 | 4 | 2.5 | 4 | 3 | 2 | Yellow | Single | Green | |
| BL05.702 | 3 | 0 | 4 | 1 | 3 | 4 | 3 | Red/pink | Single | Black | |
| BL05.703 | 3 | 0 | 4 | 1 | 3 | 4 | 3 | Lilac | Single | Green | |
| BL05.704 | 2 | 0 | 4 | 1 | 3 | 3 | 3 | Pink-soft | Semi-double | Green | |
| BL05.705 | 2 | 0 | 3 | 1 | 3 | 4 | 3 | White | Single | Green | |
| BL05.706 | 3 | 0 | 4 | 1 | 3 | 4 | 3 | Yellow | Single | Green | |
| BL05.707 | 3 | 1 | 4 | 1 | 3 | 3 | 2 | Red | Single | Black | |
| BL05.709 | 3 | 0 | 4 | 1 | 3 | 3 | 3 | Yellow-light | Single | Green | |
| BL05.710 | 3 | 3.5 | 3.5 | 1 | 3 | 2 | 2 | Pink/white | Semi-double | Black | |
| BL05.711 | 3 | 0 | 4 | 1 | 3 | 4 | 3 | Pink | Single | Green | |
| BL05.712 | 3 | 4 | 2 | 1 | 3 | 3 | 2 | Yellow-gold | Single | Green | |
| BL05.714 | 3 | 0 | 3.5 | 1 | 3 | 4 | 3 | Yellow-light | Semi-double | Green | Midi/big |
| BL05.715 | 2 | 0 | 4 | 1 | 3 | 3 | 3 | Yellow | Double | Green | Big |
| BL05.717 | 3 | 0 | 5 | 2 | 3 | 4 | 3 | Red | Semi-double | Green | |
| BL05.718 | 3 | 0 | 3.5 | 1 | 3 | 3 | 3 | Lilac | Single | Green | |
| BL05.719 | 3 | 0 | 5 | 2.5 | 3 | 4 | 3 | Yellow | Single | Green | |
| BL05.720 | 3 | 0 | 5 | 2.5 | 3 | 3 | 3 | Yellow | Single | Green | |
| BL05.721 | 3 | 0 | 5 | 3 | 4 | 4 | 3 | Yellow | Single | Green | contorta |
| BL05.722 | 2 | 0 | 4 | 2 | 3 | 3 | 3 | Yellow | Single | Green | |

39

EP 2 011 388 A1

(continued)

| Code | Category | Size of Leaves (when open) | | | | Distribution Over stem | | Color | Type | Centre | Size |
|---|---|---|---|---|---|---|---|---|---|---|---|
| | Number of Leaves | At base | Middle | At top | Overall | In Bud Stage | Open Flower Stage | | | | |
| BL05.723 | 4 | 0 | 3.5 | 1 | 3 | 3 | 3 | Red | Single | Green | |
| BL05.724 | 3 | 0 | 3.5 | 1 | 3 | 3 | 3 | Lilac | Semi-double | Green | midi |
| BL05.725 | 3 | 0 | 5 | 1 | 4 | 5 | 3 | Red | Semi-double | Black | |
| BL05.727 | 3 | 0 | 4 | 1 | 3 | 4 | 3 | Pink | Single | Black | |
| BL05.730 | 2 | 0 | 4 | 1 | 3 | 3 | 3 | White | Semi-double | Green | |
| BL05.731 | 3 | 0 | 4 | 1 | 3 | 3 | 3 | Apricot | Single | Green | |
| BL05.732 | 2 | 0 | 3.5 | 1 | 3 | 3 | 3 | Cream | Semi-double | Black | |
| BL05.733 | 3 | 0 | 5 | 1 | 4 | 4 | 3 | Lilac | Single | Black | |
| BL06.702 | 3 | 0 | 5 | 2.5 | 4 | 3 | 3 | Pink-soft | Single | Green | |
| BL06.708 | 4 | 0 | 5 | 2.5 | 4 | 5 | 4 | Pink-soft | Single | Black | |
| BL06.713 | 3 | 1 | 3 | | 3 | 2 | 2 | Yellow-light | Single | Green | midi |
| BL06.722 | 2 0 3 1 2 | | | | | 4 | 3 | Pink-white | Semi-double | Green | |
| BL06.723 | 3 | 0 | 5 | 1 | 4 | 3 | 3 | Lilac | Single | Green | |
| BL06.727 | 2 | 0 | 3 | 1 | 2 | 4 | 3 | Pink/white | Single | Green | |
| BL06.728 | 3 | 0 | 5 | 2.5 | 4 | 3 | 3 | Lilac | Single | Green | |
| BL06.734 | 3 | 0 | 5 | 1 | 3 | 4 | 3 | Red | Double | Green | |
| BL06.740 | 3 | 0 | 4 | 1 | 3 | 4 | 3 | Orange | Single | Black | |

2007 to Present

The breeding program conducted by the inventors from 2007 to present day continues to focus on creating new *Gerbera L.* plants which exhibit the leafy flower stem trait, and have the ability to exhibit the in bud shipping trait, without loosing any of the important *Gerbera* commerical traits or reducing diversification. The inventors continue to cross leafy and non-leafy *Gerbera L.* plants and select promising *Gerbera L.* seedlings based on the expression of the new and unique leafy flower stem trait, as well as, the expression of the important *Gerbera* commercial traits.

To date the breeding program has successfully introgressed the leafy flower stem trait into *Gerbera L.* plants exhibiting different 1) flowering types (single, semi-double, double, double-multi and multi-petalled), 2) all Normal-type *Gerbera* and Mini*Gerbera* inflorescence sizes (ranging from 7 cm to over 12 cm), 3) all Normal-type *Gerbera* and Mini*Gerbera* ray floret colors (whites, creams, yellows, oranges, reds, pinks, purples and bi-colors), 4) all Normal-type and Mini*Gerbera* flower center colors (black and green/yellow), and 5) basic daisy-shaped, funnel-shaped, globular-shaped (GERRONDO™) and mono-bouquet and additional crossings are being conducted for spider-shaped *Gerbera.* In addition, the weather tolerance of GERFOLIA™ *Gerbera L.* cultivars is consistent with the weather tolerance of non-leafy *Gerbera L.* cultivars, and the pest/disease resistance and susceptibility of GERFOLIA™ *Gerbera L.* cultivars is consistent with the pest/disease resistance and susceptibility of Normal-type *Gerbera* and

*MiniGerbera.*

Since expanding the breeding program in 1999, the inventors have determined that seven generations of crossings, including crossings between half siblings, are required in order to obtain the GERFOLIA™ GERFOLIA *L.* plants exhibiting the preferred embodiment of the invention. Specifically, seven generations of crossings are necessary in order to produce new, distinct and stable cultivars of *Gerbera L.* plants that produce one or more flower stems with at least 5, to about 30, or more than 30 full or partial leaves per flower stem, which are a) small to extra large in size (ranging from at least 40 mm or more, to about 200 mm or more in length, and at least 4 mm or more, to about 60 mm or more in width), and b) which are distributed either equally along the flower stem or along the top half of the flower stem. However, the selected leafy flower stem *Gerbera L.* seedlings of this breeding program, will allow for future successful breeding of leafy flower stem *Gerbera L.* seedlings with many, very big leaves per flower stem, since crossing of a leafy flower stem *Gerbera L.* seedling resulting from the 2006-2007 breeding program to a non-leafy *Gerbera L.* seedling leads to a leafy flower stem *Gerbera L.* seedling in about 3 generations.

Plants carrying genes controlling the leafy flower stem phenotype can be selected from any *Gerbera L.* population by means of identifying a plant having one or more flower stems with at least 5 leaves per flower stem. Any one of the plants identified in the *Gerbera L.* population that has one or more flower stems with at least 5 leaves per flower stem can be used as a source for the gene(s) influencing/controlling the leafy flower stem trait in a breeding program with the goal of producing new *Gerbera L.* cultivars that exhibit the leafy flower stem trait, which may express the in bud shipping trait, by producing one or more flower stems with at least 5 leaves per flower stem. The degree of foliage per flower stem or plant can be predictably increased in any *Gerbera L.* background by using the methods herein described. Recurrent selection for progeny with an increased degree of foliage per flower stem has dramatically increased the degree of foliage per flower stem or plant in diverse *Gerbera L.* genetic backgrounds. Intermating of superior genotypes which exhibit increased foliage count per flower stem through repeated generations has resulted in the selection of cultivars with an increasing degree of foliage per flower stem and plant. Periodic outcrossings is done during the breeding program in order to introduce desirable characteristics and to circumvent inbreeding depression.

Further, it has been proven in 2005 that the leafy flower stem trait remains after asexual propagation via cuttings and tissue culture. All GERFOLIA™ *Gerbera L.* cultivars, exhibiting the leafy flower stem trait have been proven to be stable through asexual propagation via cutting and tissue culture. It is also expected that GERFOLIA™ *Gerbera* cultivars can be produced as progeny from sexual crosses and sold as seed.

4. Seed Deposit with International Deposit Authority

Seeds from a *Gerbera L.* selection containing the leafy flower stem trait were deposited in the American Type Culture Collection (ATCC), P.O. Box 1549, Manassas, VA 20108, U.S.A., and accorded ATCC deposit accession number PTA-8443. 925 seeds were deposited with the ATCC on May 17, 2007. The deposited seeds produce *Gerbera L.* plants that can be crossed, either as the male or female parent, to diverse genetic backgrounds of single-type, semi-double-type, double-type, double-multi-type or multi-petalled-type *Gerbera L.* species to reproducibly and predictably produce new *Gerbera L.* selections exhibiting the leafy flower stem trait, which may have the ability to be shipped during the in bud stage of development, according to the methods described herein.

5. In Bud Shipping Trait

The breeding of *Gerbera L.* cultivars, exhibiting the leafy flower stem trait, which produce one or more flower stems with at least 5 or more full or partial leaves per flower stem, offers an economic advantage in the commercial horticultural industry since the new type of leafy *Gerbera L.* flower stem may have the ability to be shipped in the bud stage rather than in the open flower stage which is typical for *Gerbera L.* cultivars with non-leafy flower stems. The surface area of a bunch of GERFOLIA™ *Gerbera L.* flowers which can be shipped in bud stage is less than the surface area of the same bunch count of flowers of any typical *Gerbera L.* cultivars which are shipped in the open flower stage. Accordingly, standard commercial packages containing GERFOLIA™ *Gerbera L.* flowers which are shipped in the bud stage have a larger packing density compared to standard commercial packages containing any typical *Gerbera* cultivars shipped in the open flower stage. Second, the leafy flower stem trait provides further economic advantage by i) reducing the overall production cost with an earlier cutting/harvest time, and ii) extending the allowable time for commercial transportation and distribution. The foliage on the leafy flower stem provide nutrients for subsequent growth (opening of flower), and in turn, increases the holding quality of the flower stem. In turn, GERFOLIA™ *Gerbera L.* plants, which have the ability to be shipped during the in bud stage of development, have the potential to have a significant advantage in the commercial market, since commercial distribution may be extended from a local or regional market to an international market.

The intended cutting point for transport and trading in bud of a GERFOLIA™ *Gerbera L.* is indicated in Table 16 as Stage 3, and shown in FIG. 8A and 8G, as well as, closE-up views shown in FIG. 9A and 9B. The other stages are depicted to show the development and size of an average bud, and are indicated in Table 16 and shown in FIGS. 8A-8J.. In stage 3, depending on flower type, the size of the bud varies in height from about 30 mm to 40 mm, and in width from about 25 mm to 35 mm. The time from planting of a seedling until development of buds at Stage 3 is about 21 days. The time from planting of a seedling until development of buds at Stage 4/5 is about 25 days. Accordingly, the process time until a GERFOLIA™ *Gerbera L.* cultivar is ready for cutting and transport after planting of a seedling is shortened by about 4 days when compared to the process time until a typical, non-leafy *Gerbera L.* cultivar is ready for cutting and transport after planting of a seedling.

TABLE 16: Depiction of Bud Development (Stages 1-5)

| View Perspective | Stage 1 | Stage 2 | Stage 3 | Stage 3,5 | Stage 4 | Stage 5 |
|---|---|---|---|---|---|---|
| Side view | As Shown in FIG. 8A | As Shown in FIG. 8B | As Shown in FIG. 8C | As Shown in FIG. 8D | | |
| Top view | As Shown in FIG. 8E | As Shown in FIG. 8F | As Shown in FIG. 8G | As Shown in FIG. 8H | As Shown in FIG. 8I | As Shown in FIG. 8J |

A GERFOLIA™ *Gerbera L.* flower stem which has the ability to be shipped during the in bud stage of development is less prone to breakage and generally associated with a longer shelf life. The in bud shipping trait also allows for GERFOLIA™ *Gerbera L.* cultivars to be shipped when the flower stems are more compact and uniform in size. Further, when GERFOLIA™ *Gerbera L.* cultivars have the ability to be shipped during the in bud stage of development, it is more economical to grow and process *Gerbera L.* flower stems, since a larger number of GERFOLIA™ *Gerbera* flower stems can be packaged to fill a container in comparison to the non-leafy *Gerbera L.* cultivars. In addition, diminished damage to GERFOLIA™ *Gerbera L.* flower stems during shipment occurs because of the natural protection provided by the leaves on the flower stem, also making GERFOLIA™ *Gerbera L.* cultivars economically advantageous to non-leafy *Gerbera L.* cultivars. Lastly, the leafy flower stem *Gerbera L.* of the present invention which have the ability to be shipped during the in bud stage of development, provide a new and unique *Gerbera L.* end product in the commerical market due to i) the leafy flower stem phenotype, and ii) the ability for the flowers on the flower stem to open completely in a vase after purchase by the consumer.

Tables 17 and 18 show a comparision of packaging density among Gerfoia™ *Gerbera L.* cultivars and non-leafy Normal-type and Mini*Gerbera* cultivars in both dry transport and water transport.

TABLE 17: Dry Transport Box Number Comparison

| Box Dimensions (cm) | Typical *Gerbera* Ship In Open Flower Stage (Inflorescence Diameter: 10-12 cm) | *MiniGerbera* Ship In Open Flower Stage (Inflorescence Diameter: 7-9 cm) | New GERFOLIA™ *Gerbera* Ship In Bud Stage (Bud Width: 2.5-3.5 cm) | Example of Dry Transport Box Comparison |
|---|---|---|---|---|
| 100 x 30 x 12 | 50 Flower Stems 2 x 25 flower stems | N/A | Minimum of 160 Flower Stems 8 x 20 flower stems | Shown in FIG. 10A |
| 100 x 20 x 10 | N/A | 60 Flower Stems 2 x 30 flower stems | Minimum of 120 Flower Stems 6 x 20 flower stems | Shown in FIG. 10B |

TABLE 18: Water Transport (By Truck) Comparison

| Container Dimensions (cm) | Typical *Gerbera* Ship In Open Flower Stage (Inflorescence Diameter: 10-12 cm) | Mini*Gerbera* Ship In Open Flower Stage (Inflorescence Diameter: 7-9 cm) | New GERFOLIA™ *Gerbera* Ship In Bud Stage (Bud Width: 2.5-3.5 cm) |
|---|---|---|---|
| L30 x W25 x H24 | 50 Flower Stems per Container | 200 Flower Stems per Container | 250 Flower Stems per Container |
| Example of Water Transport Comparison (Side View) | Shown in FIG. 11A | Shown in FIG. 11A | Shown in FIG. 11A |
| Example of Water Transport Comparison (Top View) | Shown in FIG. 11B | Shown in FIG. 11B | Shown in FIG. 11B |

6. Plant Growth Conditions

In the present invention, GERFOLIA™ *Gerbera L.* seedlings were grown in coco peat mixture in pots with a diameter of 19 cm. No growth retardants were used, and only chemicals typical to keeping *Gerbera L.* plant material free of pests and diseases was used.

In the present invention, GERFOLIA™ *Gerbera L.* meristem plants were grown in coco peat mixture in pots with a diameter of 19 cm, in a greenhouse with an average temperature of 18˚C-20˚C, during the day the temperature averages about 20˚C, and during the night the temperature averages about 16˚C. The plants were grown under natural light conditions supplemented with artificial light. When natural light is less than 250 W/m$^2$, artificial light is used, increasing the light intensity to 750 W/m$^2$. When natural light intensities exceed 750 W/m$^2$, screens will be closed or the greenhouse will be whitewashed if the high intensity is expected to last long. Plants were grown in a coco-peat based soil mix and were watered with a solution i) containing 0.8 mmol/l ammonium, 1.0 mmol/l potassium, 0.9 mmol/l natrium, 2.0 mmol/l calcium, 0.5 mmmol/l magnesium, less than 0.2 mmol/l silicium, 1.0 mmol/l nitrate, 0.5 mmol/l chloride, 1.1 mmol/l sulphate, less than 0.2 mmol/l bicarbonate, 0.5 mmol/l phosphate, 10 μmol/l iron, 7.0 μmol/l manganese, 5.0 μmol/l zinc, 25 μmol/ 1 borium, 1.3 μmol/l copper, and 0.8 μmol/l molybdeen, ii) pH of 5.5-5.7, and iii) EC of 1.0 mS/cm.

Environmental stress factors which may affect the leafy flower stem trait of *Gerbera L.* plants, may include changes in temperature, water availability and/or light conditions during the growth process. No experiments to provoke stress factors and determine the effects statistically have been carried out among *Gerbera L.* plants exhibiting the leafy flower stem trait. However, the following observations concerning high temperature and reduce light conditions have been observed during the growing trials of *Gerbera L.* plants exhibiting the leafy flower stem trait. High temperatures up to 30˚C have been present during the testing phases of *Gerbera L.* plants exhibiting the leafy flower stem trait and have not proven to result in any differences in leaf quality or quantity. The stress factor of lack of light has only resulted in thinner flowers; this result is the same for typical *Gerbera L.* plants.

The following examples are set forth as representative of the specific and preferred embodiments of the present invention. These examples are not to be construed as limiting the scope of the invention in any manner. It should be understood that many variations and modifications can be made while remaining within the spirit and scope of the invention.

7. Examples

Example 1: Genetic Data

The data from about seven generations of crosses with *Gerbera L.* indicate that the leafy flower stem trait is a recessive multiple allele trait. Further, data from the 2006-2007 breeding program indicate that the selected GERFOLIA™ *Gerbera L.* seedlings of this breeding program , will allow for future successful breeding of GERFOLIA™ *Gerbera L.* seedlings with very many, very big leaves, since crossing of a GERFOLIA™ *Gerbera L.* seedling resulting from the 2006-2007 breeding program to a non-leafy *Gerbera L.* seedling will lead to a GERFOLIA™ *Gerbera L.* seedling in about 3 generations. Regardless of what the genetic basis for control of the leafy flower stem trait in *Gerbera L.* might be, these data demonstrate that the leafy flower stem trait can be reproducibly and predictably introgressed into diverse *Gerbera L.* genetic backgrounds, when crossing with a known parent.

Example 2: Crossing and Selfing Data

Preliminary results of crossings of *Gerbera L.* plants exhibiting the leafy flower stem trait, have revealed progeny yielding larger-sized foliage. Selfing of *Gerbera L.* plants exhibiting the leafy flower stem trait has been carried out since 2007, in Kudelstaart, The Netherlands.

In the breeding program of the present invention, a high probability of inbreeding existed because of the initial, limited gene-pool among the leafy flower stem *Gerbera L.* cultivars. Accordingly, a high priority of the breeding program was to avoid inbreeding by creating a gene-pool as broad as possible. Therefore, the leafy stem trait of the present invention was introgressed into diverse *Gerbera L.* backgrounds, by crossing leafy flower stem *Gerbera L.* cultivars with *Gerbera L.* cultivars exhibiting different 1) flowering types (single, semi-double, double, double-multi and multi-petalled), 2) all Normal-type *Gerbera* and *MiniGerbera* inflorescence sizes (ranging from 7 cm to over 12 cm), 3) all Normal-type *Gerbera* and Mini*Gerbera* ray floret colors (whites, creams, yellows, oranges, reds, pinks, purples and bi-colors), 4) all Normal-type and Mini*Gerbera* flower center colors (black and green/yellow), and 5) basic daisy-shaped, funnel-shaped, globular-shaped (GERRONDO™) and mono-bouquet.

The inventors selected specific *Gerbera L.* crossings to discuss to show that the inventors can successfully introgress the leafy stem trait of the present invention into diverse *Gerbera L.* genetic backgrounds. Table 19 provides the distribution and different leaf sizes of these selected *Gerbera L.* crossings. From the *Gerbera L.* crossings, a seedling was chosen to represent the leaf distribution of a larger group of seedlings (i.e., Crossing 1: Offspring 1-1 as shown in FIG. 12C represents 14 seedlings from Crossing 1.)

Table 19 shows the available genepool in the 2006-2007 breeding program that has proven to transfer the leafy flower stem trait successfully.

| Parents/Offspring | Photo Example | Amount | Leaves at top, middle, base of Flower Stem |
|---|---|---|---|
| **Crossing 1** | | | |
| Mother 'BL07.328' | Shown in FIG. 12A | | 2,4,0 |
| Father ' 06.132' | Shown in FIG. 12B | | 0,0,0 |
| Offspring 1-1 | Shown in FIG. 12C | 14x | 0,0,0 |
| Offspring 1-2 | Shown in FIG. 12D | 4x | 1,1,0 |
| Offspring 1-3 | Shown in FIG. 12E | 5x | 2,2,0 |
| | | | |
| **Crossing 2** | | | |
| Mother 'BL06.702'/ TERCARE | Shown in FIG. 13A | | 4,4,0 |
| Father 'M06.587' | Shown in FIG. 13B | | 0,0,0 |
| Offspring 2-1 | Shown in FIG. 13C | 14x | 0,0,0 |

(continued)

| Parents/Offspring | Photo Example | Amount | Leaves at top, middle, base of Flower Stem |
|---|---|---|---|
| Offspring 2-2 | Shown in FIG. 13D | 6x | 1,0,0 |
| Offspring 2-3 | Shown in FIG. 13E | 3x | 1,1,0 |
|  |  |  |  |
| **Crossing 3** |  |  |  |
| Mother 'BL06.702'/ TERCAR | Shown in FIG. 14A |  | 4,4,0 |
| Father 'M05.516' | Shown in FIG. 14B |  | 1,0,0 |
| Offspring 3-1 | Shown in FIG. 14C | 3x | 1,1,0 |
| Offspring 3-2 | Shown in FIG. 14D | 2x | 1,3,0 |
|  |  |  |  |
| **Crossing 4** |  |  |  |
| Mother 'BL06.706'/ TERTRUE | Shown in FIG. 15A |  | 4,5,5 |
| Father '03.113' | Shown in FIG. 15B |  | 0,0,0 |
| Offspring 4-1 | Shown in FIG. 15C | 8x | 1,0,0 |
| Offspring 4-2 | Shown in FIG. 15D | 8x | 1,1,1 |
| Offspring 4-3 | Shown in FIG. 15E | 6x | 3,4,4 |
|  |  |  |  |
| **Crossing 5** |  |  |  |
| Mother'M01.548'/ TORPEDO | Shown in FIG. 16A |  | 0,0,0 |
| Father 'BL07.774' | Shown in FIG. 16B |  | 3,4,4 |
| Offspring 5-1 | Shown in FIG. 16C | 8x | 1,1,1 |
| Offspring 5-2 | Shown in FIG. 16D | 6x | 2,2,0 |
| Offspring 5-3 | Shown in FIG. 16E | 4x | 3,4,0 |
|  |  |  |  |
| **Crossing 6** |  |  |  |
| Mother 'M06.509' | Shown in FIG. 17A |  | 0,0,0 |
| Father 'BL07.790' | Shown in FIG. 17B |  | 4,4,0 |
| Offspring 6-1 | Shown in FIG. 17C | 18x | 1,0,0 |
| Offspring 6-2 | Shown in FIG. 17D | 5x | 2,2,0 |

Crossing 1 was made between two standard (big flowering) *Gerbera L.* plants, with a flower head size measuring over 10 cm diameter. The mother seedling 'BL07.328' as shown in FIG. 12A has a lot of leaves and was crossed with the father seedling '06.132' as shown in FIG. 12B which has no leaves. In Crossing 1, the inventors successfully introgressed the leafy flower stem trait into standard (big flowering) *Gerbera L.* Only 22% of the offspring had about the same quantity of leaves per flower stem as the parent with the highest quantity of leaves per flower stem, and 61% of the offspring had no leaves per flower stem, while only 17% of the offspring had only a few leaves per flower stem. FIGS. 12C, 12D and 12E show *Gerbera L.* seedlings resulting from Crossing 1.

These segregations are similar to many like crossings conducted by the inventors. The leafy flower stem trait does not seem to be dominant as most of the offspring have no leaves per flower stem. From Crossing 1, the inventors may conclude that it will take about 7 generations until the inventors will select a GERFOLIA™ *Gerbera L.* plant with many, large-sized leaves per flower stem.

Crossing 2 was made between two Mini*Gerbera* plants, with a flower head size measuring between 7 cm to 9 cm. The mother seedling 'BL06.702 (commercial name TERCARE) as shown in FIG. 13A has a lot of leaves both at the top and the middle of the flower stem and was crossed with father seedling 'M06.587' as shown in FIG. 13B which has no leaves. In Crossing 2, , the inventors successfully introgressed the leafy flower stem trait into Mini*Gerbera*. Only 13% of the offspring had a moderate amount of leaves per flower stem and 87% of the offspring had little or no leaves per flower stem. None of the selected seedlings had the same quantity of leaves per flower stem of the parent with the highest quantity of leaves per flower stem, which is the mother 'BL06.702'. FIGS. 13C, 13D and 13E show *Gerbera L.* seedlings resulting from Crossing 2.

Crossing 2 shows that even if one of the parents has a lot of leaves, that most of the offspring are without leaves and the ones with leaves have very small leaves. These segregations are similar to many like crossings conducted by the inventors. From Crossing 2, the inventors may conclude that it will take more than 7 generations, possibly 10 to 15 generations, until the inventors will select a GERFOLIA™ *Gerbera L.* plant with many, large-sized leaves per flower stem.

Crossing 3 was made again between two Mini*Gerbera* plants, with a flower head size measuring between 7 cm to 9 cm. The mother seedling 'BL06.702' (commercial name TERCARE) as shown in FIG. 14A has a lot of leaves both at the top and the middle of the flower stem and was crossed with father seedling 'M05.516' as shown in FIG. 14B which has very small leaves at the top of the flower stem. In Crossing 3, the inventors successfully introgressed the leafy flower stem trait into Mini*Gerbera.* FIGS. 14C and 14D show *Gerbera L.* seedlings resulting from Crossing 3. 60% of the offspring had little leaves and 40% had a bit more leaves. The 40% result is more than the 13% result of Crossing 2. Accordingly, the inventors noted that it seems that when a father seedling with a few small leaves is used in stead of a father without leaves, the result is better, as more of the offspring produce leaves on the flower stem.

Crossing 4 was made between two standard (big flowering) *Gerbera L.* plants, with a flower head size measuring over 10 cm in diameter. The mother seedling was 'BL06.706' (commercial name TERTRUE) as shown in FIG. 15A and has a lot of big leaves both at the top, the middle and the bottom of the flower stem, and was crossed with father seedling '03.113' as shown in FIG. 15B which is without any leaves per flower stem. In Crossing 4, the inventors successfully introgressed the leafy flower stem trait into standard *Gerbera L.* FIGS. 15C, 15D and 15E show *Gerbera L.* seedlings resulting from Crossing 4. Of the offspring from Crossing 4, 36% of the offspring had very little leaves, only at the top of the flower stem; another 36% had little leaves located over the whole flower stem; and 27% had a moderate amount of leaves over the whole flower stem. The seedling results of Crossing 4 reveal that it is possible to introgress the leafy flower stem trait into a *Gerbera L. plant* without any leaves per flower stem. However, the segregation of Crossing 4 is not similar to most crosses conducted by the inventors, and thus, the inventors predict that the leafy flower stem trait is based on one or more recessive gene(s).

Crossing 5 was made between the mother seedling 'M01.548' (commercial name TORPEDO) as shown in FIG. 16A and has no leaves, and was crossed with father seedling 'BL07.774' as shown in FIG. 16B which has many and large-sized leaves at the top, the middle and the bottom of the flower stem. In Crossing 5, the inventors successfully introgressed the leafy flower stem trait. FIGS. 16C, 16D and 16E show *Gerbera L.* seedlings resulting from Crossing 5. Of the offspring from Crossing 5, 44% had a few leaves over the whole flower stem, another 33% had a bit more leaves at the top and the middle of the flower stem, and 22% had a good amount of leaves at the top and the middle of the flower stem. The offspring results of Crossing 5 reveal similar offspring results as Crossing 4, and the inventors may conclude that it does not matter whether a *Gerbera L.* plant with a lot of leaves is used in the cross as either the mother or father seedling. In addition, the inventors used a parent seedling which possessed a good amount of leaves over the whole stem, and thus, the inventors noted that by using such parent seedling, a GERFOLIA™ *Gerbera L.* seedling will result in about 3 generation.

Crossing 6 was made between the mother seedling 'M06.509' as shown in FIG. 17A and has no leaves, and was crossed with father seedling 'BL07.790' as shown in FIG. 17B and has many and large-sized leaves at the top and the middle of the flower stem. In Crossing 6, the inventors successfully introgressed the leafy flower stem trait. FIGS. 17C and 17D show *Gerbera L.* seedlings resulting from Crossing 6. Of the offspring from Crossing 5, 78% of the offspring had nearly no leaves and 22% had little leaves. When the inventors look at the parental seedlings and the resulting offspring of Crossing 6, the inventors note that the leafy-flower stem trait is likely recessive.

Table 20 Results of Crossings 1-6 classified by the size and presence of leaves on the flower stem in percentage (%)

| Crossing | 1 | 2 | 3 | 4 | 5 | 6 |
|---|---|---|---|---|---|---|
| Leaves on stem | | | | | | |
| Large | 22% | | 40% | 27% | 22% | |

(continued)

| Crossing | 1 | 2 | 3 | 4 | 5 | 6 |
|---|---|---|---|---|---|---|
| Small | | | | | | |
| - Small (on top of stem) | 17% | 13% | 60% | 36% | 33% | 78% |
| - Small (whole stem) | | | | 36% | 44% | |
| Absent | 61% | 87% | | | | 22% |

Crossings 1, 2 and 4 were made between a leafy flower stem *Gerfolia L.* seedling and a *Gerbera L.* seedling without leaves on the flower stem. Crossing 3 was made between a leafy flower stem *Gerfolia L.* seedling and one of the first *Gerfolia L.* seedlings exhibiting very small leaves on the flower stem. Crossing 3 is a backcross and was the most successful of the six crossings, as 100% of the *Gerfolia L.* seedlings had leaves on the flower stem, of which 40% were large leaves (see Table 20 above). Crossing 5 and 6 were made between a leafy flower stem *Gerfolia L.* seedling and a *Gerfolia L.* seedling with a leafless flower stem. Crossings 5 and 6 yield a better result, as 100% and 78% of the seedlings had leaves on the flower stem (see Table 20 above), and this result reinforcing the prediction of a recessive trait. Crossing 4 yielded a good result comparable with Crossings 5 and 6, and the inventors note that it is not inconceivable that earlier generations of *Gerbera L.* '03.113' had small leaves on the flower stem. In addition, the inventors successfully introgressed the leafy flower stem trait into GERRONDO™ *Gerbera L.* as is shown in FIGS. 21A and 21B. Thus, the above-mentioned results show that the inventors are able to successfully introgress the leafy flower stem trait into diverse *Gerbera L.* genetic backgrounds.

Example 3: Promising GERFOLIA™ *Gerbera L.* Seedlings from the BLK06-11 Crossing

Table 21 presents the genealogy of promising GERFOLIA™ *Gerbera L.* seedlings resulting from the BLK06-11 crossing. From the BLK06-11 crossing, 22 seedlings were selected for further testing, and from the initial 22 seedlings, 7 seedlings with selected for further testing. The seedling BL07.777, from the BLK06-11 crossing, was selected for further testing, including propagation trials, and is described in detailed in Example 8 below and shown in FIGS. 23A and 23B, including it genealogy in FIG. 23C. Selection of the seedlings was based on the individual seedling's expression of the new leafy flower stem trait, together with other desirable *Gerbera L.* commercial traits. Figures 18A, 18B and 18C provide perspective side views of individual GERFOLIA™ *Gerbera L.* seedlings resulting from the BLK06-11 crossing.

Table 21

| BLK06-11 | Oseedling | Oleaves | Distribution in bud | Distribution open flower | overall | top | Size leaves open flower base | |
|---|---|---|---|---|---|---|---|---|
| OFFSPRING | 22 | 5 : 17 | 11 : 11 | 15 : 7 | 9 : 13 | 8 : 14 | 100% | Increase of amount from BL05.702 |
| | | 13-15 >15 | | | big. some pointed | big. straight | 2-4 cm, : big, straight pointed | absent | Increase of size from both parents |

| PARENTS | | Oleaves | Distribution in bud | Distribution open flower | | | Size leaves open flower | |
|---|---|---|---|---|---|---|---|---|
| 1 | BL06.702 | 9-12 | | | overall top middle base | big, some points 2-4 cm, straight big, pointed absent | | Same amount of leaves Concentration on top position Bigger size leaves |
| 1 | BL05.717 | 9-12 | | | overall top middle base | >4cm, straight 2-4 cm, straight big, pointed absent | | Bigger size of leaves Extra accent on top position |

EP 2 011 388 A1

Table 21 (cont).

| GRANDPARENTS | Q leaves | Distribution in bud | Distribution open flower | | Size leaves open flower | |
|---|---|---|---|---|---|---|
| 2 BU05.701 | 9-12 | | | overall | big, some points | Bigger size of leaves. Move leaves to top position |
| | | | | top | 2-4 cm, straight | |
| | | | | middle | big, some points | Not accomplished goal: increase amount of leaves |
| | | | | base | 4cm, some points | |
| 2 BU05.703 | 9-12 | | | overall | >4cm, straight | Slight increase size of leaves |
| | | | | top | <2 cm, straight | Little bit moving up to top |
| | | | | middle | big, some points | |
| | | | | base | absent | |
| 2 BU04.727 | 9-12 | | | overall | >4 cm, straight | Increase in size |
| | | | | top | <2 cm, straight | |
| | | | | middle | big, some points | |
| 2 BU03.706 | | | | overall | | |
| | | | | top | | |
| | | | | middle | | |
| | | | | base | | |

Table 21 (cont).

| GREAT GRANDPARENTS | Qleaves | Distribution in bud | Distribution open flower | Size leaves open flower | |
|---|---|---|---|---|---|
| 3 EL04.704 | 13-15 | | | overall | big, some points |
| | | | | top | 4 cm, straight |
| | | | | middle | big, some points |
| | | | | base | absent |
| 3 EL04.712 | 13-16 | | | overall | >4cm, straight |
| | | | | top | 2-4 cm, straight |
| | | | | middle | >4 cm, some points |
| | | | | base | big, some points |
| 3 EL02.516 | 9-12 | | | overall | 2-4 cm, straight |
| | | | | top | <2 cm, straight |
| | | | | middle | 2-4 cm, straight |
| 3 EL03.705 | | | | overall | |
| | | | | top | |
| | | | | middle | |
| | | | | base | |
| 3 EL02.516 | 9-12 | | | overall | 2-4 cm, straight |
| | | | | top | <2 cm, straight |
| | | | | middle | 2-4 cm, straight |
| 3 EL03.712 | | | | overall | |
| | | | | top | |
| | | | | middle | |
| | | | | base | |
| 3 EL02.531 | | | | overall | |
| | | | | top | |
| | | | | middle | |
| 3 EL00.501 | 9-12 | | | overall | 2-4 cm, straight |
| | | | | top | <2 cm, straight |
| | | | | middle | >4 cm, some points |
| | | | | base | absent |

Table 21 (cont).

| GREAT-GREAT GRANDPARENTS | Øleaves | Distribution in bud | Distribution open flower | | Size leaves open flower |
|---|---|---|---|---|---|
| 4  BL03.709 | | | | overall | |
| | | | | top | |
| | | | | middle | |
| 4  BL02.510 | | | | overall | |
| | | | | top | |
| | | | | middle | |
| | | | | base | |
| 4  BL02.521 | 9-12 | | | overall | 2-4 cm, straight |
| | | | | top | <2 cm, straight |
| | | | | middle | 2-4 cm, straight |
| 4  BL00.501 | 9-12 | | | overall | 2-4 cm, straight |
| | | | | top | <2 cm, straight |
| | | | | middle | 4+ cm, some points |
| | | | | base | absent |
| 4  BL00.503 | | | | overall | |
| | | | | top | |
| | | | | middle | |
| 4  BL00.515 | | | | overall | |
| | | | | top | |
| | | | | middle | |
| | | | | base | |
| 4  BL02.531 | | | | overall | |
| | | | | top | |
| | | | | middle | |
| 4  BL00.501 | 9-12 | | | overall | 2-4 cm, straight |
| | | | | top | <2 cm, straight |
| | | | | middle | >4 cm, some points |
| | | | | base | absent |

etc

Example 4: GERFOLIA™ *Gerbera jamesonii* designated 'TERNOSTALGY'

The present example of the invention comprises a new and distinct cultivar of *Gerbera jamesonii,* referred to by the cultivar name 'TERNOSTALGY'.

'TERNOSTALGY' originated from a hybridization program in Kudelstaart, The Netherlands, in 2003. The female parent was *Gerbera jamesonii* designated 'BL03.709' (unpatented), and is characterized by its single flowering type, flat shaped inflorescense, diameter of 8 cm, yellow in color, with 9 to12 leaves on the flower stem, along the top half of the flower stem, and leaves on the flower stem are 8 to 11 cm long and green in color. The male parent was *Gerbera jamesonii* designated 'BL.02.510' (unpatented), and is characterized by its single flowering type, flat shaped inflorescense, 8 cm in diameter, orange in color, with 5 to 8 leaves on the flower stem, only on top of the flower stem, and the leaves on the flower stem are 4 to 6 cm long and green in color. The new GERFOLIA™ *Gerbera jamesonii* was selected by the inventors as a single flowering plant within the progeny of the stated cross in a controlled environment in Kudelstaart, The Netherlands, on or about 2004.

The first asexual reproduction of 'TERNOSTALGY' was accomplished by vegetative cuttings in April of 2004 in Kudelstaart, The Netherlands. The new *Gerbera jamesonii* 'TERNOSTALGY' is presently being propagated by vegetative cuttings and tissue culture. Horticultural examination of selected units initiated in 2004 has demonstrated that the combination of characteristics as herein disclosed for 'TERNOSTALGY' are firmly fixed and are retained through successive generations of asexual reproduction. The new *Gerbera jamesonii* reproduces true-to-type.

The following traits have been repeatedly observed and are determined to be unique characteristics of the GERFOLIA™ *Gerbera jamesonii* designated 'TERNOSTALGY', which in combination distinguish this *Gerbera jamesonii* as a new and distinct cultivar:

1. Single-flowering type, incurving funnel-shaped inflorescence measuring about 10 cm in diameter and having a general yellow tonality color (from a distance of 3 meters);

2. one or more leafy flower stems with about 15 to 20 full or partial leaves per flower stem which are about 50 to 150 mm in length and about 4 to 20 mm in width, and are distributed in the top half of the flower stem;

3. ability to be shipped during in bud stage of development;

4. plant height is 40 cm, spread is 70 cm;

5. strong stem, which is 70 cm in length;

6. flowering response time is 8 to 9 weeks, and

7. no special pest/disease resistance/susceptibility is present.

The new GERFOLIA™ *Gerbera jamesonii* designated 'TERNOSTALGY' has not been observed under all possible environmental conditions. The phenotype of 'TERNOSTALGY' may vary with variations in environment such as temperature, light intensity, and day length without any change in the genotype of the plant. The following observations, measurements and comparisons describe plants of 'TERNOSTALGY' grown in Kudelstaart, The Netherlands, under greenhouse conditions which closely approximate those generally used in commercial practice.

Plants of 'TERNOSTALGY' differ from plants of the *Gerbera jamesonii* designated 'BL.03.709' and "BL02.510" unpatented, in the following characteristics of Table 22:

Table 22

| Trait | New GERFOLIA™ 'TERNOSTALGY' | Female Parent 'BL.03.709' (unpatented) | Male Parent 'BL.02.510' (unpatented) |
|---|---|---|---|
| Flowering Type | Single | Single | Single |
| Inflorescence Shape | Incurving funnel shaped | Flat | Flat |
| Inflorescence Size | About 10 cm | About 8 cm | About 8 cm |
| Inflorescence Color | Yellow | Yellow | Orange |
| Number of Full of Partial Leaves Per Flower Stem | About 15 to about 20 | About 9 to about 12 | About 5 to about 8 |
| Leaf Size: | Length: 50 to 150 mm Width: 4 to 20 mm | Length: 80 to 110 mm Width: 10 to 17 mm | Length: 40 to 60 mm Width: 4 to 8 mm |
| Leaf Distribution per Flower Stem | Along the top half of the flower stem | Along the top half of the flower stem | Along the top half of the flower stem |
| Ability to be Shipped During In Bud Stage of Development | Yes | Yes | Yes |

Of the many *Gerbera L.* commercial cultivars known to the inventors, there is no cultivar similar in comparison to 'TERNOSTALGY'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

A. Flower Head:
Type: Capitulum
Flowering Type: Single
Diameter: About 9 to 10 cm
Color (general tonality from a distance of 3 meters:) Yellow
Shape: Incurving funnel shaped

(continued)

Involucre:

| | |
|---|---|
| Height from point of attachment of involucre to top of flower head: | About 30 mm |
| Height: | About 20 mm |
| Diameter: | About 30 to 35 mm |
| Number of bracts: | About 63 |
| Longitudinal axis of inner rows: | Straight |
| Anthocyanin: | Absent or very weak at tips |
| Pubescence: | Medium |

Ray florets:

| | |
|---|---|
| Number: | About 28 to 36 |
| Overall Shape: | Obovate |
| Longitudinal axis outer row: | Straight |
| Longitudinal axis inner row: | Absent, single flower |
| Longitudinal axis of ray female floret: | Staight |
| Longitudinal axis of ray male floret: | Reflexing |
| Outer ray floret: | |
| Cross section: | Flat |
| Length: | About 50 mm |
| Width: | About 9 to 12 mm |
| Longitudinal folding: | Medium |
| Angle of apex: | Acute |
| Shape of apex: | Pointed |
| Incisions of apex: | 0 to 1 |
| Depth of incision: | absent to very shallow |
| Color (top side): | Yellow/orange in color, RHS 23 A |
| Color (bottom side) | Yellow/orange in color, RHS 16 B |
| Color distribution on inner side: | Uniform |
| Edge of different color: | Absent |
| Striation: | Absent |
| Claw spot: | Absent |

B. Disc florets:

| | |
|---|---|
| Number: | 350 to 380 |
| Disc diameter: | About 32 to 38 mm |
| Color (mature, upperside): | Yellow/orange in color, RHS 23 A |
| Color (immature, top): | Red in color, RHS 53 A |
| Main color upperside corolla: | Female flowers: Yellow/Orange, RHS 23 A |
| | Male flowers: Orange, RHS 28 B |

C. Reproductive Organs:

| | |
|---|---|
| Style: | Main color distal part: Green/Yellow, RHS 1 A |
| Stigma: | Main color: Yellow, RHS 2 A |
| Anthers: | Main color: Yellow/orange, RHS 23 B |
| | Color of top relative to other parts is lighter |

(continued)

C. Reproductive Organs:

|  |  |  |
|---|---|---|
|  |  | Longitudinal stripes are absent |
|  |  | Intensity of anthocyanin coloration is absent |
|  | Pappus: | Main color: Yellow, RHS 4 B |
|  |  | Color of top relative to other parts is darker |
|  |  | Level of top relative to closed disc florets: far below |
|  | Fertility: | Fertility (male and female) as well as the seed setting is reasonable |

D. Peduncle:

|  |  |
|---|---|
| Length: | About 65 to 75 cm |
| Cross section: | Elliptic |
| Tendency to fasciation: | Absent |
| Thickness: | Medium |
| Strength: | Strong |
| Pubescence: | Dense |
| Color: | Yellow/green, RHS 144 C |
| Anthocyanin coloration: | At base: present weak, Red, RHS 48 C |
|  | At top: absent |
| Involucral bracts: | Present - Green in color, RHS 143 A |

II. PLANT:

A. General appearance:

| Height: | 40 to 45 cm (excluding any inflorescences). |
|---|---|
| Spread: | 65 to 70 cm |

B. Foliage:

| Leaf blade on stem | See Table 2 "overview description of leaves per flower stem", category 4" |
|---|---|
| Number of partial or full leaves per flower stem: | Up to 15 to 20 |
| Distribution of Leaves | Top half of the flower stem |
| Length: | 30 to 160 mm, 30 mm close to the flower head and 160 mm on the lowest leaves present on the flower stem |
| Width: | 4 mm to 23 mm, 4 mm close to the flower head and 23 mm on the lowest leaves presenton the flower stem |
| Thickness: | Thin |
| Blistering: | Medium |
| Pubescence: | Present |
| Depth of cuts or incisions in leaf: | Basal part: absent |
|  | Central part: absent or very shallow |
|  | Distal part: absent or very shallow |
| Color: | Upper side: green, RHS 137 A |
|  | Bottom side: green, RHS 138 A |
| Glossiness on upper side: | Weak |
| Angle of apex: | Very acute |

(continued)

| | |
|---|---|
| B. Foliage: | |
| Shape of apex: | Pointed |
| Margin of lobes: | Sinuate to serrate, sinuate close to the flower head, and serrate on the lowest leaves present on the flower stem. |
| Extensions of margin: | Very small to medium |
| Petiole: | |
| Length: | About 3 mm |
| Color: | Green, RHS 137 A |
| Anthocyanin coloration: | Absent |
| C. Disease/pest resistance/susceptibility: | No special disease/pest resistance/susceptibility |
| D. Ability to be Shipped During In Bud Stage Development | Yes |
| E. Leaf blade on plant | |
| Length: | 45 to 50 cm |
| Width: | 13 to 15 cm |
| Thickness: | medium |
| Blistering: | medium |
| Pubescence: | On upper side (midrib excluded): present |
| Depth of cuts or incisions in leaf: | Basal part: deep |
| | Central part: deep |
| | Distal part: medium |
| Color: | Upper side: green, RHS 137 A |
| | Bottom side: green, RHS 138 A |
| Glossiness on upper side: | Weak |
| Angle of apex: | Acute |
| Shape of apex: | Pointed |
| Margin of lobes: | Irregular |
| Extensions of margin: | Small |
| Petiole: | |
| Length: | About 6 cm |
| Color: | Green, RHS 144 B |
| Anthocyanin coloration: | Present - purple, RHS 183 B |
| Disease/pest resistance/susceptibility: | No special disease/pest resistance/susceptibility |
| Ability to be Shipped During In Bud Stage Development | Yes |

Example 5: GERFOLIA™ *Gerbera jamesonii* designated 'TERZORG'

The present example of the invention comprises a new and distinct cultivar of *Gerbera jamesonii,* referred to by the cultivar name 'TERZORG'.

'TERZORG' originated from a hybridization program in Kudelstaart, The Netherlands, in 2005. The female parent was *Gerbera jamesonii* designated 'TERFANCY' (unpatented), and is characterized by its single flowering type, incurving funnel shaped, diameter of 8 cm, red-purple in color, 15 to 18 leaves, along the top half of the flower stem, leaves on the flower stem are 30 to 110 mm long and green in color. The male parent was *Gerbera jamesonii* designated 'BL.05.701' (unpatented) and is characterized by its single flowering type, funnel shaped inflorescence, leaves on the flower stem are 10 cm long and yellow in color, 12 to 16 leaves, only on the top half of the flower stem, 40 to 150 mm long, green in color. The new GERFOLIA™ *Gerbera jamesonii* 'TERZORG' was selected by the inventors as a single flowering plant within the progeny of the stated cross in a controlled environment in Kudelstaart, The Netherlands, on or about 2006.

The first asexual reproduction of 'TERZORG' was accomplished by vegetative cuttings in April of 2006 in

**EP 2 011 388 A1**

Kudelstaart, The Netherlands. The new *Gerbera jamesonii* 'TERZORG' is presently being propagated by vegetative cuttings and tissue culture. Horticultural examination of selected units initiated in 2006 has demonstrated that the combination of characteristics as herein disclosed for 'TERZORG' are firmly fixed and are retained through successive generations of asexual reproduction. The new GERFOLIA™ *Gerbera jamesonii* 'TERZORG' reproduces true-to-type.

The following traits have been repeatedly observed and are determined to be unique characteristics of the GERFOLIA™ *Gerbera jamesonii* designated 'TERZORG', which in combination distinguish this *Gerbera jamesonii* as a new and distinct cultivar:

1. Single-flowering type, funnel-shaped inflorescence measuring about 8 cm in diameter and having a general pink tonality color (from a distance of 3 meters);
2. one or more leafy flower stems with about 15 to 18 full or partial leaves per flower stem which are about 30 to 130 mm in length and about 4 to 20 mm in width, and are distributed in the top half of the flower stem;
3. ability to be shipped during in bud stage of development;
4. plant height is 30 to 35 cm, spread is 40 to 50 cm;
5. strong stem which measures about 55 to 65 cm in length;
6. flowering response time is 8 to 9 weeks;
7. no special pest/disease resistance/susceptibility is present.

The new GERFOLIA™ *Gerbera jamesonii* designated 'TERZORG' has not been observed under all possible environmental conditions. The phenotype of 'TERZORG' may vary with variations in environment such as temperature, light intensity, and day length without any change in the genotype of the plant. The following observations, measurements and comparisons describe plants of 'TERZORG' grown in Kudelstaart, The Netherlands, under greenhouse conditions which closely approximate those generally used in commercial practice.

Plants of 'TERZORG' differ from plants of the *Gerbera jamesonii* designated 'TERFANCY' (unpatented) and 'BL.05.701' (unpatented), in the following characteristics of Table 23:

Table 23

| Trait | New GERFOLIA™ 'TERZORG' | 'TERFANCY' (unpatented) | 'BL.05.701' (unpatented) |
|---|---|---|---|
| Flowering Type | Single | Single | Single |
| Inflorescence Shape | Incurving funnel shaped | Incurving funnel shaped | Incurving funnel shaped |
| Inflorescence Size | About 8 cm | About 8 cm | About 10 cm |
| Inflorescence Color | Pink | Red/purple | Yellow |
| Number of Full of Partial Leaves Per Flower Stem | About 15 to about 18 | About 15 to about 18 | About 12 to about 16 |
| Leaf Size: | Length: 30 to 130 mm Width: 4 to 20 mm | Length: 30 to 110 mm Width: 2 to 18 mm | Length: 40 to 150 mm Width: 4 to 24 mm |
| Leaf Distribution per Flower Stem | Along the top half of the flower stem | Along the top half of the flower stem | Along the top half of the flower stem |
| Ability to be Shipped During In Bud Stage of Development | Yes | Yes | Yes |

Of the many *Gerbera* L. commercial cultivars known to the inventors, there is no cultivar similar in comparison to 'TERZORG'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

A. Flower Head:

| | |
|---|---|
| Type: | Capitulum |
| Flowering Type: | Single |
| Diameter: | About 7 to 9 cm |
| Color (general tonality from a distance of 3 meters:) | Pink |
| Shape: | Incurving funnel shaped |
| Involucre: | |
| Height from point of attachment of involucre to top of flower head: | About 45 mm |
| Height: | About 25 mm |
| Diameter: | About 25 to 30 mm |
| Number of bracts: | About 50 |
| Longitudinal axis of inner rows: | Straight |
| Anthocyanin: | Absent or very weak at tips |
| Pubescence: | Medium |

Ray florets:

| | |
|---|---|
| Number: | About 40 to 45 |
| Overall Shape: | Obovate |
| Longitudinal axis outer row: | Incurving |
| Longitudinal axis inner row: | Absent, single flower |
| Longitudinal axis of ray female floret: | Staight |
| Longitudinal axis of ray male floret: | Staight |
| Outer ray floret: | |
| Cross section: | Flat |
| Length: | About 40 mm |
| Width: | About 8 to 9 mm |
| Longitudinal folding: | Medium |
| Angle of apex: | Right angle |
| Shape of apex: | Pointed |
| Incisions of apex: | 0 to 1 |
| Depth of incision: | absent to very shallow |
| Color (top side): | Red/pink in color, RHS 36 B |
| Color (bottom side) | Red/pink in color, RHS 36 B |
| Color distribution on inner side: | Uniform |
| Edge of different color: | Absent |
| Striation: | Absent |
| Claw spot: | Very shallow, 15%, RHS 36 D |

B. Disc florets:

| | |
|---|---|
| Number: | 250 to 300 |
| Disc diameter: | About 20 to 30 mm |
| Color (mature, upperside): | Yellow/green in color, RHS 151 A |
| Color (immature, top): | Yellow/green in color, RHS 154 B |
| Main color upperside corolla: | Female flowers: Red, RHS 36 B |
| | Male flowers: Red, RHS 36 B |

C. Reproductive Organs:

| | |
|---|---|
| Style: | Main color distal part: Red, RHS 36 D |

(continued)

| | |
|---|---|
| Stigma: | Main color: Red, RHS 36 D |
| Anthers: | Main color: Yellow/orange, RHS 17 B |
| | Color of top relative to other parts is lighter |
| | Longitudinal stripes are absent |
| | Intensity of anthocyanin coloration is absent |
| Pappus: | Main color: Yellow, RHS 4 D |
| | Color of top relative to other parts is identical |
| | Level of top relative to closed disc florets: far below |
| Fertility: | Fertility (male and female) as well as the seed setting is reasonable |

D. Peduncle:

| | |
|---|---|
| Length: | About 55 to 65 cm |
| Cross section: | Elliptic |
| Tendency to fasciation: | Present |
| Thickness: | Medium |
| Strength: | Strong |
| Pubescence: | Medium |
| Color: | Yellow/green, RHS 146 D |
| Anthocyanin coloration: | At base: present - grey/purple RHS 186 A |
| | At top: absent |
| Involucral bracts: | Present - green, RHS 137 A |

II. PLANT:

A. General appearance:

| | |
|---|---|
| Height: | 30 to 35 cm (excluding any inflorescences). |
| Spread: | 40 to 50 cm |

B. Foliage:

| | |
|---|---|
| Leaf blade on stem | |
| Number of partial or full leaves per flower stem: | to 15 to 18 |
| Distribution of Leaves | Top half of the flower stem |
| Length: | 30 to 130 mm, 30 mm close to the flower head and 130 mm on the lowest leaves presenton the flower stem |
| Width: | 4 mm to 20 mm, 4 mm close to the flower head and 20 mm on the lowest leaves presenton the flower stem |
| Thickness: | Medium |
| Blistering: | Medium |
| Pubescence: | Present |
| Depth of cuts or incisions in leaf | Basal part: absent |
| | Central part: shallow |
| | Distal part: shallow |
| Color: | Upper side: green, RHS 137 A |
| | Bottom side: green, RHS 138 A |
| Glossiness on upper side: | Medium |
| Angle of apex: | Very acute |

(continued)

| | |
|---|---|
| Shape of apex: | Pointed |
| Margin of lobes: | Serrate |
| Extensions of margin: | At the top absent, lowest leaves on the flower stem small |
| Petiole: | |
| Length: | About 1 to2 mm |
| Color: | Green, RHS 139 D |
| Anthocyanin coloration: | Absent |

| | |
|---|---|
| C. Disease/pest resistance/susceptibility: | No special disease/pest resistance/susceptibility |
| D. Ability to be Shipped During In Bud Stage Development | Yes |
| E. Leaf blade on plant | |

| | |
|---|---|
| Length: | 290 to 320 mm |
| Width: | 100 to 140 mm |
| Thickness: | medium |
| Blistering: | medium |
| Pubescence: | On upper side (midrib excluded): present |
| Depth of cuts or incisions in leaf: | Basal part: deep |
| | Central part: deep |
| | Distal part: medium |
| Color: | Upper side: green, RHS 137 A |
| | Bottom side: green, RHS 137 A |
| Glossiness on upper side: | Medium |
| Angle of apex: | Right angle |
| Shape of apex: | Pointed |
| Margin of lobes: | Irregular |
| Extensions of margin: | Medium |
| Petiole: | |
| Length: | About 100 to 120 mm |
| Color: | Green, RHS 139 D |
| Anthocyanin coloration: | Present - Greyed/purple, RHS 187 B |

| | |
|---|---|
| Disease/pest resistance/susceptibility: | no special disease/pest resistance/susceptibility |
| Ability to be Shipped During In Bud Stage Development | Yes |

Example 6: GERFOLIA™ *Gerbera jamesonii* designated 'BL02.528'.

The present example of the invention comprises a new and distinct cultivar of *Gerbera jamesonii,* referred to by the cultivar name 'BL02.528'.
GERFOLIA™ *Gerbera jamesonii* 'BL02.528' originated from a hybridization program in Kudelstaart, The Netherlands, in 2001. The female parent was *Gerbera jamesonii* designated 'BL00.516' (unpatented), and is characterized by its semi-double flowering type, flat shaped inflorescense, diameter of 7 cm, pink in color, with 6 to 8 leaves on the flower stem, along the top half of the flower stem, leaves on the flower stem are 20 to 60 mm long and green in color. The male parent was *Gerbera jamesonii* designated 'BL00.002', unpatented, and is
characterized by its semi-double flowering type, flat shaped inflorescense, diameter of 12 cm, red/purple in color, 3 to 5 leaves on the flower stem, only on the top quarter of the flower stem, leaves on the flower stem are 20 to 40 mm long and green in color. The new GERFOLIA™ *Gerbera jamesonii* 'BL02.528' was selected by the inventors as a single flowering plant within the progeny of the stated cross in a controlled environment in Kudelstaart, The Netherlands, on or about 2002.
The first asexual reproduction of 'BL02.528' was accomplished by vegetative cuttings in April of 2002 in Kudelstaart, The Netherlands. The new GERFOLIA™ *Gerbera jamesonii* 'BL02.528' is presently being propagated by vegetative cuttings. Horticultural examination of selected units initiated in 2002 has demonstrated that the combination of characteristics as herein disclosed for 'BL02.528' are firmly fixed and are retained through successive generations of asexual reproduction. The new *Gerbera* reproduces true-to-type.

The following traits have been repeatedly observed and are determined to be unique characteristics of the GERFOLIA™ *Gerbera jamesonii* designated 'BL02.528', which in combination distinguish this *Gerbera jamesonii* as a new and distinct cultivar:

1. Multi-petalled flowering type, globular-shaped inflorescence, measuring about 10 cm in diameter and having a general pink tonality color (from a distance of 3 meters);
2. one or more leafy flower stems with about 10 to 13 full or partial leaves per flower stem which are about 20 to 20 mm in length and about 2 to 20 mm in width, and are distributed in the top half of the flower stem;
3. ability to be shipped during in bud stage of development;
4. plant height is 40 to 45 cm, spread is 65 to 75 cm;
5. strong stem which measures in length about 65 to 70 cm, strong stem;
6. flowering response time is 8 to 9 weeks;
7. and no special pest/disease resistance/susceptibility is present.

The new GERFOLIA™ *Gerbera jamesonii* designated 'BL02.528' has not been observed under all possible environmental conditions. The phenotype of 'BL02.528' may vary with variations in environment such as temperature, light intensity, and day length without any change in the genotype of the plant. The following observations, measurements and comparisons describe plants of 'BL02.528' grown in Kudelstaart, The Netherlands, under greenhouse conditions which closely approximate those generally used in commercial practice.

Plants of GERFOLIA™ *Gerbera jamesonii* 'BL02.528' differ from plants of the *Gerbera jamesonii* designated 'BL00.516' (unpatented) and 'BL.00.002' (unpatented), in the following characteristics of Table 24:

Table 24

| Trait | New GERFOLIA™ 'BL02.528' | 'BL00.516' (unpatented) | 'BL.00.002' (unpatented) |
|---|---|---|---|
| Flowering Type | Multi-Petalled | Semi-double | Semi-double |
| Inflorescence Shape | Globular | Incurving flat | Incurving flat |
| Inflorescence Size | About 10 cm | About 7 cm | About 12 cm |
| Inflorescence Color | Pink | Pink | Red/purple |
| Number of Full of Partial Leaves Per Flower Stem | About 10 to about 13 | About 6 to about 8 | About 10 to about 12 |
| Leaf Size: | Length: 20 to 120 mm Width: 2 to 20 mm | Length: 20 to 60 mm Width: 5 to 9 mm | Length: 20 to 40 mm Width: 4 to 8 mm |
| Leaf Distribution per Flower Stem | Along the top half of the flower stem | Along the top half of the flower stem | Along the top quarter of the flower stem |
| Ability to be Shipped During In Bud Stage of Development | Yes | Yes | Yes |

Of the many *Gerbera L.* commercial cultivars known to the inventors, there is no cultivar similar in comparison to 'BL02.528'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

A.      Flower Head:

| | |
|---|---|
| Type: | Capitulum |
| Flowering Type: | Multi-petalled |
| Diameter: | About 9 to 10 cm |
| Color (general tonality from a distance of 3 meters:) | Pink |
| Shape: | Globular |
| Involucre: | |
| Height from point of attachment of involucre to top of flower head: | About 35 mm |
| Height: | About 18 mm |
| Diameter: | About 38 to 42 mm |
| Number of bracts: | About 70 |
| Longitudinal axis of inner rows: | Incurving |
| Anthocyanin: | Absent or very weak at tips |
| Pubescence: | Medium |
| Ray florets: | |
| Number: | About 370 to 400 |
| Overall Shape: | Narrow obovate |
| Longitudinal axis outer row: | Incurving |
| Longitudinal axis inner row: | Reflexing |
| Longitudinal axis of ray female floret: | Staight |
| Longitudinal axis of ray male floret: | Staight |
| Outer ray floret: | |
| Cross section: | Concave |
| Length: | About 50 mm |
| Width: | About 7 to 8 mm |
| Longitudinal folding: | Strong |
| Angle of apex: | Right angle |
| Shape of apex: | Pointed |
| Incisions of apex: | 0 to 1 |
| Depth of incision: | absent to very shallow |
| Color (top side): | Red/purple in color, RHS 58 D amd 4 D at the top |
| Color (bottom side) | Yellow/green in color, RHS 154 C and 4 D and 58 D. |
| Color distribution on inner side: | Lighter to top |
| Edge of different color: | Absent |
| Striation: | Absent |
| Claw spot: | Present, 5%, RHS 4 D |

B. Disc florets:

| | |
|---|---|
| Number: | In a multi-petalled type flower e.g. the GERRONDO™, disc florets are replaced by ray florets. |
| Disc diameter: | About 35 to 40 mm |
| Color (mature, upperside): | Red/purple in color, RHS 58 D and 4 D |
| Color (immature, top): | Yellow/green in color, RHS 154 A |
| Main color upperside corolla: | Female flowers: Red/purple, RHS 58 B |
| | Male flowers: Red/purple, RHS 58 B |

(continued)

C. Reproductive Organs:

| | |
|---|---|
| Style: | Main color distal part: Yellow, RHS 11 D |
| Stigma: | Main color: Yellow, RHS 11 C |
| Anthers: | Main color: Yellow/orange, RHS 17 A |
| | Color of top relative to other parts is lighter |
| | Longitudinal stripes are absent |
| | Intensity of anthocyanin coloration is present |
| Pappus: | Main color: Yellow, RHS 4 D. |
| | Color of top relative to other parts is identical |
| | Level of top relative to closed disc florets: far below |
| Fertility: | Fertility (male and female) as well as the seed setting is very poor |

D.     Peduncle:

| | |
|---|---|
| Length: | About 65 to 70 cm |
| Cross section: | Elliptic |
| Tendency to fasciation: | Present |
| Thickness: | Medium |
| Strength: | Strong |
| Pubescence: | Medium |
| Color: | Yellow/green, RHS 144 A |
| Anthocyanin coloration: | At base: present - grey/orange RHS 176 B |
| | At top: absent |
| Involucral bracts: | Present - yellow, RHS 147 B |

II. PLANT:

A.     General appearance:

| | |
|---|---|
| Height: | 40 to 45 cm (excluding any inflorescences). |
| Spread: | 65 to 75 cm |

B.     Foliage:

| | |
|---|---|
| Leaf blade on stem | See Table 2 "overview description of leaves per flower stem", category 4" |
| Number of partial or full leaves per flower stem: | Up to 10 to 13 |
| Distribution of Leaves | Top half of the flower stem |
| Length: | 20 to 120 mm, 20 mm close to the flower head and 120 mm on the lowest leaves present on the flower stem |
| Width: | 2 mm to 20 mm, 2 mm close to the flower head and 20 mm on the lowest leaves present on the flower stem |
| Thickness: | Medium |
| Blistering: | Medium |
| Pubescence: | Present |
| Depth of cuts or incisions in leaf: | Basal part: absent to shallow |
| | Central part: absent to shallow |
| | Distal part: absent to shallow |
| Color: | Upper side: green, RHS 137 A |

(continued)

| | | |
|---|---|---|
| | | Bottom side: green, RHS 137 C |
| | Glossiness on upper side: | Medium |
| | Angle of apex: | Very acute |
| | Shape of apex: | Pointed |
| | Margin of lobes: | Serrate |
| | Extensions of margin: | Small |
| | Petiole: | |
| | Length: | About 1 to 2 mm |
| | Color: | Green/yellow, RHS 144 A |
| | Anthocyanin coloration: | Absent |
| C. Disease/pest resistance/susceptibility: | | No special disease/pest resistance/susceptibility |
| D. Ability to be Shipped During In Bud Stage Development | | Yes |
| E. Leaf blade on plant | | |
| | Length: | 50 +/- 4 mm |
| | Width: | 20 +/- 3 mm |
| | Thickness: | Medium |
| | Blistering: | Medium |
| | Pubescence: | On upper side (midrib excluded): present |
| | Depth of cuts or incisions in leaf: | Basal part: deep |
| | | Central part: deep |
| | | Distal part: medium |
| | Color: | Upper side: yellow/green, RHS 147 A |
| | | Bottom side: green, RHS 137 C |
| | Glossiness on upper side: | Medium |
| | Angle of apex: | Right angle |
| | Shape of apex: | Pointed |
| | Margin of lobes: | Serrate |
| | Extensions of margin: | Small |
| Petiole: | | |
| Length: | | About 20+/-2 mm |
| Color: | | Green/yellow, RHS 144 B |
| Anthocyanin coloration: | | Present - Greyed/purple, RHS 184 C |
| Disease/pest resistance/susceptibility: | | no special disease/pest resistance/susceptibility |
| Ability to be Shipped During In Bud Stage Development | | Yes |

Example 7: GERFOLIA™ *Gerbera jamesonii* designated 'TERTRUE'

The present example of the invention comprises a new and distinct cultivar of *Gerbera jamesonii,* referred to by the cultivar name 'TERTRUE'.

GERFOLIA™ *Gerbera jamesonii* 'TERTRUE' originated from a hybridization program in Kudelstaart, The Netherlands, in 2005. The female parent was *Gerbera jamesonii* 'TERNOSTALGY' (unpatented), and is characterized by its single flowering type, funnel shaped inflorescense, diameter of 10 cm, yellow in coulour, with 15 to 20 leaves on the flower stem, along the top half of the flower stem, leaves on the flower stem are 50 to 150 mm long and green in color. The male parent was *Gerbera jamesonii* designated 'BL05.707' unpatented, and is characterized by its single type flowering, funnel shaped inflorescense, diameter of 10 cm, red in color, with 8 to 10 leaves on the flower stem, only on the top half of the flower stem, leaves on the flower stem are 25 to 90 mm long and green in color. The new GERFOLIA™ *Gerbera jamesonii* 'TERTRUE' was selected by the inventors as a single flowering plant within the progeny of the stated cross in a controlled environment in Kudelstaart, The Netherlands, on or about 2006.

The first asexual reproduction of 'TERTRUE' was accomplished by vegetative cuttings in April of 2006 in Kudelstaart, The Netherlands. The new *Gerbera* 'TERTRUE' is presently being propagated by vegetative cuttings. Horticultural examination of selected units initiated in 2006 has demonstrated that the combination of characteristics as herein disclosed for 'TERTRUE' are firmly fixed and are retained through successive generations of asexual reproduction. The new *Gerbera* reproduces true-to-type.

The following traits have been repeatedly observed and are determined to be unique characteristics of the GERFOLIA™ *Gerbera jamesonii* designated 'TERTRUE', which in combination distinguish this *Gerbera jamesonii* as a new and distinct cultivar:

1. Single flowering type, funnel shaped inflorescence, measuring about 10 cm in diameter and having a general orange tonality color (from a distance of 3 meters);
2. one or more leafy flower stems with about 10 to 14 full or partial leaves per flower stem which are about 40 to 140 mm in length and about 3 to 35 mm in width, and are distributed in the top half of the flower stem;
3. ability to be shipped during in bud stage of development;
4. plant height is 30 to 35 cm, spread is 60 to 70 cm;
5. strong stem which measures in length about 55 to 60 cm, strong stem;
6. flowering response time is 8 to 9 weeks; and
7. no special pest/disease resistance/susceptibility is present.

The new GERFOLIA™ *Gerbera jamesonii* designated 'TERTRUE' has not been observed under all possible environmental conditions. The phenotype of 'TERTRUE' may vary with variations in environment such as temperature, light intensity, and day length without any change in the genotype of the plant. The following observations, measurements and comparisons describe plants of 'TERTRUE' grown in Kudelstaart, The Netherlands, under greenhouse conditions which closely approximate those generally used in commercial practice.

Plants of GERFOLIA™ *Gerbera jamesonii* 'TERTRUE' differ from plants of the *Gerbera jamesonii* designated 'TERNOSTALGY' (unpatented) and 'BL05.707' (unpatented), in the following characteristics of Table 25:

Table 25

| Trait | New GERFOLIA™ 'TERTRUE' | 'TERNOSTALGY' (unpatented) | 'BL05.707' (unpatented) |
|---|---|---|---|
| Flowering Type | Single | Single | Single |
| Inflorescence Shape | Funnel shaped | Incurving funnel shaped | Funnel shaped |
| Inflorescence Size | About 10 cm | About 10 cm | About 10 cm |
| Inflorescence Color | Orange | Yellow | Red |
| Number of Full of Partial Leaves Per Flower Stem | About 10 to about 14 | About 15 to about 20 | About 8 to about 10 |
| Leaf Size: | Length: 40 to 140 mm Width: 3 to 35 mm | Length: 50 to 150 mm Width: 4 to 20 mm | Length: 25 to 90 mm Width: 2 to 10 mm |
| Leaf Distribution per Flower Stem | Equally along the flower stem | Along the top half of the flower stem | Along the top quarter of the flower stem |
| Ability to be Shipped During In Bud Stage of Development | Yes | Yes | Yes |

Of the many *Gerbera L.* commercial cultivars known to the inventors, there is no cultivar similar in comparison to 'TERTRUE'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

A. Flower Head:

| | | |
|---|---|---|
| Type: | | Capitulum |
| Flowering Type: | | Single |
| Diameter: | | About 9 to 11 cm |
| Color (general tonality from a distance of 3 meters:) | | Orange |
| Shape: | | Funnel shaped |
| Involucre: | | |
| | Height from point of attachment of involucre to top of flower head: | About 40 mm |
| | Height: | About 25 mm |
| | Diameter: | About 30 to 35 mm |
| | Number of bracts: | About 45 |
| | Longitudinal axis of inner rows: | Staight |
| | Anthocyanin: | Absent or very weak at tips |
| | Pubescence: | Medium |
| Ray florets: | | |
| | Number: | About 50 to 60 |
| | Overall Shape: | Obovate |
| | Longitudinal axis outer row: | Staight |
| | Longitudinal axis inner row: | Absent, single flowe |
| | Longitudinal axis of ray female floret: | Reflexing |
| | Longitudinal axis of ray male floret: | Reflexing |
| | Outer ray floret: | |
| | Cross section: | Flat |
| | Length: | About 55 mm |
| | Width: | About 9 to 11 mm |
| | Longitudinal folding: | Medium |
| | Angle of apex: | Right angle |
| | Shape of apex: | Pointed |
| | Incisions of apex: | 0 |
| | Depth of incision: | absent |
| | Color (top side): | Orange/red in color, RHS 33 A |
| | Color (bottom side) | Orange/red in color, RHS 32 C |
| | Color distribution on inner side: | Uniform |
| | Edge of different color: | Absent |
| | Striation: | Absent |
| | Claw spot: | Absent |
| B. Disc florets: | | |
| | Number: | 350 to 380 |
| | Disc diameter: | About 35 to 40 mm |
| | Color (mature, upperside): | Greyed/purple in color, RHS 185 A |
| | Color (immature, top): | Greyed/purple in color, RHS 187 A |
| | Main color upperside corolla: | Female flowers: Orange/red, RHS 32 A |
| | | Male flowers: Orange/red, RHS 43 A |

C. Reproductive Organs:

(continued)

| | | |
|---|---|---|
| | Style: | Main color distal part: Yellow, RHS 3 A |
| | Stigma: | Main color: Yellow/green, RHS 1 A |
| | Anthers: | Main color: Yellow/orange, RHS 15 A |
| | | Color of top relative to other parts is lighter |
| | | Longitudinal stripes are absent |
| | | Intensity of anthocyanin coloration is absent |
| | Pappus: | Main color: red, RHS 53 A |
| | | Color of top relative to other parts is darker |
| | | Level of top relative to closed disc florets: far below |
| | Fertility: | Fertility (male and female) as well as the seed setting is reasonable |
| D. Peduncle: | | |
| | Length: | About 55 to 60 cm |
| | Cross section: | Elliptic |
| | Tendency to fasciation: | Absent |
| | Thickness: | Thick |
| | Strength: | Strong |
| | Pubescence: | Dense |
| | Color: | Greyed/red, RHS 182 B |
| | Anthocyanin coloration: | At base: present - grey/orange RHS 176 B |
| | | At top: absent |
| | Involucral bracts: | Present - green, RHS 143 A |

II. PLANT:

| | | | |
|---|---|---|---|
| A. | | General appearance: | |
| | | Height: | 30 to 35 cm (excluding any inflorescences). |
| | | Spread: | 60 to 70 cm |
| B. | | Foliage: | |
| | | Leaf blade on stem | See Table 2 "overview description of leaves per flower stem", category 4/5" |
| | | Number of partial or full leaves per flower stem: | 10 to 14 |
| | | Distribution of Leaves | Equally along the flower |
| | | Length: | 40 to 140 mm, 40 mm close to the flower head and 140 mm on the lowest leaves presenton the flower stem |
| | | Width: | 3 mm to 35 mm, 3 mm close to the flower head and 35 mm on the lowest leaves presenton the flower stem |
| | | Thickness: | Medium |
| | | Blistering: | Medium |
| | | Pubescence: | Present |
| | | Depth of cuts or incisions in leaf: | Basal part: absent |
| | | | Central part: shallow to medium |
| | | | Distal part: shallow |
| | | Color: | Upper side: green, RHS 137 A |
| | | | Bottom side: green, RHS 137 C |

(continued)

| | | |
|---|---|---|
| | Glossiness on upper side: | Medium |
| | Angle of apex: | Very acute |
| | Shape of apex: | Pointed |
| | Margin of lobes: | Serrate |
| | Extensions of margin: | Absent to small |
| | Petiole: | |
| | Length: | About 1 to 2 mm |
| | Color: | Green, RHS 143 C |
| | Anthocyanin coloration: | Absent |
| C. Disease/pest resistance/susceptibility: | | No special disease/pest resistance/susceptibility |
| D. Ability to be Shipped During In Bud Stage Development | | Yes |
| E. Leaf blade on plant | | |
| | Length: | 40 to 50 mm |
| | Width: | 11 to 16 mm |
| | Thickness: | Medium |
| | Blistering: | Medium |
| | Pubescence: | On upper side (midrib excluded): present |
| | Depth of cuts or incisions in leaf | Basal part: deep |
| | | Central part: deep |
| | | Distal part: medium |
| | Color: | Upper side: Green, RHS 139 A |
| | | Bottom side: Green, RHS 137 B |
| | Glossiness on upper side: | Medium |
| | Angle of apex: | Right angle |
| | Shape of apex: | Pointed |
| | Margin of lobes: | Dentate |
| | Extensions of margin: | Small to medium |
| | Petiole: | |
| | Length: | About 12 to 15 mm |
| | Color: | Green/yellow, RHS 144 B |
| | Anthocyanin coloration: | Present - Greyed/red, RHS 182 B |
| Disease/pest resistance/susceptibility: | | no special disease/pest resistance/susceptibility |
| Ability to be Shipped During In Bud Stage Development | | Yes |

Example 8: GERFOLIA™ *Gerbera jamesonii* designated 'BL07.777'

The present example of the invention comprises a new and distinct cultivar of *Gerbera jamesonii,* referred to by the cultivar name 'BL07.777'.

GERFOLIA™ *Gerbera jamesonii* 'BL07.777' originated from a hybridization program in Kudelstaart, The Netherlands, in 2006. The female parent was *Gerbera jamesonii* designated 'TERZORG' (unpatented), and is characterized by its single flowering type, incurving is funnel shaped, diameter of 8 cm, pink in color, with 15 to 18 leaveson the flower stem, along the top half of the flower stem, leaves on the flower stem are 30 to 110 mm long and green in color. The male parent was *Gerbera jamesonii* designated 'BL05.717' unpatented, and is characterized by its semi-double flowering type, flat shaped inflorescense, 7 cm in diameter, red in color, with 12 to 15 leaves on the flower stem, equally distributed on the flower stem, the leaves on the flower stem are 20 to 90 mm long and green in color. The new GERFOLIA™ *Gerbera jamesonii* 'BL07.777' was selected by the inventors as a single flowering plant within the progeny of the stated cross in a controlled environment in Kudelstaart, The Netherlands, on or about 2007.

The first asexual reproduction of 'BL07.777' was accomplished by vegetative cuttings in April of 2007 in Kudelstaart, The Netherlands. The new *Gerbera* 'BL07.777' is presently being propagated by vegetative cuttings. Horticultural examination of selected units initiated in 2007 has demonstrated that the combination of characteristics as herein disclosed for 'BL07.777' are firmly fixed and are retained through successive generations of asexual reproduction. The new *Gerbera* reproduces true-to-type.

The following traits have been repeatedly observed and are determined to be unique characteristics of the GERFOLIA™ *Gerbera jamesonii* designated 'BL07.777', which in combination distinguish this *Gerbera jamesonii* as a new and distinct cultivar:

1. Single flowering type, incurving funnel shaped, inflorescence measuring about 9 cm in diameter and having a general orange tonality color (from a distance of 3 meters);
2. one or more leafy flower stems with about 13 to 16 full or partial leaves per flower stem which are about 40 to 110 mm in length and about 9 to 18 mm in width, and are distributed in the top half of the flower stem;
3. ability to be shipped during in bud stage of development;
4. plant height is 35 to 40 cm, spread is 50 to 55 cm;
5. strong stem which measures in length about 50 to 55 cm;
6. flowering response time is 8 to 9 weeks; and
7. no special pest/disease resistance/susceptibility is present.

The new GERFOLIA™ *Gerbera jamesonii* designated 'BL07.777' has not been observed under all possible environmental conditions. The phenotype of 'BL07.777' may vary with variations in environment such as temperature, light intensity, and day length without any change in the genotype of the plant. The following observations, measurements and comparisons describe plants of 'BL07.777' grown in Kudelstaart, The Netherlands, under greenhouse conditions which closely approximate those generally used in commercial practice.

Plants of 'GERFOLIA™ *Gerbera jamesonii* BL07.777' differ from plants of the *Gerbera jamesonii* designated 'TERZORG' (unpatented) and 'BL05.717' (unpatented), in the following characteristics of Table 26:

Table 26

| Trait | New GERFOLIA™ 'BL07.777' | 'Terzorg' (unpatented) | 'BL05.717' (unpatented) |
|---|---|---|---|
| Flowering Type | Single | Single | Semi-double |
| Inflorescence Shape | Incurving funnel shaped | Incurving funnel shaped | Funnel shaped |
| Inflorescence Size | About 9 cm | About 8 cm | About 7 cm |
| Inflorescence Color | Orange | Pink | Red |
| Number of Full of Partial Leaves Per Flower Stem | About 13 to about 16 | About 15 to about 18 | About 12 to about 15 |
| Leaf Size: | Length: 40 to 110 mm Width: 9 to 18 mm | Length: 30 to 130 mm Width: 4 to 20 mm | Length: 20 to 90 mm Width: 2 to 10 mm |
| Leaf Distribution per Flower Stem | Along the top half of the flower stem | Along the top half of the flower stem | Equally along the flower stem |
| Ability to be Shipped During In Bud Stage of Development | Yes | Yes | Yes |

Of the many *Gerbera L.* commercial cultivars known to the inventors, there is no cultivar similar in comparison to 'BL07.777'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

A.    Flower Head:

| | |
|---|---|
| Type: | Capitulum |
| Flowering Type: | Single |
| Diameter: | About 8 to 9 cm |
| Color (general tonality from a distance of 3 meters:) | Orange |
| Shape: | Funnel shaped |
| Involucre: | |
|     Height from point of attachment of involucre to top of flower head: | About 40 mm |
|     Height: | About 20 mm |
|     Diameter: | About 28 to 32 mm |
|     Number of bracts: | About 35 |
|     Longitudinal axis of inner rows: | Incurving |
|     Anthocyanin: | Absent or very weak at tips |
|     Pubescence: | Medium |
| Ray florets: | |
|     Number: | About 45 to 50 |
|     Overall Shape: | Obovate |
|     Longitudinal axis outer row: | Incurving |
|     Longitudinal axis inner row: | Absent, single flowe |
|     Longitudinal axis of ray female floret: | Incurving |
|     Longitudinal axis of ray male floret: | Staight |
|     Outer ray floret: | |
|       Cross section: | Concave |
|       Length: | About 50 mm |
|       Width: | About 7 to 10 mm |
|       Longitudinal folding: | Medium to strong |
|       Angle of apex: | Acute |
|       Shape of apex: | Pointed |
|       Incisions of apex: | 0 to 1 |
|       Depth of incision: | Absent to very shallow |
|       Color (top side): | Orange/red in color, RHS 32 A |
|       Color (bottom side) | Yellow/red in color, RHS 22 B |
|       Color distribution on inner side: | Uniform |
|       Edge of different color: | Absent |
|       Striation: | Absent |
|       Claw spot: | Absent |

B.    Disc florets:

| | |
|---|---|
| Number: | 300 to 320 |
| Disc diameter: | About 23 to 25 mm |
| Color (mature, upperside): | Greyed/purple in color, RHS 185 A |
| Color (immature, top): | Greyed/purple in color, RHS 187 A |
| Main color upperside corolla: | Female flowers: Orange/red, RHS 34 A |
| | Male flowers: Orange/red, RHS 34 A |

C.    Reproductive Organs:

| | |
|---|---|
| Style: | Main color distal part: Yellow, RHS 23 B |
| Stigma: | Main color: Yellow, RHS 23 B |

(continued)

| | |
|---|---|
| Anthers: | Main color: Yellow, RHS 21 C |
| | Color of top relative to other parts is lighter |
| | Longitudinal stripes are absent |
| | Intensity of anthocyanin coloration is absent |
| Pappus: | Main color: yellow, RHS 23 D |
| | Color of top relative to other parts is identical |
| | Level of top relative to closed disc florets: far below |
| Fertility: | Fertility (male and female) as well as the seed setting is reasonable |
| D. Peduncle: | |
| Length: | About 50 to 55 cm |
| Cross section: | Elliptic |
| Tendency to fasciation: | Present |
| Thickness: | Medium |
| Strength: | Strong |
| Pubescence: | Dense |
| Color: | Yellow/Green, RHS 146 B |
| Anthocyanin coloration: | At base: present - greyed/red RHS 182 B |
| | At top: absent |
| Involucral bracts: | Present - green, RHS 143 A |

II. PLANT:

| | | |
|---|---|---|
| A. | General appearance: | |
| | Height: | 35 to 40 cm (excluding any inflorescences). |
| | Spread: | 50 to 55 cm |
| B. | Foliage: | |
| | Leaf blade on stem | See Table 2 "overview description of leaves per flower stem", category 4" |
| | Number of partial or full leaves per flower stem: | to 13 to 16 |
| | Distribution of Leaves | On the top half of the flower stem |
| | Length: | 40 to 110 mm |
| | Width: | 9 mm to 18 mm |
| | Thickness: | Medium |
| | Blistering: | Medium |
| | Pubescence: | Present |
| | Depth of cuts or incisions in leaf: | Basal part: absent |
| | | Central part: shallow |
| | | Distal part: shallow |
| | Color: | Upper side: green RHS 147 A |
| | | Bottom side: green RHS 137 B |
| | Glossiness on upper side: | Medium |
| | Angle of apex: | Acute |
| | Shape of apex: | Pointed |
| | Margin of lobes: | Serrate |
| | Extensions of margin: | Small |

(continued)

|  |  |  |
|---|---|---|
|  | Petiole: |  |
|  | Length: | About 1 to 2 mm |
|  | Color: | Yellow/green, RHS 144 A |
|  | Anthocyanin coloration: | Absent |
| C. | Disease/pest resistance/susceptibility: | No special disease/pest resistance/ susceptibility |
| D. | Ability to be Shipped During In Bud Stage Development | Yes |
| E. | Leaf blade on plant |  |
|  | Length: | 40 to 50 mm |
|  | Width: | 11 to 16 mm |
|  | Thickness: | Medium |
|  | Blistering: | Medium |
|  | Pubescence: | On upper side (midrib excluded): present |
|  | Depth of cuts or incisions in leaf: | Basal part: deep |
|  |  | Central part: deep |
|  |  | Distal part: medium |
|  | Color: | Upper side: Yellow, RHS 147 A |
|  |  | Bottom side: Green, RHS 137 C |
|  | Glossiness on upper side: | Medium |
|  | Angle of apex: | Right angle |
|  | Shape of apex: | Pointed |
|  | Margin of lobes: | Serrate |
|  | Extensions of margin: | Medium |
|  | Petiole: |  |
|  | Length: | About 14 cm |
|  | Color: | Green/yellow, RHS 146 C |
|  | Anthocyanin coloration: | Present - Greyed/red, RHS 185 A |
| Disease/pest resistance/susceptibility: |  | no special disease/pest resistance/ susceptibility |
| Ability to be Shipped During In Bud Stage Development |  | Yes |

Example 9: GERFOLIA™ *Gerbera jamesonii* designated 'BL07.790'

The present example of the invention comprises a new and distinct cultivar of *Gerbera jamesonii,* referred to by the cultivar name 'BL07.790'.

GERFOLIA™ *Gerbera jamesonii* 'BL07.790' originated from a hybridization program in Kudelstaart, The Netherlands, in 2006. The female parent was *Gerbera jamesonii* designated 'TEREAGER' (unpatented), and is characterized by its single flowering type, flat inflorescense shape, diameter of 8 cm, red in color, with 15 to 18 leaves on the flower stem, along the top half of the flower stem, leaves on the flower stem are 40 to 110 mm long and green in color. The male parent was *Gerbera jamesonii* designated 'BL04.713' (unpatented), and is characterized by its single flowering type, flat inflorescense shape, diameter of 7 cm, orange in color, with 15 to 20 leaves on the flower stem, distributed equally along the flower stem, leaves on the flower stem are 20 to 120 mm long and green in color. The new GERFOLIA™ *Gerbera jamesonii* 'BL07.790' was selected by the inventors as a single flowering plant within the progeny of the stated cross in a controlled environment in Kudelstaart, The Netherlands, on or about 2007.

**[0222]** The first asexual reproduction of 'BL07.790' was accomplished by vegetative cuttings in April of 2007 in Kudelstaart, The Netherlands. The new *Gerbera* 'BL07.790' is presently being propagated by vegetative cuttings. Horticultural examination of selected units initiated in 2007 has demonstrated that the combination of characteristics as herein disclosed for 'BL07.790' are firmly fixed and are retained through successive generations of asexual reproduction. The new *Gerbera* reproduces true-to-type.

The following traits have been repeatedly observed and are determined to be unique characteristics of the GERFOLIA™ *Gerbera jamesonii* designated 'BL07.790', which in combination distinguish this *Gerbera jamesonii* as a new and distinct cultivar:

1. Single flowering type, flat inflorescence, measuring about 8 cm in diameter and having a general red tonality color (from a distance of 3 meters);

2. one or more leafy flower stems with about 16 to 20 full or partial leaves per flower stem which are about 50 to 150 mm in length and about 8 to 23 mm in width, and are distributed in the top half of the flower stem;

3. ability to be shipped during in bud stage of development;

4. plant height is 30 to 35 cm, spread is 55 to 60 cm;

5. strong stem which measures in length about 60 to 65 cm;

6. flowering response time is 8 to 9 weeks, and

7. no special pest/disease resistance/susceptibility is present.

The new GERFOLIA™ *Gerbera jamesonii* designated 'BL07.790' has not been observed under all possible environmental conditions. The phenotype of 'BL07.790' may vary with variations in environment such as temperature, light intensity, and day length without any change in the genotype of the plant. The following observations, measurements and comparisons describe plants of 'BL07.790' grown in Kudelstaart, The Netherlands, under greenhouse conditions which closely approximate those generally used in commercial practice.

Plants of GERFOLIA™ *Gerbera jamesonii* 'BL07.790' differ from plants of the *Gerbera jamesonii* designated "'TEREAGER' (unpatented) and 'BL 04.713' (unpatented), in the following characteristics of Table 27:

Table 27

| Trait | New GERFOLIA™ 'BL07.790' | 'TEREAGER' (unpatented) | 'BL 04.713' (unpatented) |
|---|---|---|---|
| Flowering Type | Single | Single | Single |
| Inflorescence Shape | Flat | Flat | Flat |
| Inflorescence Size | About 8 cm | About 8 cm | About 7 cm |
| Inflorescence Color | Red | Red | Orange |
| Number of Full of Partial Leaves Per Flower Stem | About 16 to about 20 | About 15 to about 18 | About 15 to about 20 |
| Leaf Size: | Length: 50 to 150 mm Width: 8 to 23 mm | Length: 40 to 110 mm Width: 3 to 14 mm | Length: 20 to 120 mm Width: 2 to 12 mm |
| Leaf Distribution per Flower Stem | Top half of the flower stem | Top half of the flower stem | Equally along the flower stem |
| Ability to be Shipped During In Bud Stage of Development | Yes | Yes | Yes |

Of the many *Gerbera L.* commercial cultivars known to the inventors, there is no cultivar similar in comparison to 'BL07.790'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

    A.    Flower Head:

        Type:                      Capitulum

        Flowering Type:        Single

        Diameter:             About 7 to 8 cm

        Color (general tonality from a distance of 3 meters:)    Red

(continued)

| | | |
|---|---|---|
| Shape: | | Flat |
| Involucre: | | |
| | Height from point of attachment of involucre to top of flower head: | About 25 mm |
| | Height: | About 20 mm |
| | Diameter: | About 20 to 22 mm |
| | Number of bracts: | About 30 |
| | Longitudinal axis of inner rows: | Incurving |
| | Anthocyanin: | Absent or very weak at tips |
| | Pubescence: | Medium |
| Ray florets: | | |
| | Number: | About 30 to 35 |
| | Overall Shape: | Obovate |
| | Longitudinal axis outer row: | Strongly incurving |
| | Longitudinal axis inner row: | Absent, single flowe |
| | Longitudinal axis of ray female floret: | Reflexing |
| | Longitudinal axis of ray male floret: | Reflexing |
| | Outer ray floret: | |
| | Cross section: | Flat |
| | Length: | About 30 mm |
| | Width: | About 11 to 12 mm |
| | Longitudinal folding: | Medium |
| | Angle of apex: | Obtuse |
| | Shape of apex: | Rounded |
| | Incisions of apex: | 0 to 1 |
| | Depth of incision: | absent to very shallow |
| | Color (top side): | Orange/red in color, RHS 33 A |
| | Color (bottom side) | Orange/red in color, RHS 31 B |
| | Color distribution on inner side: | Uniform |
| | Edge of different color: | Absent |
| | Striation: | Absent |
| | Claw spot: | Present 10% RHS 31 D |
| B. | Disc florets: | |
| | Number: | 225 to 250 |
| | Disc diameter: | About 23 to 26 mm |
| | Color (mature, upperside): | Greyed/purple in color, RHS 187 B |
| | Color (immature, top): | Greyed/purple in color, RHS 187 A |
| | Main color upperside corolla: | Female flowers: Orange/red, RHS 35 A |
| | | Male flowers: Orange/red, RHS 34 A |
| C. | Reproductive Organs: | |
| | Style: | Main color distal part: Yellow, RHS 4 A |
| | Stigma: | Main color: Yellow/green, RHS 1 A |
| | Anthers: | Main color: Yellow, RHS 13 A |
| | | Color of top relative to other parts is lighter |
| | | Longitudinal stripes are absent |
| | | Intensity of anthocyanin coloration is absent |

(continued)

| | | |
|---|---|---|
| Pappus: | | Main color: greyed/purple, RHS 184 B |
| | | Color of top relative to other parts is darker |
| | | Level of top relative to closed disc florets: far below |
| Fertility: | | Fertility (male and female) as well as the seed setting is reasonable |
| D. | Peduncle: | |
| | Length: | About 60 to 65 cm |
| | Cross section: | Elliptic |
| | Tendency to fasciation: | Absent |
| | Thickness: | Medium |
| | Strength: | Strong |
| | Pubescence: | Dense |
| | Color: | Yellow/Green, RHS 144 A |
| | Anthocyanin coloration: | At base: present - greyed/orange RHS 176 B |
| | | At top: absent |
| | Involucral bracts: | Present - green, RHS 143 B |

II. PLANT:

| | | | |
|---|---|---|---|
| A. | General appearance: | | |
| | Height: | | 30 to 35 cm (excluding any inflorescences). |
| | Spread: | | 55 to 60 cm |
| B. | Foliage: | | |
| | Leaf blade on stem | | See Table 2 "overview description of leaves per flower stem", category 4/5" |
| | | Number of partial or full leaves per flower stem: | 16 to 20 |
| | | Distribution of Leaves | Equally along the flower |
| | | Length: | 50 to 150 mm |
| | | Width: | 8 mm to 23 mm |
| | | Thickness: | Medium |
| | | Blistering: | Medium |
| | | Pubescence: | Present |
| | | Depth of cuts or incisions in leaf: | Basal part: absent |
| | | | Central part: medium |
| | | | Distal part: medium |
| | | Color: | Upper side: green RHS 137 A |
| | | | Bottom side: green RHS 137 C |
| | | Glossiness on upper side: | Medium |
| | | Angle of apex: | Very acute |
| | | Shape of apex: | Pointed |
| | | Margin of lobes: | Serrate |
| | | Extensions of margin: | Medium |
| | Petiole: | | |
| | Length: | | About 25 +/- 3 cm |
| | Color: | | Green, RHS 143 C |
| | Anthocyanin coloration: | | Absent |
| C. | Disease/pest | | No special disease/pest resistance/susceptibility |

(continued)

| | | resistance/susceptibility: | |
|---|---|---|---|
| D. | | Ability to be Shipped During In Bud Stage Development: | Yes |
| E. | | Leaf blade on plant: | |
| | | Length: | 35 to 40 cm |
| | | Width: | 15 to 18 cm |
| | | Thickness: | Medium |
| | | Blistering: | Medium |
| | | Pubescence: | On upper side (midrib excluded): present |
| | | Depth of cuts or incisions in leaf: | Basal part: deep |
| | | | Central part: deep |
| | | | Distal part: medium |
| | | Color: | Upper side: green, RHS 139 A |
| | | | Bottom side: green, RHS 137 C |
| | | Glossiness on upper side: | Medium |
| | | Angle of apex: | Obtuse |
| | | Shape of apex: | Rounded |
| | | Margin of lobes: | Serrate |
| | | Extensions of margin: | Medium |
| | | Petiole: | |
| | | Length: | About 10 cm |
| | | Color: | Green/yellow, RHS 144 B |
| | | Anthocyanin coloration: | Present - Greyed/purple, RHS 185 B |
| Disease/pest resistance/susceptibility: | | | no special disease/pest resistance/susceptibility |
| Ability to be Shipped During In Bud Stage Development | | | Yes |

Example 10: GERFOLIA™ *Gerbera jamesonii* designated 'BL07.774'

The present example of the invention comprises a new and distinct cultivar of *Gerbera jamesonii,* referred to by the cultivar name 'BL07.774'.

GERFOLIA™ *Gerbera jamesonii* 'BL07.774' originated from a hybridization program in Kudelstaart, The Netherlands, in 2006. The female parent was *Gerbera jamesonii* designated 'BL06.730'(unpatented), and is characterized by its single flowering type, funnel shaped inflorescense, diameter of 9 cm, pink of color, 8 to 12 leaves, along the top half of the flower stem, leaves on the flower stem are 30 to 130 mm long and green in color. The male parent was *Gerbera jamesonii* designated 'BL05.724' unpatented, and is characterized by its semi-double flowering type, flat shaped inflorescense, diameter of 8 cm, red/purple in color, 14 to 17 leaves on the flower stem, distributed on the top half of the flower stem, leaves on the flower stem are 20 to110 mm and green in color. The new GERFOLIA™ *Gerbera jamesonii* 'BL07.774' was selected by the inventors as a single flowering plant within the progeny of the stated cross in a controlled environment in Kudelstaart, The Netherlands, on or about 2007.

The first asexual reproduction of 'BL07.774' was accomplished by vegetative cuttings in April of 2007 in Kudelstaart, The Netherlands. The new *Gerbera* 'BL07.774' is presently being propagated by vegetative cuttings. Horticultural examination of selected units initiated in 2007 has demonstrated that the combination of characteristics as herein disclosed for 'BL07.774' are firmly fixed and are retained through successive generations of asexual reproduction. The new *Gerbera* reproduces true-to-type.

The following traits have been repeatedly observed and are determined to be unique characteristics of the GERFOLIA™ *Gerbera jamesonii* designated 'BL07.774', which in combination distinguish this *Gerbera jamesonii* as a new and distinct cultivar:

1. Single flowering type, flat inflorescence, measuring about 8 cm in diameter and having a general red/purple tonality color (from a distance of 3 meters);

2. one or more leafy flower stems with about 12 to 15 full or partial leaves per flower stem which are about 30 to 180 mm in length and about 5 to 25 mm in width, and are distributed on the top two thirds of the flower stem;

3. ability to be shipped during in bud stage of development;

4. plant height is 25 to 35 cm, spread is 30 to 40 cm;

5. strong stem which measures in length about 50 to 60 cm;

6. flowering response time is 8 to 9 weeks, and

7. no special pest/disease resistance/susceptibility is present.

The new GERFOLIA™ *Gerbera jamesonii* designated 'BL07.774' has not been observed under all possible environmental conditions. The phenotype of 'BL07.774' may vary with variations in environment such as temperature, light intensity, and day length without any change in the genotype of the plant. The following observations, measurements and comparisons describe plants of 'BL07.774' grown in Kudelstaart, The Netherlands, under greenhouse conditions which closely approximate those generally used in commercial practice.

Plants of GERFOLIA™ *Gerbera jamesonii* 'BL07.774' differ from plants of the *Gerbera jamesonii* designated 'BL06.730'(unpatented) and 'BL05.724' (unpatented), in the following characteristics of Table 28:

Table 28

| Trait | New GERFOLIA™ 'BL07.774' | 'BL06.730' (unpatented) | 'BL05.724' (unpatented) |
|---|---|---|---|
| Flowering Type | Single | Single | Semi-double |
| Inflorescence Shape | Flat | Funnel | Flat |
| Inflorescence Size | About 8 cm | About 9 cm | About 8 cm |
| Inflorescence Color | Red/purple | Pink | Red/pmple |
| Number of Full of Partial Leaves Per Flower Stem | About 12 to about 15 | About 8 to about 12 | About 14 to about 17 |
| Leaf Size: | Length: 30 to 180 mm Width: 5 to 25 mm | Length: 30 to 130 mm Width: 4 to 20 mm | Length: 20 to 110 mm Width: 2 to 18 mm |
| Leaf Distribution per Flower Stem | Top two thirds of the flower stem | Top half of the flower stem | Top two thirds of the flower stem |
| Ability to be Shipped During In Bud Stage of Development | Yes | Yes | Yes |

Of the many *Gerbera L.* commercial cultivars known to the inventors, there is no cultivar similar in comparison to 'BL07.774'.

In the following description, color references are made to the Royal Horticultural Society Colour Chart (RHS), except where general colors of ordinary significance are used. The color values were determined at 11:00 a.m. on June 30, 2008, under artificial light in a greenhouse, in Kudelstaart, The Netherlands. The age of the plant described is about 9 months old.

I. INFLORESCENCE:

| | | |
|---|---|---|
| A. | Flower Head: | |
| | Type: | Capitulum |
| | Flowering Type: | Single |
| | Diameter: | About 8 to 9 cm |
| | Color (general tonality from a distance of 3 meters:) | Red/purple |
| | Shape: | Flat |
| | Involucre: | |
| | Height from point of attachment of involucre to top | About 30 mm |

(continued)

| | |
|---|---|
| of flower head: | |
| Height: | About 20 mm |
| Diameter: | About 25 to 30 mm |
| Number of bracts: | About 35 |
| Longitudinal axis of inner rows: | Incurving |
| Anthocyanin: | Absent or very weak at tips |
| Pubescence: | Medium |
| Ray florets: | |
| Number: | About 35 to 40 |
| Overall Shape: | Obovate |
| Longitudinal axis outer row: | Incurving |
| Longitudinal axis inner row: | Absent, single flowe |
| Longitudinal axis of ray female floret: | Staight |
| Longitudinal axis of ray male floret: | Staight |
| Outer ray floret: | |
| Cross section: | Flat |
| Length: | About 28 mm |
| Width: | About 7 to 8 mm |
| Longitudinal folding: | Medium |
| Angle of apex: | Right angle |
| Shape of apex: | Pointed |
| Incisions of apex: | 0 to 1 |
| Depth of incision: | absent to very shallow |
| Color (top side): | Red/purple in color, RHS 57 C |
| Color (bottom side) | Red/purple in color, RHS 62 B |
| Color distribution on inner side: | Uniform |
| Edge of different color: | Absent |
| Striation: | Absent |
| Claw spot: | Absent |
| B. Disc florets: | |
| Number: | 225 to 275 |
| Disc diameter: | About 27 to 30 mm |
| Color (mature, upperside): | Yellow/green in color, RHS 153 B |
| Color (immature, top): | Yellow/green in color, RHS 145 A |
| Main color upperside corolla: | Female flowers: Red/purple, RHS 57 D |
| | Male flowers: Red/purple, RHS 57 B |
| C. Reproductive Organs: | |
| Style: | Main color distal part: Purple, RHS 75 D |
| Stigma: | Main color: Yellow/green, RHS 150 D |
| Anthers: | Main color: Yellow, RHS 17 B |
| | Color of top relative to other parts is lighter |
| | Longitudinal stripes are absent |
| | Intensity of anthocyanin coloration is absent |
| Pappus: | Main color: Yellow, RHS 14 D |
| | Color of top relative to other parts is identical |
| | Level of top relative to closed disc florets: far below |

(continued)

| | | | |
|---|---|---|---|
| | | Fertility: | Fertility (male and female) as well as the seed setting is reasonable |
| | D. | Peduncle: | |
| | | Length: | About 50 to 60 cm |
| | | Cross section: | Elliptic |
| | | Tendency to fasciation: | Absent |
| | | Thickness: | Medium |
| | | Strength: | Strong |
| | | Pubescence: | Dense |
| | | Color: | Yellow/Green, RHS 144 A |
| | | Anthocyanin coloration: | At base: present - red/purple RHS 68 B |
| | | | At top: absent |
| | | Involucral bracts: | Present - green, RHS 143 A |

II. PLANT:

| | | | |
|---|---|---|---|
| | A. | General appearance: | |
| | | Height: | 25 to 35 cm (excluding any inflorescences). |
| | | Spread: | 30 to 40 cm |
| | B. | Foliage: | |
| | | Leaf blade on stem | See Table 2 "overview description of leaves per flower stem", category 5" |
| | | Number of partial or full leaves per flower stem: | Up to 12 to 15 |
| | | Distribution of Leaves | On top two thirds of stem |
| | | Length: | 30 to 180 mm |
| | | Width: | 5 mm to 25 mm |
| | | Thickness: | Medium |
| | | Blistering: | Medium |
| | | Pubescence: | Present |
| | | Depth of cuts or incisions in leaf: | Basal part: absent |
| | | | Central part: shallow |
| | | | Distal part: shallow |
| | | Color: | Upper side: green RHS 147 A |
| | | | Bottom side: green RHS 147 B |
| | | Glossiness on upper side: | Medium |
| | | Angle of apex: | Acute |
| | | Shape of apex: | Pointed |
| | | Margin of lobes: | Entire to sinnuate |
| | | Extensions of margin: | Absent |
| | | Petiole: | |
| | | Length: | About 12 mm |
| | | Color: | Green, RHS 146 A |
| | | Anthocyanin coloration: | Absent |
| | C. | Disease/pest resistance/susceptibility: | No special disease/pest resistance/susceptibility |
| | D. | Ability to be Shipped During In Bud Stage Development | Yes |
| | E. | Leaf blade on plant | |

(continued)

| | | |
|---|---|---|
| | Length: | 29 to 33 cm |
| | Width: | 8 to 10 cm |
| | Thickness: | Medium |
| | Blistering: | Medium |
| | Pubescence: | On upper side (midrib excluded): present |
| | Depth of cuts or incisions in leaf: | Basal part: medium |
| | | Central part: medium |
| | | Distal part: shallow |
| | Color: | Upper side: green, RHS 137 A |
| | | Bottom side: green, RHS 146 A |
| | Glossiness on upper side: | Medium |
| | Angle of apex: | Obtuse |
| | Shape of apex: | Rounded |
| | Margin of lobes: | Dentate |
| | Extensions of margin: | Small |
| | Petiole: | |
| | Length: | About 7 to 10 cm |
| | Color: | Green/yellow, RHS 146 D |
| | Anthocyanin coloration: | Present - Greyed/purple, RHS 185 B |
| C. | Disease/pest resistance/susceptibility: | no special disease/pest resistance/susceptibility |
| D. | Ability to be Shipped During In Bud Stage Development | Yes |

The examples described herein are illustrative of the present invention and are not intended to be limitations thereon. Different embodiments of the present invention have been described according to the present invention. Many modifications and variations may be made to the methods and plants described and illustrated herein without departing from the spirit and scope of the invention.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention, which is defined by the following claims. All publications and patent applications mentioned in this specification are indicative of the level of skill of those in the art to which the invention pertains.

All publications and patent applications are herein incorporated by reference to the same extent as if each individual publication or patent application were specifically and individually indicated to be incorporated by reference in its entirety.

**Claims**

1. A *Gerbera L.* plant, with one or more flower stems containing foliage, with at least 5 or more full or partial leaves per flower stem.

2. The leafy flower stem *Gerbera L.* plant according to claim 1, wherein substantially all the flower stems produced by said plant contain foliage with at least 5 or more full or partial leaves per flower stem.

3. The leafy flower stem *Gerbera L.* plant according to claims 1 or 2, wherein the leaves of the flower stem are (i) small to extra large in size, ranging from (a) at least 40 mm or more in length, and (b) at least 4 mm or more in width, and (ii) distributed either equally along the flower stem or along the top half of the flower stem.

4. A *Gerbera L.* plant according to claim 1, wherein the quantity of leaves per flower stem are at least 5, to about 30, or more than 30 full or partial leaves per flower stem.

**5.** The leafy flower stem *Gerbera L.* plant according to claim 4, wherein substantially all the flower stems produced by said plant contain at least 5, to about 30, or more than 30 full or partial leaves per flower stem.

**6.** The leafy flower stem *Gerbera L.* plant according to claims 4 or 5, wherein the leaves of the flower stem are (i) small to extra large in size, ranging from (a) at least 40 mm, to about 200 mm, or more than 200 mm in length, and (b) at least 4 mm, to about 60 mm, or more than 60 mm in width, and (ii) distributed either equally along the flower stem or along the top half of the flower stem.

**7.** A *Gerbera L.* plant according to claim 1, wherein the quantity of leaves per flower stem are at least 5, 6, 8, 10, 12, 14, 16, 18, 20, 22, 24, 26, 28, 30, any integer between 5 and 30, or more than 30 full or partial leaves per flower stem.

**8.** The leafy flower stem *Gerbera L.* plant according to claim 7, wherein substantially all the flower stems produced by said plant contain at least 5, 6, 8, 10, 12, 14, 16, 18, 20, 22, 24, 26, 28, 30, any integer between 5 and 30, or more than 30 full or partial leaves per flower stem.

**9.** The leafy flower stem *Gerbera L.* plant according to claims 7 or 8, wherein the leaves of the flower stem are (i) small to extra large in size, ranging from (a) at least 40, 50, 60, 70, 80, 90, 100, 110, 120, 130, 140, 150, 160, 170, 180, 190, 200, any integer between 40 and 200, or more than 200 mm in length, and (b) at least 4, 6, 8, 10, 12, 14, 16, 18, 20, 22, 24, 26, 28, 30, 32, 34, 36, 38, 40, 42, 44, 46, 48, 50, 52, 54, 56, 58, 60, any integer between 4 and 60, or more than 60 mm in width, and (ii) distributed either equally along the flower stem or along the top half of the flower stem.

**10.** The leafy flower stem *Gerbera L.* plant according to claims 1, 4 or 7, wherein said plant is selected from the group consisting of *G. aberdarica, G. abyssinica, G. ambigua, G. anandria, G. aspleniifolia, G. aurantiaca, G. bojeri, G. bonatiana, G. bracteata, G. brevipes, G. burchellii, G. burmanni, G. candollei, G. cavaleriei, G. chilensis, G. cineraria, G. connata, G. conrathii, , G. cordata, G. coronopifolia, G. curvisquama, G. delavayi, G. discolor, G. diversifolia, G. elegans, G. elliptica, G. emirnensis, G. ferruginea, G. flava, G. galpinii, G. glandulosa, G. henryi, G. hieracioides, G. hirsuta, G. hypochaeridoides, G. integralis, G. integripetala, G. jamesonii, G. knorringiana, G. kokanica, G. kraussii, G. kunzeana, G. lacei, G. lagascae, G. lanuginosa, G. lasiopus, G. leandrii, G. leiocarpa, G. leucothrix, G. lijiangensis, G. lynchii, G. macrocephala, G. nepalensis, G. nervosa, G. nivea, G. parva, G. peregrina, G. perrieri, G. petasitifolia, G. piloselloides, G. plantaginea, G. plicata, G. podophylla, G. pterodonta, G. pulvinata, G. pumila, G. randii, G. raphanifolia, G. ruficoma, G. saxatilis, G. semifloscularis, G. serotina, G. speciosa, G. tanantii, G. tomentosa, G. tuberosa, G. uncinata, G. viridifolia, G. welwitschii and G. wrightii.*

**11.** A method of breeding a leafy flower stem *Gerbera L.* plant that produces one or more flower stems, including observing, measuring and comparing said flower stem having a degree of foliage per flower stem defined as a measure of at least 5 or more full or partial leaves per flower stem, comprising the steps of:

    a. crossing a first single-type *Gerbera L.* plant having the leafy flower stem trait in its genetic background, either as the male or female parent to:

        i. a *Gerbera L.* plant without the leafy flower stem trait in its genetic background; or
        ii. A second *Gerbera L.* plant having the leafy flower stem trait in its genetic background;

    b. screening F1 progeny;
    c. selecting F1 progeny having the leafy flower stem trait in its genetic background;
    d. crossing said F1 progeny to:

        i. a second *Gerbera L.* plant without the leafy flower stem trait in its genetic background; or
        ii. A third *Gerbera L.* plant having the leafy flower stem trait in its genetic background;

    e. screening F2 progeny;
    f. selecting F2 progeny having the leafy flower stem trait in its genetic background;
    g. crossing said F2 progeny to:

        i. a third *Gerbera L.* plant without the leafy flower stem trait in its genetic background; or
        ii. A fourth *Gerbera L.* plant having the leafy flower stem trait in its genetic background;

h. screening F3 progeny;
i. selecting F3 progeny having the leafy flower stem trait in its genetic background;
j. crossing said F3 progeny to:

i. a fourth *Gerbera L.* plant without the leafy flower stem trait in its genetic background; or
ii. A fifth *Gerbera L.* plant having the leafy flower stem trait in its genetic background;

k. screening F4 progeny;
l. selecting F4 progeny having the leafy flower stem trait in its genetic background;
m. crossing said F4 progeny to:

i. a fifth *Gerbera L.* plant without the leafy flower stem trait in its genetic background; or
ii. A sixth *Gerbera L.* plant having the leafy flower stem trait in its genetic background;

n. screening F5 progeny;
o. selecting F5 progeny having the leafy flower stem trait in its genetic background;
p. crossing said F5 progeny to:

i. a sixth *Gerbera L.* plant without the leafy flower stem trait in its genetic background; or
ii. A seventh *Gerbera L.* plant having the leafy flower stem trait in its genetic background;

q. screening F6 progeny;
r. selecting F6 progeny having the leafy flower stem trait in its genetic background;
s. crossing said F6 progeny to:

i. a seventh *Gerbera L.* plant without the leafy flower stem trait in its genetic background; or
ii. A eighth *Gerbera L.* plant having the leafy flower stem trait in its genetic background; and

t. screening F7 progeny;
u. selecting leafy flower stem progeny.

12. A method of breeding a leafy flower stem *Gerbera L.* plant that produces one or more flower stems, including observing, measuring and comparing said flower stem having a degree of foliage per flower stem defined as a measure of having at least 5 or more full or partial leaves per flower stem, comprising the steps of:

a. crossing a first single-type *Gerbera L.* plant without the leafy flower stem trait in its genetic background, either as the male or female parent to:

i. a second *Gerbera L.* plant without the leafy flower stem trait in its genetic background; or
ii. A *Gerbera L.* plant having the leafy flower stem trait in its genetic background;

b. screening F1 progeny;
c. selecting F1 progeny having the leafy flower stem trait in its genetic background;
d. crossing said F1 progeny to:

i. a third *Gerbera L.* plant without the leafy flower stem trait in its genetic background; or
ii. A second *Gerbera L.* plant having the leafy flower stem trait in its genetic background;

e. screening F2 progeny;
f. selecting F2 progeny having the leafy flower stem trait in its genetic background;
g. crossing said F2 progeny to:

i. a fourth *Gerbera L.* plant without the leafy flower stem trait in its genetic background; or
ii. A third *Gerbera L.* plant having the leafy flower stem trait in its genetic background;

h. screening F3 progeny;
i.. selecting F3 progeny having the leafy flower stem trait in its genetic background;
j. crossing said F3 progeny to:

i. a fifth *Gerbera L.* plant without the leafy flower stem trait in its genetic background; or

ii. A fourth *Gerbera L.* plant having the leafy flower stem trait in its genetic background;

l. screening F4 progeny;

m. selecting F4 progeny having the leafy flower stem trait in its genetic background;

n. crossing said F4 progeny to:

i. a sixth *Gerbera L.* plant without the leafy flower stem trait in its genetic background; or

ii. A fifth *Gerbera L.* plant having the leafy flower stem trait in its genetic background;

o. screening F5 progeny;

p. selecting F5 progeny having the leafy flower stem trait in its genetic background;

q. crossing said F5 progeny to:

i. a seventh *Gerbera L.* plant without the leafy flower stem trait in its genetic background; or

ii. A sixth *Gerbera L.* plant having the leafy flower stem trait in its genetic background;

r. screening F6 progeny;

s. selecting F6 progeny having the leafy flower stem trait in its genetic background;

t. crossing said F6 progeny to:

i. An *Gerbera L.* plant having the leafy flower stem trait in its genetic background; and

u. screening F7 progeny;

v. selecting leafy flower stem progeny.

13. The method according to claims 11 or 12, wherein said first, second, third, fourth, fifth, sixth, seventh, or eighth *Gerbera L.* plants are the same or different cultivars.

14. A *Gerbera L.* plant, with one or more flower stems with at least 5, to about 30, or more than 30 full or partial leaves per flower stem, produced by one of the methods according to claims 11 or 12.

15. A *Gerbera L.* plant, with one or more flower stems with at least 5, 6, 8, 10, 12, 14, 16, 18, 20, 22, 24, 26, 28, 30, any integer between 5 and 30, or more than 30 full or partial leaves per flower stem, produced by one of the methods according to claims 11 or 12.

16. Method of selling of *Gerbera L.* flowers comprising harvesting at the bud stage 3 and making them commercially available to consumers.

FIG. 1

By Category

FIG. 2A

FIG. 2B

FIG. 2C

FIG. 2D

FIG. 2E

FIG. 2F

FIG. 2G

FIG. 2H

FIG. 2I

FIG. 2J

FIG. 2K

FIG. 2L

FIG. 2M

FIG. 3A

FIG. 3B

FIG. 3C

FIG. 3D

FIG. 3E

FIG. 3F

FIG. 3G

FIG. 3H

FIG. 3I

FIG. 3J

FIG. 3K

FIG. 3L

FIG. 4A

FIG. 4B

FIG. 4C

FIG. 4D

FIG. 4E

FIG. 4F

FIG. 4G

FIG. 5

40 cm

FIG. 6

1 meter box

FIG. 7A

FIG. 7B

FIG. 8A FIG. 8B FIG. 8C FIG. 8D

FIG. 8E FIG. 8F FIG. 8G FIG. 8H FIG. 8I FIG. 8J

FIG. 9A

FIG. 9B

FIG. 10A

Normal Gerbera

GERFOLIA™

FIG. 10B

Minigerbera

GERFOLIA™

FIG. 11A

FIG. 11B

FIG. 12B

FIG. 12A

FIG. 12D FIG. 12E

FIG. 12C

FIG. 13B

FIG. 13E

FIG. 13A

FIG. 13D

FIG. 13C

FIG. 14A

FIG. 14B

FIG. 14C

FIG. 14D

FIG. 15B

FIG. 15E

FIG. 15D

FIG. 15A

FIG. 15C

FIG. 16B

FIG. 16A

FIG. 16E

FIG. 16D

FIG. 16C

FIG. 17B

FIG. 17A

FIG. 17D

FIG. 17C

FIG. 18A

FIG. 18B

FIG. 18C

FIG. 19B

FIG 19A

FIG. 19C

FIG. 20B

FIG 20A

FIG. 20C

FIG. 21B

FIG 21A

FIG. 21C

FIG. 22B

FIG 22A

FIG. 22C

FIG. 23B

FIG 23A

FIG. 23C

FIG. 24B

FIG 24.A

FIG. 24C

FIG. 25B

FIG 25.A

FIG. 25C

Europäisches
Patentamt

European
Patent Office

Office européen
des brevets

## PARTIAL EUROPEAN SEARCH REPORT

**Application Number**

which under Rule 63 of the European Patent Convention EP 08 07 5608
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TOPOONYANONT NOPMANEE ET AL: "Bushiness in Gerbera jamesonii: Abnormal shoot development during micropropagation" JOURNAL OF HORTICULTURAL SCIENCE AND BIOTECHNOLOGY, vol. 74, no. 6, November 1999 (1999-11), pages 675-679, XP009107646 ISSN: 1462-0316 Abstract Page 675 col 2 ----- | 1-10,14, 15 | INV. A01H5/02 |
| A | US 11 289 P (STRAVERS LAMBERTUS JOHANNES MA [NL]) 14 March 2000 (2000-03-14) * the whole document * ----- | 1-10,14, 15 | |
| A | US 11 291 P (STRAVERS LAMBERTUS JOHANNES MA [NL]) 14 March 2000 (2000-03-14) * the whole document * ----- | 1-10,14, 15 | |

TECHNICAL FIELDS
SEARCHED      (IPC)

A01H

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 3 November 2008 | Obel, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

**Europäisches Patentamt**
**European Patent Office**
**Office européen des brevets**

**INCOMPLETE SEARCH
SHEET C**

**Application Number**

EP 08 07 5608

Claim(s) not searched:
        11-13,16

Reason for the limitation of the search (non-patentable invention(s)):

Article 52 (2)(c) EPC - Scheme, rules and method for doing business
Article 53 (b) EPC - Essentially biological process for the production of plants

## ANNEX TO THE EUROPEAN SEARCH REPORT
## ON EUROPEAN PATENT APPLICATION NO.

EP 08 07 5608

This annex lists the patent family members relating to the patent documents cited in the above-mentioned European search report.
The members are as contained in the European Patent Office EDP file on
The European Patent Office is in no way liable for these particulars which are merely given for the purpose of information.

03-11-2008

| Patent document cited in search report | | Publication date | Patent family member(s) | Publication date |
|---|---|---|---|---|
| US 11289 | P | 14-03-2000 | NONE | |
| US 11291 | P | 14-03-2000 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60948380 B

**Non-patent literature cited in the description**

- **BARIGOZZI, C. ; L. QUAGLIOTTI.** Current Research on Breeding of Gerbera. *Proceedings of the Eucarpia Meeting on Carnation and Gerbera, Allasio,* 1978, 57-68

- **VAN OS, D.P.M.** Stageverslag Gerbera Veredeling, Practical Period Agriculture. University Wageningen, Dept. of Plant Breeding, 1995